# EUROPEAN PATENT SPECIFICATION

(11) **EP 1 173 622 B1**
(45) Date of publication and mention of the grant of the patent: **15.06.2005**
(21) Application number: 00940234.8
(22) Date of filing: 30.04.2000
(51) Int. Cl.: C12Q 1/68

(54) **DIAGNOSTIC SEQUENCING BY A COMBINATION OF SPECIFIC CLEAVAGE AND MASS SPECTROMETRY**
DIAGNOSTISCHE SEQUENZIERUNG DURCH EINE KOMBINATION VON SPEZIFISCHER SPALTUNG UND MASSENSPEKTROMETRIE
SEQUENCAGE DIAGNOSTIQUE PAR COMBINAISON DE CLIVAGE SPECIFIQUE ET DE SPECTROMETRIE DE MASSE

(30) Priority: 30.04.1999 US 131984 P
(43) Date of publication of application: 23.01.2002
(73) Proprietor: Methexis Genomics N.V., 9052 Zwijnaade (BE)
(72) Inventor: ZABEAU, Marc, B-9000 Gent (BE); STANSSENS, Patrick, B-9830 Sint-Martens-Latem (BE)
(74) Representative: De Clercq, Ann
(86) International application number: PCT/EP2000/003904
(87) International publication number: WO 2000/066771

(56) References cited:
- WO-A-94/21663
- WO-A-97/33000
- WO-A-98/20166
- WO-A-98/54571
- US-A- 5 436 143
- US-A- 5 643 798
- US-A- 5 869 242

## Description

### FIELD OF INVENTION

The present invention is in the field of nucleic acid-based diagnostic assays. More particularly, it relates to methods useful for the "diagnostic sequencing" of regions of sample nucleic acids for which a prototypic or reference sequence is already available (also referred to as 're-sequencing'), or which may be determined using the methods described herein. This diagnostic technology is useful in areas that require such re-sequencing in a rapid and reliable way: (i) the identification of the various allelic sequences of a certain region/gene, (ii) the scoring of disease-associated mutations, (iii) the detection of somatic variations, (iv) studies in the field of molecular evolution, (v) the determination of the nucleic acid sequences of prokaryotic and eukaryotic genomes; (vi) identifying one or more nucleic acids in one or more biological samples; (vii) and determining the expression profile of genes in a biological sample and other areas.

### BACKGROUND OF INVENTION

Complete reference genome sequences for a number of model organisms as well as humans are currently available or are expected to become available in the near future. A parallel challenge is to characterize the type and extent of variation in the sequences of interest because it underlies the heritable differences among individuals and populations. In humans, the vast majority of sequence variation consists of nucleotide substitutions referred to as single nucleotide polymorphisms (SNPs). DNA sequencing is the most sensitive method to discover polymorphisms [Eng C. and Vijg J. *et al., Nature Biotechnol*. 15: 422-426 (1997)]. A growing panel of such sequence variants, together with powerful methods to monitor them [Landegren U. *et al., Genome Res.* 8: 769-776 (1998)], is useful in linkage studies to identify even the most subtle disease susceptibility loci [Lander E. and Schork N., *Science* 265: 2037-2048 (1994); Risch N. and Merikangas K., *Science* 273: 1516-1517 (1996)]. Also, the identification of all (functional) allelic variants will require the re-sequencing of particular regions in a large number of samples [Nickerson D. *et al., Nature Genet.* 19: 233-240 (1998)]. Although a number of methods to monitor known SNPs have been developed [Landegren U. *et al., Genome Res.* 8: 769-776 (1998)], re-sequencing is likely to be routinely applied to secure diagnoses of patients. Indeed, in a significant number of disease-associated genes that have been surveyed thus far, literally hundreds or even thousands of different mutations have been identified and catalogued. Consequently, sequence determination represents the ultimate level of resolution and may be the preferred method to monitor which mutation or combination of mutations, out of a large number of mutations of known clinical relevance, is present.

It would appear that the field of biomedical genetics will rely heavily on sequencing technology. Hence, there is a need for advanced sequencing methods that are time- and cost-competitive, and at the same time accurate and robust. Recent developments in this area include improvements to the basic dideoxy chain termination sequencing method [Sanger *et al. Proc. Natl. Acad. Sci. USA* 74: 5463-5467 (1977); reviewed by Lipshutz R. and Fodor S. *et al., Current Opinion in Structural Biology* 4: 376-380 (1994)], as well as new approaches that are based on entirely new paradigms. Two such novel approaches are sequencing-by-hybridization (SBH) [Drmanac R. *et al., Science* 260: 1649-1652 (1993)] and pyro-sequencing [Ronaghi M. *et al., Science* 281: 363-365 (1998); Ronaghi M. *et al., Anal. Biochem.* 242: 84-89 (1996)]. While the concepts of these approaches have been experimentally validated, their ultimate acceptance and usage may depend on the type of application *- e.g. de novo* sequencing, re-sequencing, and genotyping of known SNPs.

Recently, progress has also been made in the use of mass spectroscopy (MS) to analyze nucleic acids [Crain, P.F. and McCloskey, J.A., *Current Opinion in Biotechnology* 9: 25-34 (1998), and references cited therein]. One promising development has been the application of MS to the sequence determination of DNA and RNA oligonucleotides [Limbach P., *Mass Spectrom. Rev. 15*: 297-336 (1996); Murray K., *J. Mass Spectrom.* 31: 1203-1215 (1996)]. MS and more particularly, matrix-assisted laser desorption/ionization MS (MALDI MS) has the potential of very high throughput due to high-speed signal acquisition and automated analysis off solid surfaces. It has been pointed out that MS, in addition to saving time, measures an intrinsic property of the molecules, and therefore yields a significantly more informative signal [Köster H. *et al., Nature Biotechnol*., **14**: 1123-1128 (1996)].

Sequence information can be derived directly from gas-phase fragmentation [see for example Nordhoff *E. et al., J. Mass Spectrom.,* **30**:99-112 (1995); Little D. *et al., J. A. Chem. Soc.,* **116**: 4893-4897 (1994); Wang B. *et al*., WO 98/03684 and WO 98/40520; Blöcker H. *et al*., EP 0 103 677; Foote S. *et al*., WO 98/54571]. In contrast, indirect methods measure the mass of fragments obtained by a variety of methods in the solution phase, *i.e.,* prior to the generation of gas phase ions. In its simplest form, mass analysis replaces the gel-electrophoretic fractionation of the fragment-ladder (*i.e.,* a nested set of fragments that share one common endpoint) generated by the sequencing reactions. The sequencing reactions need not necessarily be base-specific because the base-calling may also be based on accurate mass measurement of fragments that terminate at successive positions and that differ from one another by one nucleotide residue. The fragment-ladder can be generated by the Sanger method [Köster H. *et al., Nature Biotechnol.,***14**: 1123-1128 (1996); Reeve M.A., Howe R. P., Schwarz T., U.S. 5,849,542; Köster H., U.S. 5,547,835; Levis R. and Romano L., U.S. 5,210,412 and U.S. 5,580,733; Chait B. and Beavis R., U.S. 5,453,247], by base-specific partial RNA digestion [Hahner S. *et al., Nucleic Acids Res.,* **25:** 1957-1964 (1997); Köster H., WO 98/20166] or by chemical cleavage [Isola N. *et al., Anal. Chem.,* **71:** 2266-2269 (1999); references cited in Limbach P., *Mass Spectrom. Rev.,* **15:** 297-336 (1996)]. An alternative method consists of analyzing the ladder generated by exonuclease digestion from either the 3'- or 5'-end [Pieles U. *et al., Nucleic Acids Res.,* **21:** 3191-3196 (1993); Köster H., U.S. 5,851,765; Engels J. *et al*., WO 98/45700; Tarr G. and Patterson D., WO 96/36986; Patterson D., U.S. 5,869,240].

A severe limitation of both the direct and indirect MS methodologies under the current performance conditions is the poor applicability to chain lengths beyond ∼30-50 nucleotides. As a consequence, it has been suggested that the prospects for MS lie with DNA diagnostic assays, rather than large-scale sequencing [Smith L., *Nature Biotechnol.,* **14:** 1084-1087 (1996)]. Given the fact that MS represents an exquisite means to analyze short nucleotide fragments, the various MS-based processes that have been described for nucleic acid based diagnostic purposes generally involve the derivation and analysis of such relatively short fragments [see for example Köster H., WO 96/29431; Köster H. *et al*., WO 98/20166; Shaler T. *et al*., WO 98/12355; Kamb A., U.S. 5,869,242; Monforte J. *et al*., WO 97/33000; Foote S. *et al*., WO 98/54571].

Some of the MS-based assays have been used for the scoring of defined mutations or polymorphisms. Other processes derive multiple oligonucleotide fragments and yield a 'mass-fingerprint' so as to analyze a larger target nucleic acid region for mutations and/or polymorphisms. The latter MS analyses are however considerably less informative in that they are essentially restricted to the detection of sequence variations. The methods cannot be applied to diagnostic sequencing of nucleic acids, where the term diagnostic sequencing means the unequivocal determination of the presence, the nature and the position of sequence variations. At best, the measurements confirm the base composition of small fragments whose masses are determined with sufficient accuracy to reduce the number of possible compositional isomers. Also, it will be realized that only certain changes in composition (as revealed by shifts in the mass spectrum) can be unambiguously assigned to a polymorphism or mutation. A match between the spectrum of the interrogated sequence and a reference-spectrum obtained from wild-type sequence or sequences known to contain a given polymorphism, is assumed to indicate that the interrogated nucleic acid region is wild-type or incorporates the previously known polymorphisms, thereby disregarding certain other possible interpretations.

While most methods in the art do yield sequence-related information, they do not disclose that a combination of several different mass spectra, obtained after complementary digestion reactions, allows for the effective survey of a nucleic acid region and provides an unambiguous assignment of both known as well as previously unknown sequence variations that occur relative to a reference nucleic acid with a known nucleotide sequence.

In view of the limitations of the methods described above, the art would clearly benefit from a new procedure for the diagnostic sequencing of nucleic acids that would overcome the shortcomings of the processes discussed above.

In comparison with conventional sequencing technology, *i.e*., the gel-electrophoretic analysis of fragment ladders, the methods of the present invention are more suited for the simultaneous analysis of multiple target sequences. In general, each particular sequence or sequence variant is associated with a distinct set of mass peaks. Consequently, the sequencing reactions according to the methods of the present invention lend themselves readily to (i) multiplexing (*i*.*e*., the analysis of two or more target non-contiguous target regions from a single biological sample), (ii) the analysis of heterozygous samples, as well as (iii) pooling strategies (*i*.*e*., the simultaneous sequencing of the analogous regions derived from two or more different biological samples).

Because of the multiplex capacity, the present methods can be adapted as a tool for the genome-wide discovery and scoring of polymorphisms (*e*.*g*., SNPs) useful as markers in genetic linkage studies. The unambiguous identification/diagnosing of a number of variant positions is less demanding than full sequencing and, consequently, a considerable number of target genomic loci can be combined and analyzed at the same time, especially when their lengths are kept relatively small. The number of markers that can be scored in parallel will depend on the level of genetic diversity in the species of interest and on the precise method used to prepare and analyze the target nucleic acids, but may typically be in the order of a few tens to up to 100 with current MS capabilities. The addition of multiplexing to the high-precision and high-speed characteristics of MS constitutes a new marker technology that enables the large-scale and cost-effective scoring of several (tens of) thousands of markers. Some aspects of the application of the present methods to genome-wide genotyping are described in Example 5.

Sequencing reactions according to the methods of the present invention yield, in principle, a discrete set of fragments for each individual sequence or sequence variant whereas conventional sequence ladders stack on top of one another. Therefore, such sequences or sequence variants can be analyzed even when present as a lesser species. This is a useful quality for the analysis of clinical samples which are often genetically heterogeneous because of the presence of both normal and diseased cells or in itself (*e*.*g*., cancerous tissue, viral quasi-species). Additionally, the ability to detect mutations at a low ratio of mutant over wild-type allele makes it practicable to pool individual biological samples, a strategy which should permit a more cost-effective search for genomic sequence variations in a population.

The present invention rests in part on the insight that integration of the data obtained in a set of complementary fingerprints produced by an appropriate set of complementary cleavage reactions of the invention represents a level of characterization of a sample nucleic acid essentially equal to sequence determination. The present invention is also directed to the use of cleavage protocols that result in the generation of cleavage products that range from mono- and dinucleotides to fragments of a few tens of nucleotides that are particularly suited for analysis by MS. At the same time, the present method is distinct from the other fragmentation processes that are limited to screening target nucleic acids for a wide range of potential mutations. According to the present invention, a combination of several different mass spectra, obtained after complementary digestion reactions, coupled with systematic computational analysis allows the survey of a selected target nucleic acid or region thereof and leads to the unambiguous assignment of both known and previously unknown sequence variations. In certain aspects of the present invention, knowledge of the reference sequence in combination with the methods disclosed herein allows modeling of the experimental approach, anticipation of potential ambiguities, and the design of an adequate resolution.

### SUMMARY OF INVENTION

The present invention is directed to a mass spectroscopic method for detecting or analyzing a particular nucleic acid sequence. The present invention is useful for *de novo* sequencing or re-sequencing nucleic acid in a rapid and reliable way which permits, for example, the identification of the various allelic sequences of a certain region/gene, the identification and scoring of disease-associated mutations, the detection of somatic variations, determining genetic diversity in molecular evolution, and the determination of the genomic sequences *e.g.,* of viral and bacterial isolates.

In one embodiment, the present invention is directed to a method for mass spectrometry based determination and/or analysis of the sequence of a target nucleic acids present in a biological sample. In this method, said target nucleic acid is derived from said biological samples, and obtained target nucleic acid are subjected to a set of four separate base-specific complementary cleavage reactions, wherein each cleavage reaction generates a non-ordered set of fragments which is a set of fragments without a common end point. In a subsequent step, the non-ordered fragments, are analyzed by mass spectrometry. A systematic computational analysis is then performed on the mass spectra of the non-ordered set of fragments to determine and/or analyze the sequence of the target nucleic acid. In the method of the present invention said complementary cleavage reactions refer to target nucleic acid digestions characterized by varying specificity and/or to digestion of alternative forms of the target sequence.

In one aspect of this embodiment, the biological sample is derived from an organism selected from the group consisting of eukaryotes prokaryotes, and viruses.

The one or more target nucleic acids may be selected from the group consisting of a single stranded DNA, a double stranded DNA, a cDNA, a single stranded RNA, a double stranded RNA, a DNA/RNA hybrid, and a DNA/RNA mosaic nucleic acid.

In a second embodiment, the one or more target nucleic acids are selected from the group consisting of an amplified nucleic acid fragment, a cloned nucleic acid fragment, and a series of non-contiguous DNA fragments from the genome. In one aspect of this invention, the amplified one or more target nucleic acids are derived by one or more consecutive amplification procedures selected from the group consisting of *in vivo* cloning, the polymerase chain reaction (PCR), reverse transcription followed by the polymerase chain reaction (RT-PCR), strand displacement amplification (SDA), and transcription based processes.

In a preferred embodiment, the amplified target nucleic acid is a transcripts generated from a single stranded or double stranded target target nucleic acids by a process comprising the steps of: (a) linking operatively a transcription control sequence to said target nucleic acid; and (b) transcribing one or both strands of the target nucleic acid of step a) using (an) RNA polymerase(s) that recognize(s) the transcription control sequence.

In a further preferred embodiment, the transcription control sequences are operatively linked to the target nucleic acid by PCR amplification using primers that incorporate the transcriptional control sequences as 5'-extensions.

Said transcription control sequence may be selected from the group consisting of eukaryotic transcription control sequences, prokaryotic transcription control sequences, and viral transcription control sequences. The prokaryotic transcription control sequence may be selected from the group consisting of T3, T7, and SP6 promoters. The cognate RNA polymerases which utilize the T3, T6 or SP6 promoters may be either a wild-type or a mutant RNA polymerases, the mutant polymerases being capable of incorporating into the transcript non-canonical substrates with a 2'-deoxy, 2'-*O*-methyl, 2'-fluoro or 2'-amino substituent. According to the invention the mutant RNA polymerase may be either T7 or SP6 mutant polymerase.

In a third embodiment, the derived target nucleic acids incorporates (a) nucleoside(s) that are (is) modified on the base, the sugar, and/or the phosphate moiety. Such modifications may alter the specificity of cleavage by certain reagent(s), and/or the mass of the cleavage products, and/or the length of the cleavage products.

Said modification(s) may be introduced through the enzymatic incoryoration of modified deoxynucleoside triphosphates, modified ribonucleoside triphosphates, and/or modified dideoxynucleoside triphosphates, or wherein the modification is introduced chemically, or wherein the modification is introduced through a combination of both methods. In one aspect the modification may consist of a 2'-deoxy, 2'-*O*-methyl, 2'-fluoro or 2'-amino substituent on the nucleotide triphosphates. In a second aspect, the modification may consist of phosphorothioate intemucleoside linkages or phosphorothioate internucleoside linkages further reacted with an alkylating reagent. In another aspect, the modification may consist of a methyl group on C5 of the uridine-5'-monophosphate subunits. In yet another aspect, the modification may consist of nucleotides that incorporate alternative isotopes.

In a fourth embodiment, the target nucleic acid is purified prior to cleavage. Said purification may be achieved through immobilization or by chromatography

In a fifth embodiment complementary cleavage reactions are selected from the group consisting of enzymatic cleavage, chemical cleavage, and physical cleavage.

The complementary cleavage reactions may be characterized by a relaxed mono-nucleotide, mono-nucleotide, relaxed di-nucleotide, or di-nucleotide specificity. In a preferred embodiment, the target nucleic acid may be subjected to chemical digestion reaction consisting of treatment with alkali or with reagents used in the Maxam & Gilbert sequencing method. In another preferred embodiment, the target nucleic acid is subjected to enzymatic cleavage reaction using an enzyme(s) selected from the group consisting of endonucleases and exonucleases. According to the invention, said endonuclease may be selected from the group consisting of restriction enzymes, RNA endonucleases, DNA endonucleases and non-specific phosphodiesterases. In a more preferred embodiment, the endonuclease(s) may be (a) selective or non-selective RNA endonuclease(s), selected from the group consisting of the G-specific T₁ ribonuclease, the A-specific U₂ ribonuclease, the A/U specific phyM ribonuclease, the U/C specific ribonuclease A, the C-specific chicken liver ribonuclease (RNaseCL3), and cusativin, non-specific RNase-I, and pyrimidine-adenosine preferring RNases isolated from *E. coli, Enterobacter sp*., or *Saccharomyces cerevisiae.* In one aspect of this preferred embodiment, the target nucleic acid is a phosphorothioate-modified single-stranded DNA or RNA and wherein the nucleic acid digestions are performed with nuclease P1.

In a sixth embodiment of the invention, the target nucleic acid may be a mosaic RNA/DNA nucleic acid or a modified mosaic RNA/DNA nucleic acid, prepared with mutant polymerases, and wherein the cleavage reagents may be RNA endonucleases. DNA endonucleases or alkali.

Alternatively, the target nucleic acid may be a transcript, a modified transcript, a mosaic RNA/DNA transcript or a modified mosaic RNA/DNA transcript, prepared with wild type or mutant RNA polymerases, and wherein the cleavage reagents may be (a) selective or non-selective RNA endonuclease(s) or alkali.

If the target nucleic acid is a mosaic RNA/DNA transcript that incorporates either dCMP, dUMP or dTMP, prepared with mutant T7 or SP6 polymerase, the cleavage reagent may be a pyrimidine-specific Rnase. Said pyrimidine-specific Rnase may be for instance RNase-A.

In a seventh embodiment, the set of non-ordered fragments of step (b) of the method of the present invention may be additionally purified using cation exchange beads.

In an eighth embodiment, the set of non-ordered fragments of step (b) of the method of the present invention may be applied onto a solid support. Said solid support may be chosen from a group consisting of solid surfaces, plates and chips.

In a ninth embodiment, the mass spectrometry based determination and/or analysis spectroscopical analysis of the nucleic acid fragments is performed using a mass spectrometer selected from the group consisting of Matrix-Assisted Laser Desorption/Ionization-Time-of-flight (MALDI-TOF), Electrospray-Ionization (ESI), and Fourier Transform-Ion Cyclotron Resonance (FT-ICR). In a preferred embodiment the mass spectrometer used for the analysis of the cleavage fragments is MALDI-TOF.

In a tenth embodiment, the method according to the present invention may be used for mass sipectometry based determination and/or analysis of a target nucleic acid present in a biological sample, wherein for said target nucleic acid a reference nucleic acid sequence is known; with said method comprising an additional step wherein the mass spectra of the non-ordered fragments obtained in step c) of the method of the present invention are compared with the known or predicted mass spectra for a reference nucleic acid sequence, and deducing there from, by systematic computational analysis, all or part of the nucleotide sequence of the target nucleic acid, and comparing the deduced nucleic acid sequence with the reference nucleic acid to determine whether the target nucleic acid has the same sequence or a different sequence from the reference nucleic acid.

In one aspect of this invention, the nucleic acid sequence difference that is determined is a deletions, substitution, insertions or combinations thereof. Said nucleic acid sequence difference/variation may be for instance a Single Nucleic Polymorphism (SNP).

In another aspect, the method of the present invention can be used for scoring known as well as unknown nucleotide sequence variations of a target nucleic acids derived from a biological sample, for which a known reference nucleic acid sequence is available. In this embodiment, a target nucleic acids derived from a biological sample, and a reference nucleic acid are subjected to a set of four separate base-specific complementary cleavage reactions, and the products of the cleavage reactions are analyzed by mass spectroscopic methods. The mass spectra of the target nucleic acid is then compared with the mass spectra of the reference nucleic acid sequence, and the nucleotide sequence variations/mutations in the target nucleic acids are scored by comparing the nucleic sequence between the target nucleic acid and reference nucleic acid by systematic computational analysis.

This method allows the identification of the various allelic sequences of a certain region/gene, the scoring of disease-associated mutations, the detection of somatic variations, or studies in the field of molecular evolution.

In another aspect, this method can be used for genome-wide genotyping of a biological sample . In this method, a target nucleic acid(s), derived from a biological sample, (is) are amplified and then subjected to a set of four separate base-specific complementary cleavage reactions. In one aspect, multiple targets are derived from a single sample and are analyzed simultaneously. The products of the cleavage reactions are then analyzed by mass spectroscopic methods. The mass spectra of the known or unknown target nucleic acid is compared with the mass spectra of a reference nucleic acid. This comparison is then used to infer the genotype of an organism from which the biological sample is derived and to determine therefrom the genetically relevant nucleic acid sequence variations of the known or unknown nucleic acids.

In yet another aspect, the method of the present invention can be used to identify/detect a target nucleic acids in a biological samples. In this method, target nucleic acids, derived from a biological sample, are amplified and then subjected to a set of four separate base-specific complementary cleavage reactions. In one aspect, multiple targets are derived from a single sample and are analyzed simultaneously. The products of the cleavage reactions are then analyzed by mass spectroscopic methods. The identity of a target nucleic acid(s) is deduced by comparing the mass spectra of the known or unknown target nucleic acid(s) with each other or by comparison with a plurality of mass spectra of reference nucleic acids.

In a eleventh embodiment, the method of the present invention may be used for mass spectometry based determination and/or analysis of the sequence of a target nucleic acid of unknown sequence present in a biological sample. If by using said method the sequence is not uniquely defined after step (d) of the method of the present invention, steps (a) throuph (d) are repeated, thereby generating modified forms of said target nucleic acid and/or different portions of said target nucleic acid, and performing supplementary mono- and/or di-nucleotide specific cleavage reactions rendering supplementary sets of non-ordered fragments until the combined data converge into a unique sequence solution. Said method may be used for genome wide genotyping of a biological sample.

In a twelfth embodiment, the target nucleic acid may be prepared by the concomitant amplification of multiple fragments. Furthermore, biological sample of the present method may comprise a pool of samples.

Also encompassed by the present invention is a kit for mass spectrometry based determination and/or analysis of the sequence of a target nucleic acid present in a biological sample according to a method of the invention, the kit comprising: nucleotide triphosphates; (a) polymerase(s); (a) nucleic acid cleaving agent(s) to perform different base specific cleavage reactions; and, a computer program comprising codes for performing a systematic computational analysis on the mass spectra obtained from the mass spectrometrical analysis of non-ordered fragments when executed on a data processing system. Said program comprises the steps of 1/ subjecting a reference nucleic acid and sequence variants thereof to the different base specific cleavage to generate fragments, computing the mass of each fragment, generating the mass spectra of the fragments from the reference nucleic acid and the sequence variants thereof for each of the base specific cleavage reactions, and, 2/ matching these computationally derived mass spectra with the spectra obtained experimentally in the different base specific cleavage reactions. This kit may further comprise cation exchange beads in order to purify the non-ordered set of fragments generated by means of a method according to the present invention; a solid support whereon the non-ordered set of fragments may be applied;

The kits of the invention may be used for mass spectometry based determination and/or analysis of the sequence of a target nucleic acid, for determining and/or analyzing sequence differences, for scoring known as well as unknown nucleotide sequence variations, for detecting/identifying, or, for performing genome wide genotyping using a target nucleic acid present in a biological sample, for which target nucleic acid a reference nucleic acid sequence is known. Alternatively, said kits may be used for mass spectometry based determination and/or analysis of the sequence of a target nucleic acid, for detecting/identifying, or, for performing genome wide genotyping using a target nucleic acid of unknown sequence present in a biological sample.

### DESCRIPTION OF DRAWINGS

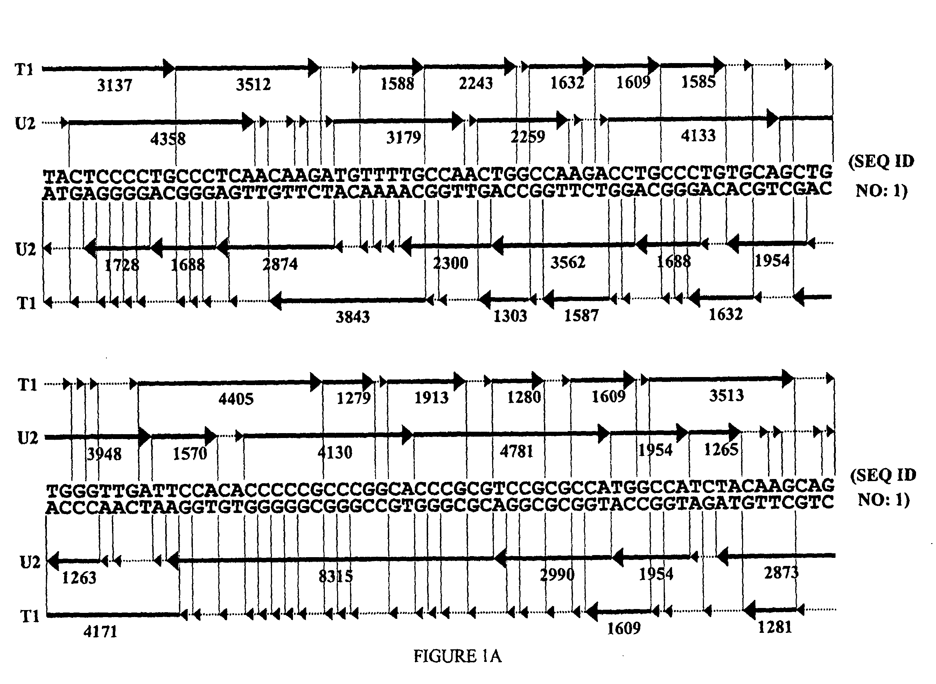

Figure 1A (SEQ ID NO: 1) graphically represents the first 120 nucleotides of exon 5 of human p53 as well as the fragments that would result from cleavage of the (+) and (-) strand transcript after G (RNase-T1) or A (RNase-U2). The dotted and full arrows correspond to the resulting ≤3-mer and ≥4-mer cleavage products. The arrows from left to right represent fragments from the (+) strand, while the arrows from right to left represent fragments from the (-) strand. The numbers indicate the neutral molecular masses of the ≥4-mer ribonucleotide fragments. The calculation assumes that all fragments contain 5'-hydroxyl and 3'-phosphate groups.

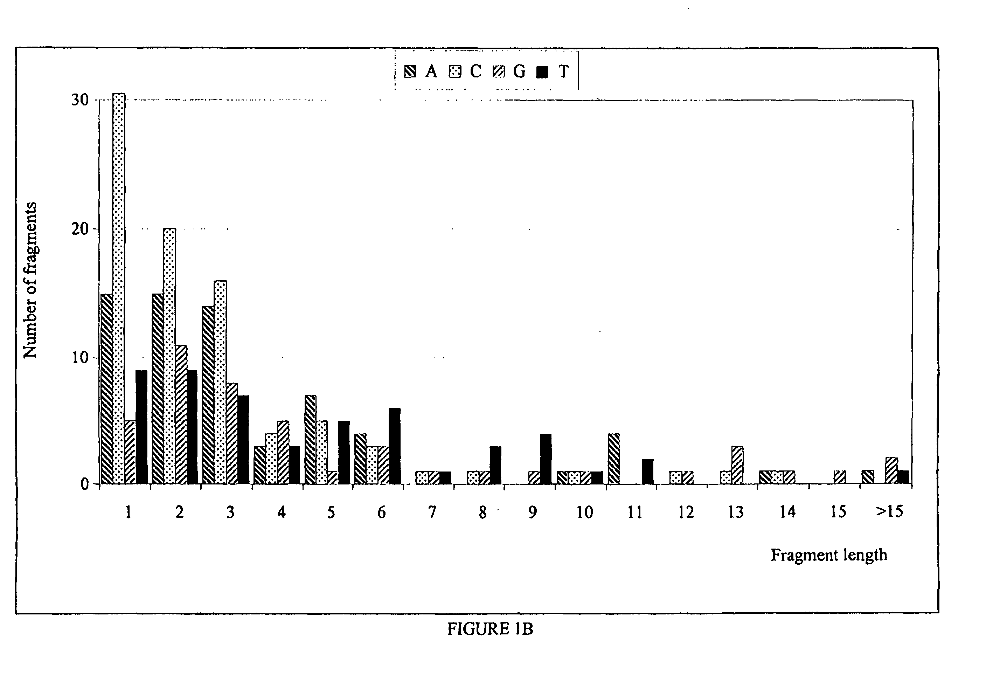

Figure 1B shows the size distribution of the products that result from base-specific cleavage of a 245 nucleotides long exemplary sequence.

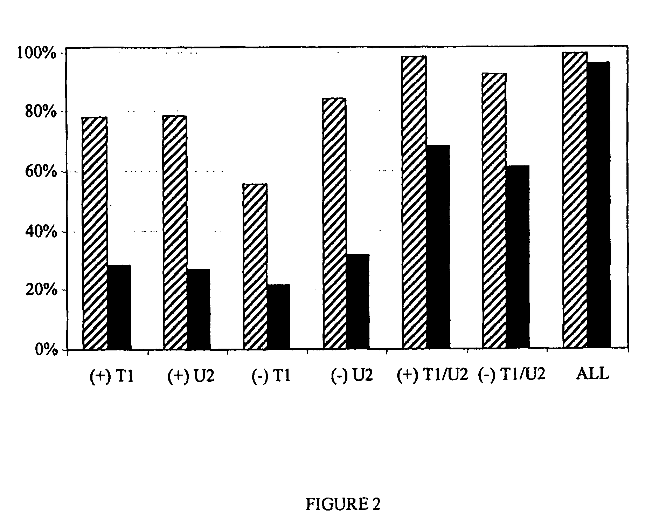

Figure 2 summarizes the results of the mutational simulation analysis of a 200-base-pair segment of the HIV protease gene and shows the percentages of the mutational changes that can be detected (hatched bars) and mapped (filled bars). The results were computed for single RNase digests of the (+) and (-) strands with respectively RNase-T1 (T1) and RNase-U2 (U2), separately or combined (T1/U2). All refers to the analysis with the four different reactions.

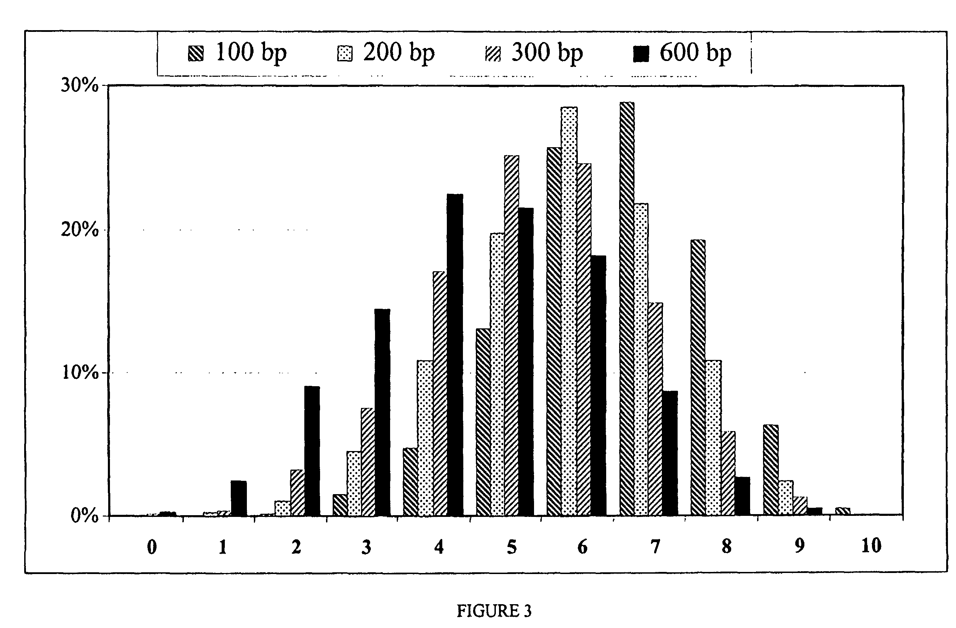

Figure 3 shows the distributions of the number of diagnostic fragments obtained for the mutational simulation analysis of 1.200 base-pair sequence of HIV when using different length segments of respectively 100, 200, 300, and 600 base-pairs.

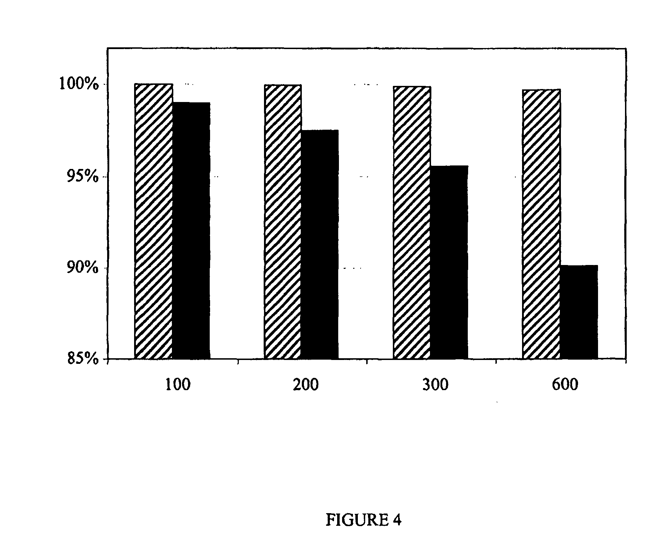

Figure 4 summarizes the results of the mutational simulation analysis of 1.200 base-pair sequence of HIV and shows the percentages of the single nucleotide substitutions that can be detected (hatched bars) and mapped unambiguously (filled bars) as a function of the length of the interrogated segments.

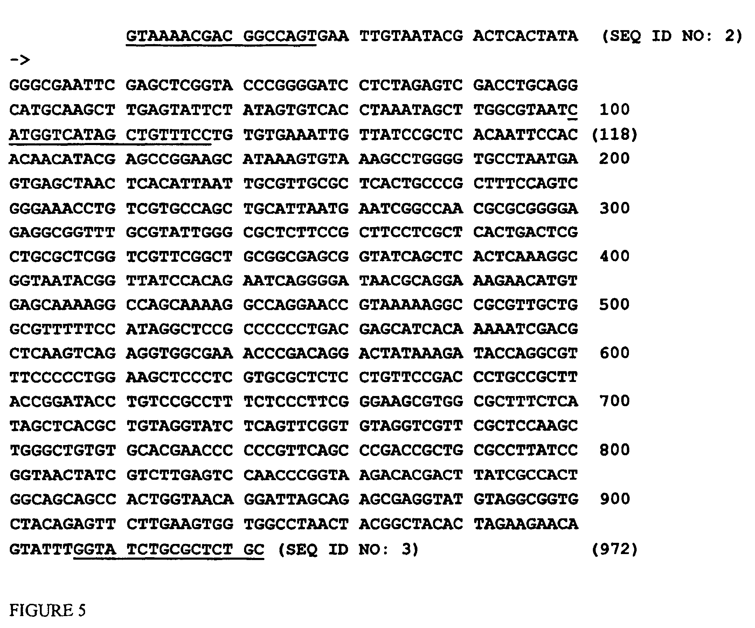

Figure 5 (SEQ ID NO: 2 and SEQ ID NO: 3) is a graphic representation of the pGEM3-Zf(+) derived nucleotide sequences used as a model in Examples 2 and 4. The regions corresponding to the PCR primers are underlined. Two PCR products (158 and 1012 base-pairs long) were generated. Both amplification products encompass the phage T7 promoter site; the transcription initiation site is indicated with an arrow. The numbering refers to the respective transcripts (118 and 972 nucleotides).

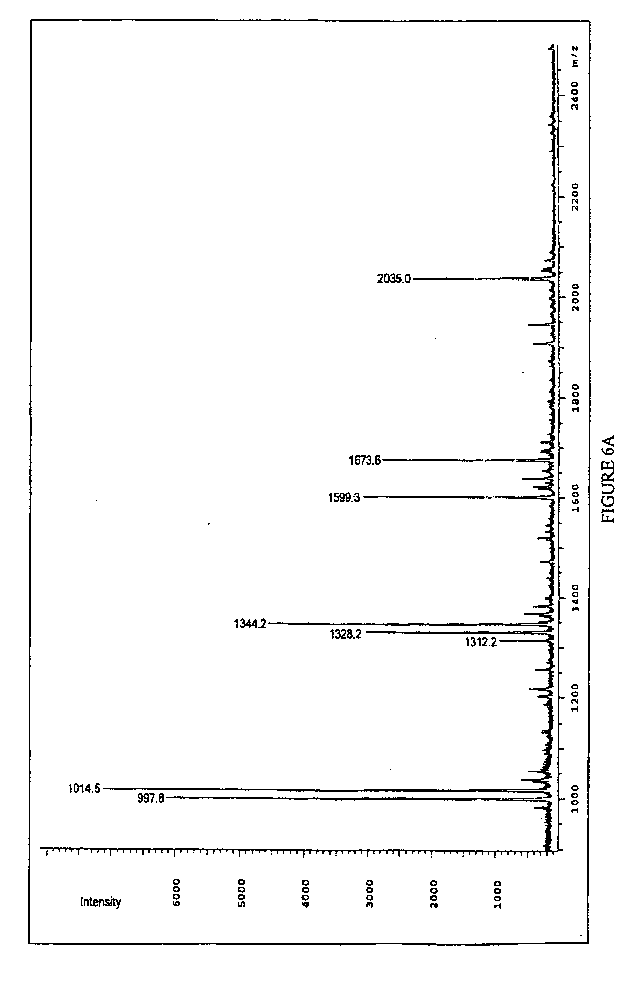

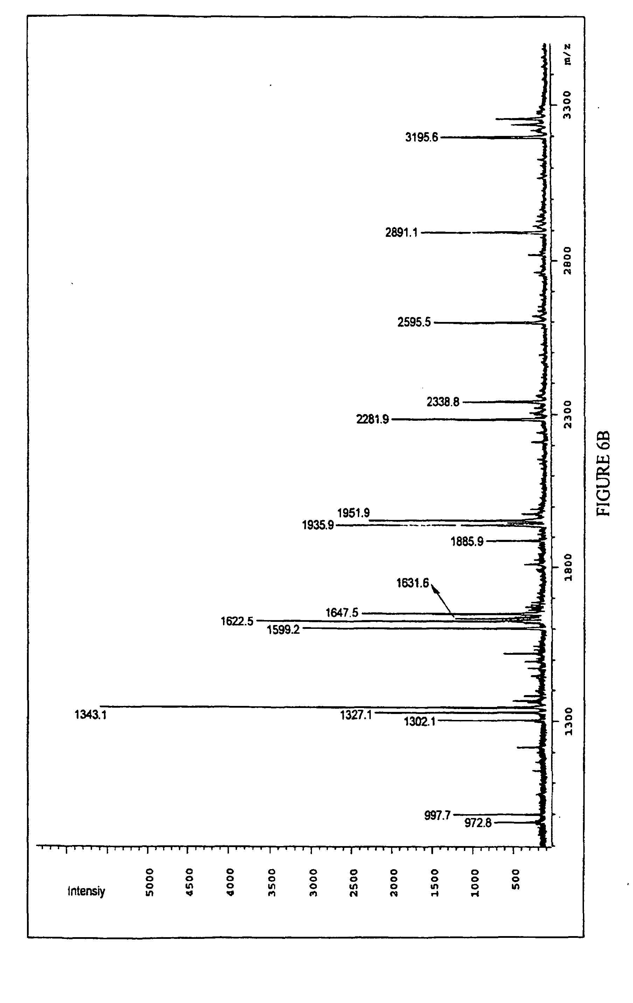

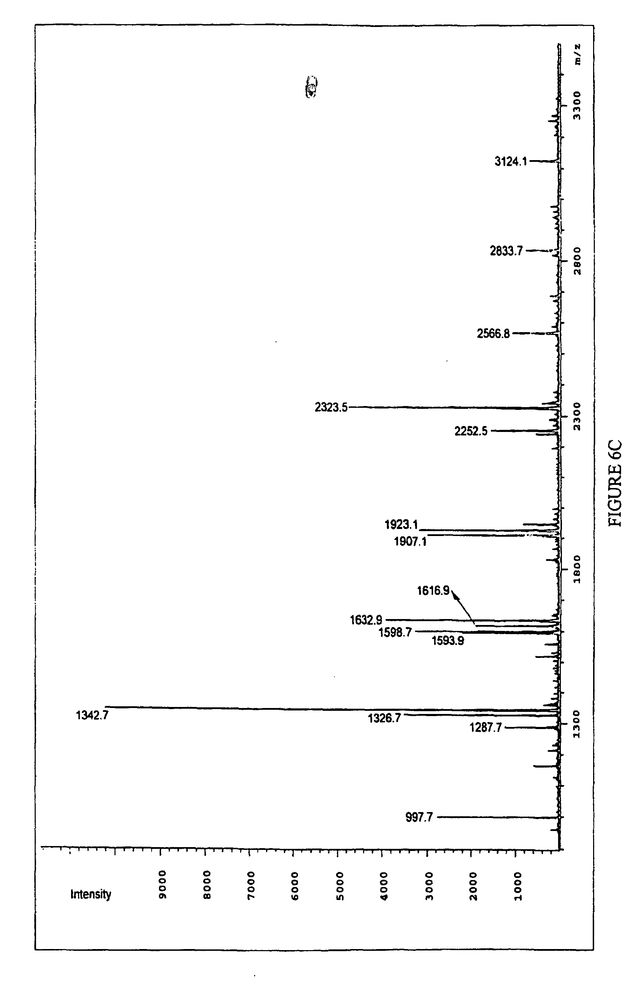

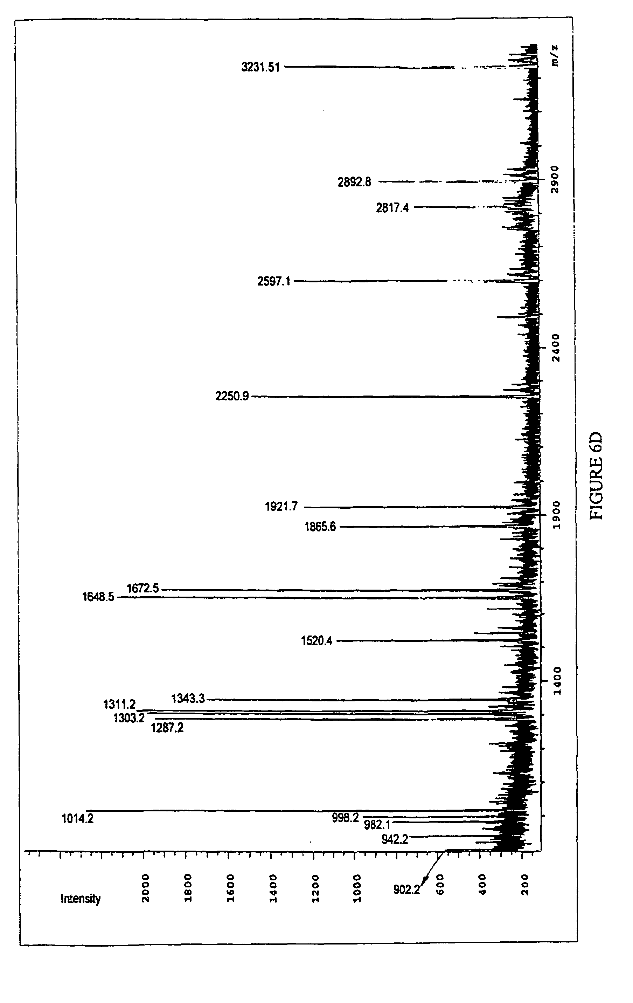

Figure 6 is a graphical representation of the MALDI-TOF mass spectra of the RNase-A cleavage reactions of pGEM3-Zf(+) derived transcripts. The following transcripts were digested: (A) a regular transcript synthesized with rNTPs, (B) a transcript in which UMP residues are replaced by dTMP, (C) a transcript where UMP is replaced by dUMP, and (D) one that incorporates dCMP instead of CMP. Observed masses are indicated above the peaks that match with predicted digestion products (see Table II).

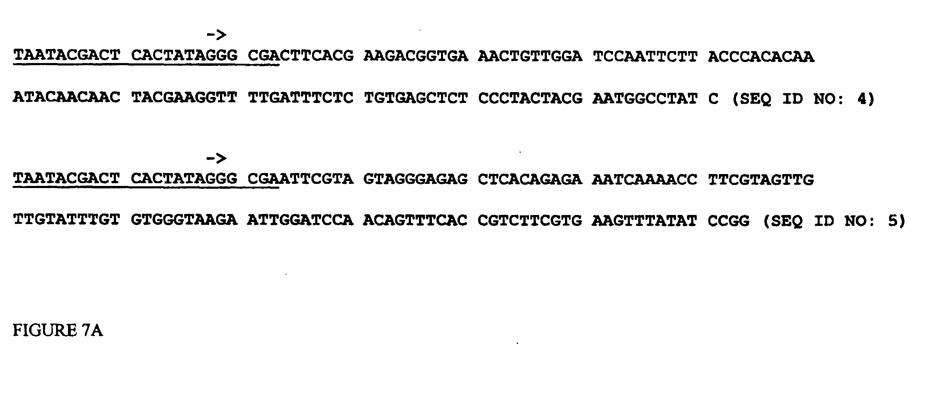

Figure 7A (SEQ ID NO: 4 and SEQ ID NO: 5) is a graphical representation of PCR products and transcripts used for diagnostic sequencing of the RNase-T1 coding region. Two parallel amplification reactions were performed with either the upstream or downstream primer tagged to the T7 promoter. The amplification products allow the transcription of the (+; upper sequence) or (-; lower sequence) strand. The underlined region shows the appended T7 promoter site. An arrow indicates the transcription initiation site.

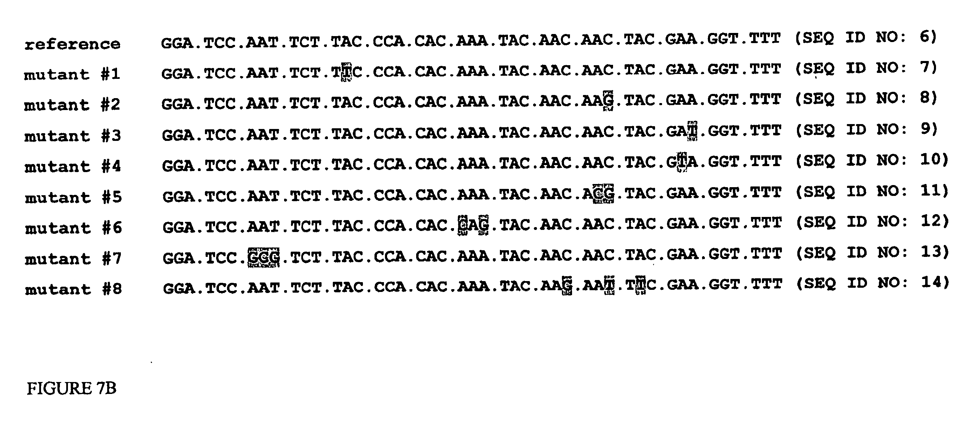

Figure 7B (SEQ ID NO: 6 through SEQ ID NO: 14)shows the position and nature of a number of single, double, and triple mutations in RNase-T1 (reference denotes the wild-type coding region).

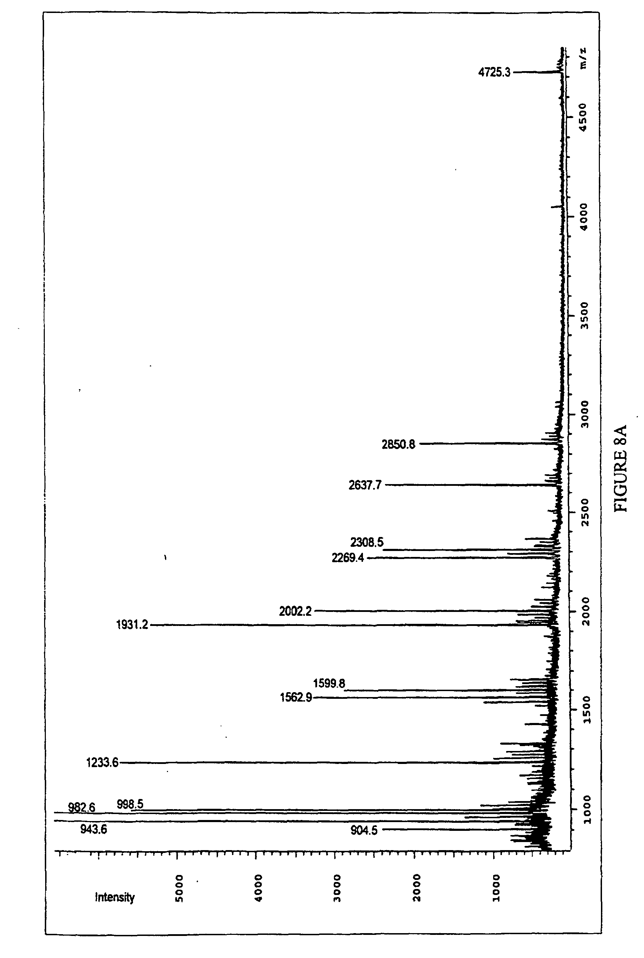

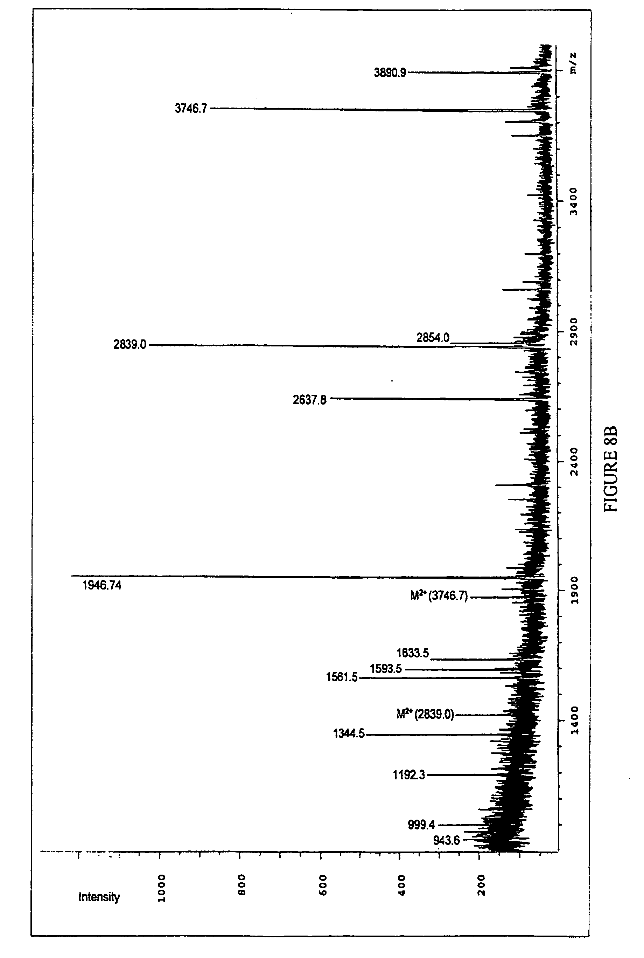

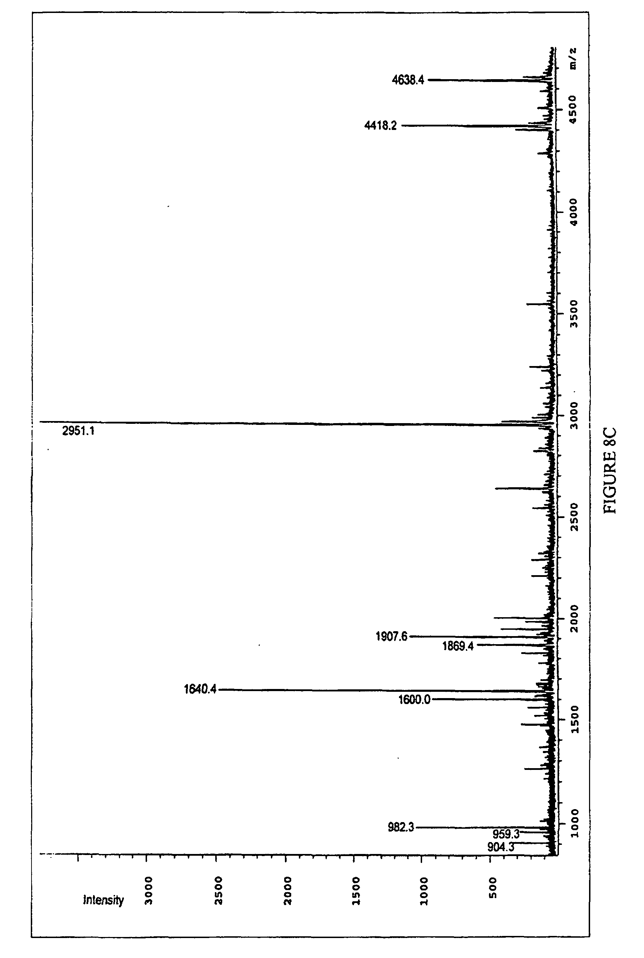

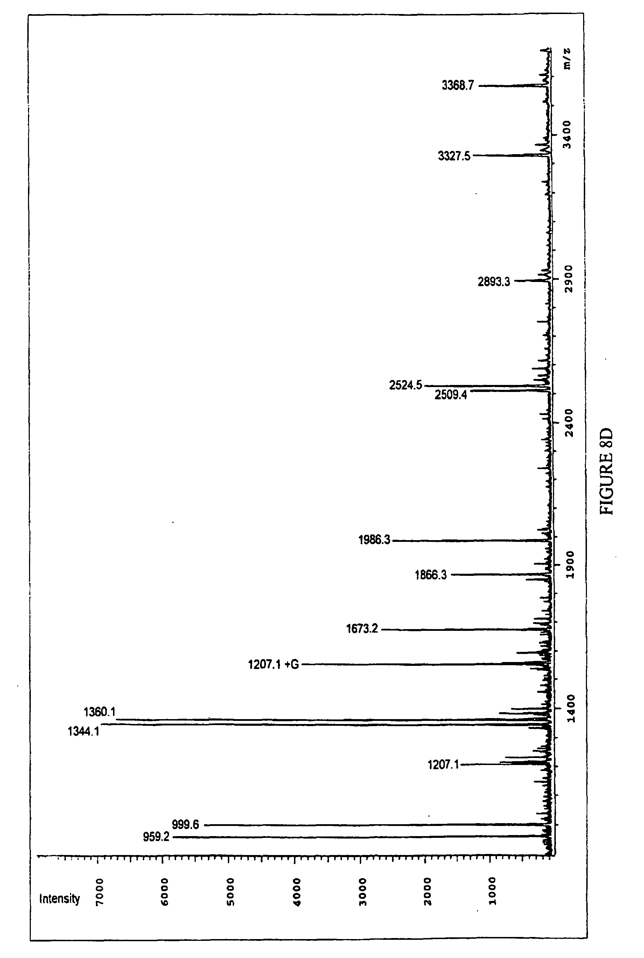

Figure 8 is a graphical representation of the MALDI-TOF mass spectra obtained for RNase-T1 analysis. Four transcripts were digested with RNase-A: (A) dU-incorporating transcript of the (+) strand, (B) dC-transcript of the (+) strand, (C) dU-transcript of the (-) strand, (D) dC-transcript of the (-) strand. The observed masses of predicted peaks are indicated. Presumed double protonated peaks are labeled M²⁺ with the mass of the parental [M+H]⁺ peak indicated between parentheses (Figure 8B). One of the peaks in Figure 8D (1207.1+G) is best explained by assuming the addition of an extra G-residue at the transcript 3'-end. Figure 8C only shows the 900-4800 Da mass range; the digestion product of 11124 Da was not detected.

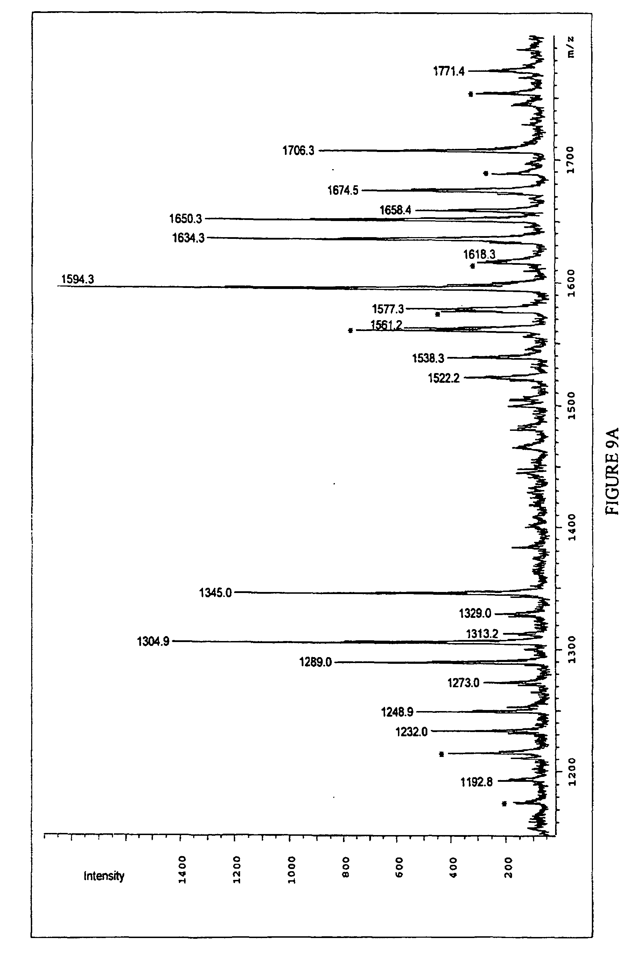

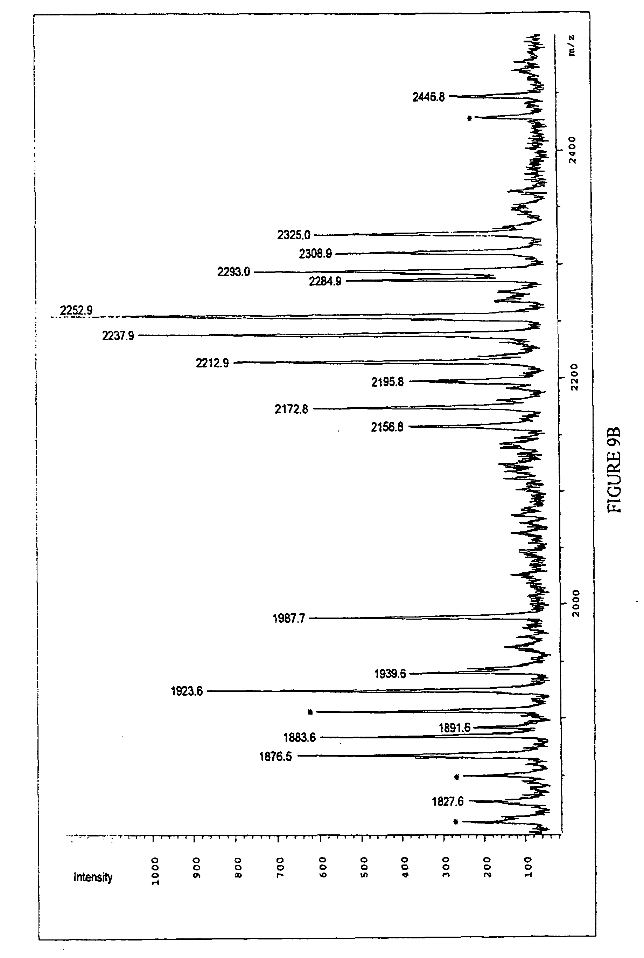

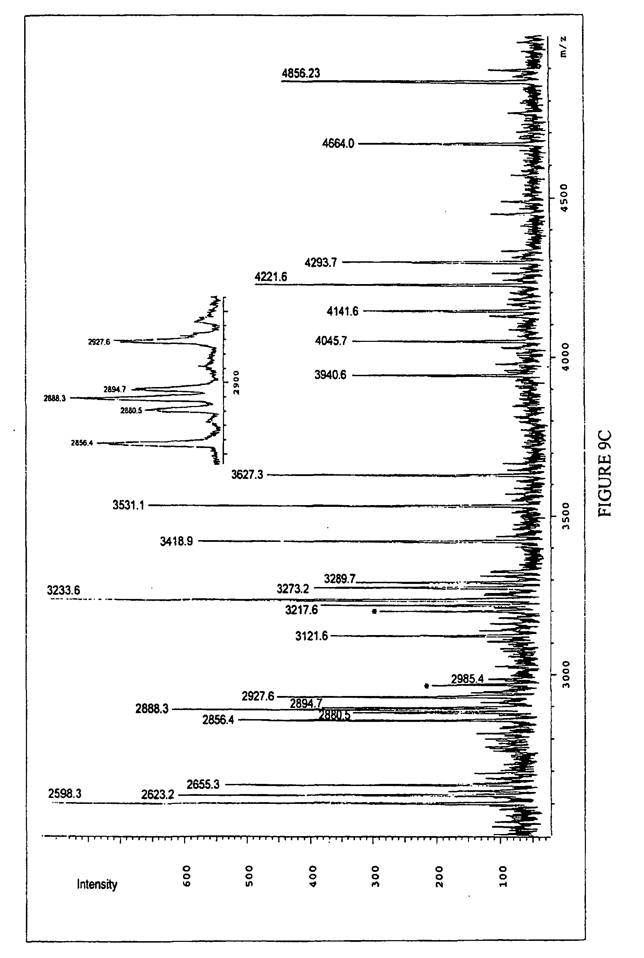

Figure 9 (panels A, B, and C) is a graphical representation of the MALDI-TOF mass spectra of the RNase-A cleavage reaction of a pGEM3-Zf(+) derived T7-transcript of 972 nucleotides long. The transcript incorporates dCMP instead of CMP residues. The observed masses of the predicted peaks is indicated. An asterisk indicates 2',3'-cyclic phosphate reaction intermediates (see Table V).

### DETAILED DESCRIPTION OF INVENTION

With current capabilities in mass spectroscopy, it is impractical to sequence nucleic acids greater than ∼50 bases in length. Consequently, an impractical and cumbersome number of independent sequencing reactions is necessary to cover the thousands of bases of a gene or other genetic region of interest. The methods of the present invention described below overcome this limitation. At the same time, the present method is distinct from the other fragmentation processes that are limited to screening target nucleic acids for a wide range of potential mutations. Indeed, the appropriate choice of complementary cleavage reactions as described herein allows the determination of the exact location and nature of a genetic variation. Also, it is demonstrated herein that computational protocols are an integral part of the described method. The methods and algorithms are required to deduce, on the basis of the reference sequence(s), the relation between (i) the spectral changes associated with one or more cleavage reactions of a given nature, and (ii) the uniquely defined sequence variations.

Sequencing reactions according to the methods of the present invention can be multiplexed, i.e. used for the simultaneous analysis of multiple non-contiguous target regions [*supra*]. Therefore, the methods can be adapted as a tool for the genome-wide discovery and/or scoring of polymorphisms (*e*.*g*. SNPs) useful as markers in genetic linkage studies. Indeed, it will be recognized that the unambiguous identification/diagnosing of a number of variant positions is less demanding than full sequencing and that consequently a considerable number of target genomic loci can be combined and analyzed in parallel, especially when their lengths are kept relatively small. The number of markers that can be scored in parallel will depend on the level of genetic diversity in the species of interest and on the precise method used to prepare and analyze the target nucleic acids, but may typically be in the order of a few tens to up to 100 or more with current MS capabilities. The addition of multiplexing to the high-precision and high-speed characteristics of MS constitutes a new marker technology that enables the large-scale and cost-effective scoring of several (tens of) thousands of markers. Some aspects of the application of the present methods to genome-wide genotyping are described in Example 5.

The present invention provides a mass spectroscopy (MS) based nucleic acid sequencing method that overcomes some of the drawbacks inherent in the prior art. In contrast to the previously described methods, the methods of the present invention do not require the generation of a ladder, *i*.*e*. an ordered set of nested nucleic acid fragments characterized by a common end. Rather, the disclosed methods rely on a combination of complementary fragmentation reactions and the analytical resolution power of MS to improve mass resolution and mass accuracy. The present invention is directed to the use of enzymatic cleavage protocols that result in the generation of cleavage products that range from mono- and dinucleotides to fragments of a few tens of nucleotides that are particularly suited for analysis by MS. According to the present invention, a combination of several different mass spectra, obtained after complementary digestion reactions, coupled with systematic computational analysis allows the survey of a selected nucleic acid or region thereof and leads to the unambiguous assignment of both known and previously unknown sequence variations.

The present invention is also directed to methods for the diagnostic sequencing (also referred to as re-sequencing) of all or part of a sample nucleic acid, *i.e.* the determination of the presence, the nature and the location of the sequence variations that occur relative to a related known reference sequence. The sequence variations may either be previously identified or hitherto unknown. Diagnostic sequencing according to the present invention may focus on particular positions in a nucleic acid sequence, *e.g.* when scoring previously known mutations or polymorphisms.

The term "mapping", as used herein, will be understood to include both the characterization, *i.e.* determination of the nature, and the position of the sequence variations.

The terms "target DNA", "target sequence", "target nucleic acid" and the like, as used herein, refer to the sequence region which is to be sequenced or re-sequenced entirely or in part as well as to the nucleic acid material that is actually subjected to one or more complementary cleavage reactions.

The terms "reference nucleic acid sequence", "related sequence", "previously known sequence", and the like, refer to a nucleic acid region, the sequence of which has previously been determined which corresponds to the target. The reference and target sequences may be found to be identical or may differ. The reference sequence need not derive from the same species. In many applications, several different sequence variants will be available as reference. The differences between a target sequence and its reference sequence may be simple (*e.g.,* single nucleotide substitutions, deletions and insertions; microsatellite polymorphisms) or complex (*e.g*., substitution, insertion, and deletion of multiple nucleotides). In certain situations, one may not know in advance to what reference sequence, if any, the target nucleic acid corresponds. In such situations the interrogated target sequence typically corresponds to a portion of a (much) larger reference sequence and/or to one out of a plurality of different references.

The terms "unambiguous", "unique", "unequivocal", and the like, are used to indicate that only a single sequence variation or combination of sequence variations can explain the observed mass spectral changes.

The terms "complementary (cleavage) reactions", "complementary cleavages" and the like, as used herein, refer to target nucleic acid digestions characterized by varying specificity [e.g., stringent or relaxed mono- and di-nucleotide specificity; digestion with a combination of reagents; partial cleavage] and/or to digestion alternative forms of the target sequence [*e.g.,* the complementary (+) and (-) strands; incorporation of modified subunits; analysis of variable portions of the target sequence].

The terms "transcript" and "transcription", as used herein, refer to the synthesis of a nucleic acid polymer by means of an RNA polymerase. In addition to canonical subunits (having a 2'-OH group), a transcript may incorporate non-canonical substrates (having any other substituent than a hydroxyl group at the 2'-position). Canonical and non-canonical substrates may contain additional modifications.

The term "genotyping," as used herein, refers to determining the genetic constitution, which is the particular set of alleles inherited by the organism as a whole, or the type of allele found at a particular locus of interest.

The term "expression profiling," as used herein, refers to method(s) for determining the mRNA expression profile of a given cell or a population of cells at a given time under a given set of conditions.

Nucleotides are designated as follows. A ribonucleoside triphosphate is referred to as NTP or rNTP; N can be A, G, C, U or m⁵U to denote specific ribonucleotides. Likewise, deoxynucleoside triphosphate substrates are indicated as dNTPs, where N can be A, G, C, T, or U. Throughout the text, monomeric nucleotide subunits are denoted as A, G, C, or T with no particular reference to DNA or RNA. When necessary, the nature of the nucleoside monophosphates is clarified by the use of more specific abbreviations such as U, m⁵U, CMP, and UMP to refer to ribonucleotides and dC, dU, dCMP, dUMP and dTMP to indicate deoxynucleotides. Note that T is not an alternative designation for m⁵U.

### Sequencing via non-ordered sets of specific cleavage fragments

The methods of the present invention allow the interrogation every position in a given target sequence without creating a fragment-ladder, *i*.*e*. a nested set of fragments that share one common endpoint. The method comprises, in part, subjecting one or more target nucleic acids to a set of complementary mononucleotide- and/or dinucleotide-specific cleavages, the products of which are analyzed by mass spectroscopy (MS). A preferred method according to the invention includes the specific cleavage of the one or more target nucleic acids at each nucleotide by way of two or more separate reactions. The digestion products obtained in mononucleotide- and dinucleotide-specific cleavage reactions such as those described herein range from mononucleotides to fragments of a few tens of nucleotides and are particularly well suited for analysis by MS. This aspect of the invention overcomes the technical limitation of the short read lengths encountered when analyzing fragment-ladders under the current MS performance. The mass spectra obtained with the methods do not provide a simple readout of the sequence. Computational approaches provided herein allow the comparative analysis of the obtained spectra with those known or predicted for the related reference sequence.

The ability to detect and map sequence variants based on the non-ordered set of cleavage fragments according to the present invention resides in part in the combination of the various complementary site-specific reactions. For example, one cleavage scheme useful in the practice of the present invention makes use of the mononucleotide-specific ribonuclease-T1 (RNase-T1, G-specific) and RNase-U2 (A-specific; the limited specificity of this enzyme is recognized and will be dealt with below). Both purines (A/G) and pyrimidines (C/T) in a target nucleic acid can be examined by cleaving an RNA copy of the two complementary strands of a target nucleic acid with both enzymes. MS analysis of the fragments generated by only a single mononucleotide-specific reaction would detect the presence of most sequence variations but only a minority of the mutations - in essence those affecting the nucleotide that is recognized - would also be localized. Since the methods of the present invention examine each of the four bases in a given sequence, each of the twelve possible nucleotide substitutions result in the loss of one cleavage site and the concomitant gain of another cleavage site. This principle is illustrated in Table I for the RNase-T1 and RNase-U2 cleavage reactions on the two complementary transcripts of a hypothetical target nucleic acid. Transitions affect both the RNase-T1 and RNase-U2 cleavage patterns of either the (+) or the (-) strand. As can be seen in Table 1, all transversions change the cleavage pattern of both strands of the transcript: they affect either one of the RNase digests on both strands, or the T1 digest of one strand and the U2 digest of the complementary strand. In addition to altering two cleavage patterns, each single nucleotide substitution also affects the molecular mass of one fragment in each of the remaining two digestion reactions (Table I). In conclusion, complementary cleavage reactions of the present invention results in a high degree of built-in redundancy. Each nucleotide substitution is potentially associated with a maximum of ten differences (data points) with respect to the reference spectrum. The loss and gain of a cleavage site are associated with both the disappearance and appearance of three peaks; two additional peaks undergo a shift as a result of a mass difference. In practice, the 1 Da mass difference between C and U(T) may result in the loss of a significant amount of information (Table I). More particularly, in G- and A-specific cleavage reactions, the C/U transitions may go unnoticed while the observed mass difference may not be unambiguously assigned to a certain transversion. However, in preferred methods of the present invention directed to the analysis of RNA target sequences the method makes use of C and/or U analogs that exhibit more favorable mass differences, thus allowing the unambiguous assignment of the mass difference to a particular transversion. Example 1 and Table I illustrate that 5-methyluridine is an example of such a useful analog [m⁵U; R.I. Chemical, Orange, CA; *see also* to Hacia J. *et al., Nucleic Acids Res.* **26:** 4975-4982 (1998) for the incorporation of m⁵UTP during *in vitro* transcription reactions].

Figure 1A shows, by way of example, a 120-nucleotide segment of exon 5 of the p53 gene as well as a graphical representation of the digestion products generated by RNase-T1 and RNase-U2 on an RNA copy of each strand. Figure 1B displays the size distribution of the base-specific digestion fragments derived from another exemplary sequence and illustrates that mono-, di- and tri-nucleotides are considerably more numerous than the larger digestion products. This distribution is expected for mononucleotide specific cleavage reactions that generate fragments with an average length of four nucleotides. Contrary to the size distribution, the number of different molecular masses that oligonucleotides can assume rapidly increases with the size of the fragment. Because of the constrained composition of digestion products (*e*.*g*. only one G in the case of RNase-T1), the number of molecular masses of mono-, di- and tri-nucleotides is limited to 1, 3 and 6, respectively. Consequently, mono-, di- and tri-nucleotides are often non-informative in the methods of the present invention because their number exceeds the limited mass space. Figure 1A illustrates that in certain parts of the target sequence one of the cleavage reactions produces many small fragments due to an over-representation of the recognized nucleotide and, consequently, yields virtually no information. However using the method of the present invention, this problem is minimized by the complementary nature of the four reactions which ensures that the fragments derived from the same region by the other digestions (interrogating under-represented nucleotides) are correspondingly larger. This indicates a basic attribute of the methods of the present invention. Each of the four cleavage reactions yields information about a particular mutational alteration (see Table I) and, in general, the redundancy in this information enables the identification of the mutation (nature and location) even when part of the information is missing from the spectra as described above.

The methods of the present invention are therefore largely, yet not completely, sequence-independent and permits the re-sequencing of virtually any variation. Computer simulations of diagnostic sequencing by the present methods, more particularly those involving digestion of RNA copies of each strand with the RNases T1 and U2, have shown that for target sequences of up to three hundred base-pairs ∼90% or more of all possible single nucleotide substitutions are associated with ≥4 data points. Fewer than 1% of the substitutions do not result in spectral changes. More than 95% of all possible single nucleotide substitutions give rise to unique spectral changes and can therefore be unambiguously identified (see Example 1 and Figures 3 and 4).

In summary, deduction of the sequence according to the methods of the present invention is based on the integration of the information that resides in a complementary set of 'mass-fingerprints' as well as the previous knowledge about a related reference sequence. The relationship between this multitude of data allows inferring the presence, nature and position of sequence variations in an unambiguous way. It is illustrative of the method that the derivation of the sequence is not critically dependent on the accuracy, *i.e.,* the absolute values of the mass measurements. It is rather the coherent ensemble of mass-shifts and appearances/disappearances of cleavage sites that uniquely define the sequence. The computer simulations, described herein, assumed a resolution of 5 Da or 0.1%, a figure which is well above what can be achieved with state-of-the-art equipment. Also, it should be pointed out that the determination of the correct base composition is limited anyway to short fragments, even in the case of high-precision measurements [*e.g*., 5-mers in the case of unrestrained sequences and if the measurement has an accuracy of 0.01% or better; Limbach P., *Mass Spectrom. Rev.* **15:** 297-336 (1996)]. Other methods in the art, which involve the accurate mass determination to assign the correct base composition to one or more fragments, will generally permit the detection of most sequence variations but not their unequivocal mapping. In these experiments it is generally assumed that a certain experimental observation relates to one particular previously known sequence variation, ignoring the fact that alternative sequence variations can explain the same result.

The present invention encompasses several additional embodiments and aspects described hereinafter and certain other embodiments will be readily apparent to one of ordinary skill in the art.

### Target nucleic acid preparation and fragmentation

### (a) Derivation of target nucleic acid and approaches to cleaving with base-specificity

Nucleic acid molecules can be isolated from a particular biological sample using any of a number of procedures, which are well-known in the art, the particular isolation procedure chosen being appropriate for the particular biological sample. To obtain an appropriate quantity of isolated target nucleic acid on which to perform the methods of the present invention, amplification of the target nucleic acid may be necessary. Examples of appropriate amplification procedures for use in the invention include but are not limited to: cloning [Sambrook *et al., Molecular Cloning: A Laboratory Manual*, Cold Spring Harbor Laboratory Press (1989)], polymerase chain reaction (PCR) [Newton C.R. and Graham A., PCR, BIOS Publishers (1994)] and variations such as RT-PCR [Higuchi *et al., Bio*/*Technology* **11:** 1026-1030 (1993)] and allele-specific amplification (ASA), strand displacement amplification (SDA) [Terrance Walker *G. et al., Nucleic Acids Res.* **22:** 2670-77 (1994)], and transcription based processes.

One embodiment of the present invention is directed to methods for sequencing (re-sequencing, etc.) Nucleic acid comprising the digestion of an RNA copy of each strand of the target nucleic acid with the RNases T1 and U2. One of the advantages of the method is the use of RNA, which exhibits higher sensitivity and better stability in MALDI-MS compared to DNA [Hahner S. *et al., Nucleic Acids Res.* **25:** 1957-1964 (1997)]. Typically, the first stage of this aspect of the invention involves the amplification of the target nucleic acid by PCR or reverse-transcription followed by PCR. (RT-PCR) This can be achieved with a pair of dedicated primers that incorporate promoter sequences as non-annealing 5'-extensions. In a second stage, these promoters are used for the specific transcription of the adjacent sequences including the target sequences. Preferably, the promoter sequences are small and permit the *in vitro* transcription by a single subunit cognate RNA polymerase such as those deriving from bacteriophage T7, T3 and SP6. Preferred for use in this aspect of the invention are C and/or U analogs that can be incorporated during transcription and that exhibit favorable mass differences [*e*.*g*. m⁵U; *supra*]. The use of PCR primers that carry different promoter sequences permits the generation of an RNA copy of both strands in two parallel strand-specific transcription reactions. Both strands may also be transcribed from the same promoter sequence: this requires two parallel amplification reactions with only one promoter tagged primer. Alternatively, the *in vitro* transcripts may also be produced from sequences cloned in special purpose vectors such as the pGEM-type vectors available from Promega (Madison, WI) which contain appropriate promoters. The third step further comprises the treatment of the resultant RNA transcripts with one or more complementary mononucleotide-specific RNases (e.g RNase-T1 and RNase-U2), such that each desired position in the target sequence is interrogated. The final step in the process consists of the mass-spectrometric analysis of the RNA fragments resulting from the complementary cleavage reactions and the comparison of the spectra obtained with those of the known reference sequence.

The target nucleic acid can be DNA, cDNA, any type ofRNA, DNA/RNA hybrid, or of mosaic RNA/DNA composition [depending on the ratio ofribo- and deoxyribonucleoside triphosphates (rNTP/dNTP) in the synthesis reaction; Sousa R. and Padilla R., *EMBO J*. **14:** 4609-4621 (1995); Conrad F. *et al., Nucleic Acids Res.* **23**: 1845-1853 (1995)]. The target sequence may also include modifications that are either introduced during or after enzymatic synthesis.

In general, different forms of each target sequence will be prepared so as to be able to perform a complementary set of mono-specific cleavage reactions. The cleavage reactions may be performed enzymatically and/or chemically. The mononucleotide-specificity of the digestion reactions may reside in the cleaving agent (*e.g* RNase T1), in the structure of the target nucleic acid, or in a combination of both. For example, RNase A (specific for both C- and U-residues) can be made monospecific by modifications of the substrate sequence that block the ribonucleolytic action at C or U residues. RNase A cleavage at U residues can in theory be prevented by chemical modification [Simoncsits A. *et al., Nature* **269:** 833-836 (1977)]. The enzymatic incorporation of nucleotide analogs, most notably those modified at the 2'-hydroxyl group of the ribose is particularly preferred in the practice of the invention. A variety of such analogs have been demonstrated to be substrates for T7 RNA polymerase; *e.g.* 2'-fluoro, 2'-amino [Aurup H. *et al., Biochemistry* **31:** 9636-9641 (1992)], 2'-*O*-methyl [Conrad F. *et al., Nucleic Acids Res.* **23:** 1845-1853 (1995)], as well as 2'-deoxy NTPs [Sousa R. and Padilla R., *EMBO J.* **14:** 4609-4621 (1995); Conrad F. *et al., Nucleic Acids Res.* **23:** 1845-1853 (1995)]. The above strategy may also be used to improve the specificity of certain RNases such as RNase U2 which is said to cleave GpN phosphodiester bonds in extensive digests [Brownlee G., in *"Laboratory Techniques in Biochemistry and Molecular Biology*" (Work T.S. and Work E., *eds*.), North-Holland, Amsterdam, pp 199-200 (1972)]. Mosaic DNA/RNA target sequences that incorporate only one specific rNTP and that can be obtained quite efficiently with particular mutant polymerases [Sousa R. and Padilla R., *EMBO J.* **14:** 4609-4621 (1995); Gao G. *et al., Proc. Natl. Acad. Sci. USA* **94:** 407-411 (1997); Bonnin A. *et al., J. Mol. Biol.* **290:** 241-251 (1999)], may allow mono-specific cleavages by alkaline treatment or by digestion with a non-specific RNase such as RNase-I [Meador J. *et al., Eur. J. Biochem.* **187:** 549-553 (1990)].

Alternative strategies to obtain selective cleavage of target sequences make use of phosphorothioate chemistry. DNA and RNA polymers with phosphorothioate internucleoside linkages in the Rp stereo-configuration are readily synthesized [*see* Eckstein F., *Ann. Rev. Biochem.* **54:** 367-402 (1985) and references cited therein]. Such phosphorothioate linkages can be specifically hydrolyzed following alkylation [Gish G. and Eckstein F., *Nucleic Acids Symp. Ser*. pp 253-256 (1987); Gish G. and Eckstein F., *Science* **240**: 1520-1522 (1988)]. Mono-nucleotide specific fragmentation according to this aspect of the invention would require the synthesis of targets making use of one particular α-thio nucleotide triphosphate substrate. Some nucleases (*e*.*g*. nuclease P1) cannot hydrolyze Rp phosphorothioate diesters; indirect selective cleavage (at a natural phosphodiester) may thus be obtained with target sequences that incorporate three different αS-dNTPs (or αS-rNTPs).

### (b) Alternative complementary reactions

The performance of the present sequencing methods will be understood by those skilled in the art to be dependent on the following interrelated factors: (1) the length of the region to be sequenced, (2) the resolution of the MS analysis, and (3), to some extent, the sequence itself. The longer the region of interest and, consequently, the larger the number of digestion products, the more important the resolution becomes. Also, the length of the region to be sequenced is directly proportional to the number of single nucleotide substitutions that cannot be unambiguously mapped on the basis of the four base-specific fragmentation patterns only (Example 1; Figure 4). Some sequence motifs are intrinsically difficult to sequence. An example of such a sequence is CTAGC₁C₂C₃C₄C₅GATC (SEQ ID NO: 15), where mutations at C₁ and C₂ cannot be discriminated from the same type of mutations at C₅ and C₄, respectively. Another such sequence is GAG₁A₂G₃A₄GA, where G₁->A cannot be discriminated from the G₃->A mutation; similarly, A₂->G and A₄->G cannot be distinguished. Finally, the four mono-nucleotide specific cleavages may also be insufficient to analyze complex sequence variations (see discussion below). Most preferably, therefore, the practicing of the present invention includes a computer-aided simulation of the re-sequencing strategy of the intended region. Such simulation and analysis will reveal possible problematic positions in the sequence and can be used to assess the usefulness of certain additional complementary cleavage reactions as countermeasures to overcome such sequencing difficulties.

One such measure consists of dividing the target region and deriving two or more (partially overlapping) segments (e.g., amplicons) from the sample nucleic acid rather than sequencing the target region as a whole. In addition to setting the length, this allows to exert some control over the composition. This would abrogate problems arising when the region of interest contains a duplicated segment. A second measure consists of carrying out one or more alternative or additional reactions involving target fragments that incorporate one or more modified nucleotides that exhibit different molecular masses such as is described above. Those of skill in the art will know of the existence of a wealth of mass-modified nucleotide analogs, many of which are useful and can be reconciled with the enzymatic procedures of the present method. The nucleotide analogs will differentially affect the masses of many of the digestion products and will therefore yield a significantly different spectrum that may reveal the required information. The analogs U and m⁵U [*supra*] exemplify this. Simulation studies (which model the present invention) have indicated that the use of U resolves certain sequence ambiguities observed with m⁵U (data not shown), while overall the latter nucleotide analog results in considerably fewer sequence ambiguities (*see* Example 1).

Another option consists of performing one or more additional reactions on the complementary strand. Compared to, for example, a G-specific cleavage of one strand, the C-reaction of the complementary sequence will yield a different set of fragments characterized by other mass differences. The effect of including reactions on the complementary strand of the target sequence is therefore similar to the use of nucleotide analogs.

Still another alternative provided by the present invention and which is useful in obviating the potential problems exemplified above includes using reactions with alternative specificities of cleavage. For example, partial base-specific cleavage can be achieved by changing the reaction conditions or by use of a specially prepared target wherein the cleavable and uncleavable (*e.g.* 2'-modified; *supra*) forms of one particular nucleotide occur randomly. Alternatively, instead of partial base-specific cleavages, one or more specific digestions characterized by a greater stringency can be performed (*e.g.* dinucleotide- or relaxed dinucleotide-specificity; *see* below). The digestion of the target sequence, in double stranded DNA form, with restriction enzymes is still another alternative provided by the present invention. Double digestion (*i*.*e*. a combination of two base-specific cleavages) of target nucleic acid alone or in combination with other digestion methods of the present invention also represents an informative alternative within the scope of the present invention.

The present invention may also involve the analysis of truncated target sequences. More specifically, cleavage of chain terminated sequences prepared, for example, by incorporation of a particular 3'-deoxy nucleotide substrate, will yield spectra that contain additional fragments when compared to the spectrum of the full target nucleic acid and will consequently provide additional information that will, in certain cases, allow a more unambiguous indemnification of sequence variation. This approach will be particularly useful for the characterization of lengthy digestion products or regions containing complex sequence variations.

### (c) Alternative complementary reactions: cleavage characterized by a greater than mononucleotide specificity

In still another of its embodiments, the method of the present invention also includes nucleolytic processes that are characterized by a dinucleotide- or a relaxed dinucleotide-specificity. Such stringency of cleavage will facilitate the analysis of longer target sequences because the size distribution of the resultant digestion products is even better suited for analysis by MS than fragments with an average length of 4 nucleotides that are generated by mononucleotide-specific cleavage. Useful in this aspect of the invention are, for example, restriction endonuclease reagents capable of cutting DNA at dinucleotide sequences such as those described by Mead D. *et al*., WO 94/21663 (PCT/US94/03246). RNases that preferentially hydrolyze pyrimidine-adenosine (CA and UA) bonds have also been identified which are useful in the practice of the present invention [*E. coli* RNase-M, Cannistraro V. and Kennell D., *Eur. J. Biochem.* **181:** 363-370 (1989); as in an endoribonuclease isolated from *Saccharomyces cerevisiae*, Stevens A. *et al., J. Bacteriol*. **164:** 57-62 (1985); and as is the *Enterobacter sp.* C-ribonuclease, described by Marotta C. *et al., Biochemistry* **12:** 2901-2904 (1973)]. As disclosed and exemplified in the present invention, the specificity of these enzymes can, if need be, essentially be restricted to CA- or UA-bonds by the use of target nucleic acids that incorporate dUMP (or dTMP) on the one hand and dCMP on the other hand.

Stringent or relaxed dinucleotide-specific cleavage may also be engineered through the enzymatic and chemical modification of the target nucleic acid. By way of non-limiting example, transcripts of the nucleic acid of interest may be synthesized with a mixture of regular and α-thio-substrates and the phosphorothioate internucleoside linkages may subsequently be modified by alkylation using reagents such as an alkyl halide (*e*.*g*. iodoacetamide, -iodoethanol) or 2,3-epoxy-1-propanol. The phosphotriester bonds formed by such modification are not expected to be substrates for RNases. Using this procedure, a mono-specific RNase, such as RNase-T1, can be made to cleave any three, two or one out the four possible GpN bonds depending on which substrates are used in the α-thio form for target preparation. The repertoire of dinucleotide-specific reagents useful in the practice of the present invention may be further expanded by using additional RNases, such as RNase-U2 and RNase-A. In the case of RNase-A, the specificity may be restricted to CpN or UpN dinucleotides through the enzymatic incorporation of the 2'-modified form of the appropriate substrates as described above. For example, to make RNase-A specific for CpG dinucleotides, a transcript (target) is prepared using the following substrates: αS-dUTP, αS-CTP, αS-ATP, and GTP. Thus, using the indicated methods described herein, it is possible to engineer all 16 dinucleotide specificities. However, not all dinucleotide-specific reagents described herein would be required if the complementary strand of the target nucleic acid is included in the analysis.

The strategy outlined above makes it possible to prevent cleavage within homopolymer tracts (stretches of A's, G's, C's or T's) by an RNase that is made specific (or is made specific as described above) for the repeated nucleotide. Indeed, incorporation of a particular αS-NTP, followed by alkylation, will selectively prevent cleavage within repeated stretches of that nucleotide, allowing cleavage to occur at the 3'-side of the last nucleotide in the repeat. Simulation studies, similar to those described in Example 1, have identified this as a particularly useful strategy. Sequence analysis by digestion of the two complementary strands with RNase-T1 and RNase-U2 yielded a 5- to 10-fold reduction in the number of ambiguous mutations when αS-GMP and αS-AMP were incorporated in the respective transcripts. These studies also suggest that the selective blockage of cleavage within repeats is accompanied by a relatively small increase in the average length of the digestion products, thereby resulting in considerably less loss of information.

Variations in the methods of the present invention may be found
1. the use of other or additional RNases (alone or in combination) having similar or alternative specificities;
2. the use of mutant or chemically modified RNases with useful characteristics vis-à-vis the methods of the present invention [see for example, Loverix S. *et al., Nature Struct. Biol*. **5:** 365-368 (1998) for an RNase T1 mutant that prefers the phosphorothioate analog over the natural phosphodiester substrate; *see also* Contreras R. and Fiers W., *FEBS Lett.* **16**: 281-283 (1971) for the production of limited digests with a chemically modified RNase];
3. the use of other nucleotide analogs that exhibit different masses and/or reactivities, including nucleotides that incorporate alternative isotopes; and
4. alternative specific fragmentation methods, either chemical [Maxam A. and Gilbert W., *Proc. Natl. Acad. Sci. USA* **74:** 560-564 (1977); Richterich P. *et al*., *Nucleic Acids Res.* **23:** 4922-4923 (1995)], or enzymatic.

### Multiplex reactions

In another embodiment, the methods of the present invention are directed to the simultaneous sequence determination of at least two non-contiguous regions in a sample nucleic acid. In contrast to traditional sequencing methods that generate a fragment-ladder (*i*.*e*. a nested set of fragments that share a common endpoint), the strategies outlined herein are equally useful for multiplex sequencing. Multiplex sequencing, according to the present invention, generally involves the co-amplification of selected regions of target nucleic acids. This can be achieved by using sets of dedicated primer pairs which flank or are co-terminal with a target nucleic acid to be amplified. Alternatively, the preparation of the multiple target nucleic acids comprises the concomitant amplification of restriction fragments derived from the sample nucleic acid. Some approaches are illustrated and exemplified in Example 5. A special case of multiplex sequencing consists of the simultaneous analysis of the two complementary strands of a double stranded target nucleic acid.

In yet another embodiment, the methods of the present invention can be used for the simultaneous sequence determination of the corresponding target region(s) of at least two biological samples. A sequence variation in one out of a pool of analogous target nucleic acids may go unnoticed when analyzing conventional sequence ladders by means of gel electrophoresis. With the present methods, a sequence variation will, as a rule, yield one or more distinct peaks in the various complementary mass spectra. This feature should allow the detection of mutations at a significantly lower ratio of mutant to wild-type allele and therefore permit the analysis of larger pools. The ability to pool renders the present methods useful for the discovery of sequence variations across particular target regions in a given population. For this application, typically 5-10 samples may be combined. In case the mutations have previously been identified, considerably more samples, e.g. several tens, can be combined. The characteristics that render the present method useful for the analysis of sample pools make the method also effective for the analysis of heterozygous samples (*i*.*e*., an equimolar mix of two alleles).

### Mass spectrometric methods

Mass-spectrometric methods useful in the practice of the present invention include ionization techniques such as matrix assisted laser desorption ionization (MALDI) and electrospray (ES). These ion sources can be matched with various separation/detection formats such as time-of-flight (TOF; using linear or reflectron configurations), single or multiple quadrupole, Fourier transform ion cyclotron resonance (FTICR), ion trap, or combinations of these as is known in the art of mass spectrometry. [Limbach P., *Mass Spectrom. Rev.* **15:** 297-336 (1996); Murray K., *J. Mass Spectrom.,* **31:** 1203-1215 (1996)].

Because the present methods generally require the analysis of complex oligonucleotide fragment mixtures, the MALDI approach, mostly resulting in singly charged molecules, is preferred over ES where significant multiple charging will further increase the number of spectral peaks. For the desorption/ionization process, numerous matrix/laser combinations can be employed.

### Sequence determination of simple versus complex variations

In another embodiment, the methods of the present invention are directed to the diagnostic sequencing of one or more target nucleic acids that, in comparison with a related reference nucleic acid, incorporates a sequence variation other than a single nucleotide substitution. Such a sequence variation can involve the deletion or insertion of one or more nucleotides as well as the substitution of multiple nucleotides.

Similar to single nucleotide substitutions, the insertion or deletion of a single nucleotide represents a simple sequence variation whose analysis using methods of the present invention is straightforward. Both of these types of sequence variations are associated with a characteristic set of (maximum nine) changes in the four complementary mononucleotide-specific fragmentation patterns. It will be understood that the methods of the present invention, similar to other sequencing methods, may not unambiguously locate the point of insertion or deletion when it concerns one nucleotide in a stretch of identical nucleotides. This, however, may be taken into consideration when performing a computer assisted analysis of whether the observed spectra relate in a unique way to a specific sequence variant in accordance with the practice of the present invention.

Analysis of a microsatellite DNA [also referred to as VNTR (variable number tandem repeat) or SSR (simple sequence repeat)] represents a special case whose analysis is readily achieved using the methods of the present invention. Although multiple nucleotides are involved with VNTRs or SSRs, the interpretation of the spectral changes on the basis of the known reference sequence is rather simple and the polymorphism (an altered number of repeat units) may readily be characterized.

The methods of the present invention may also be used to analyze more complex sequence variations such as those where multiple nucleotides are affected either through insertion, deletion, substitution or a combination thereof. The analysis of a number of double and triple mutants is described below in Example 3d. Multiple substitutions within a target sequence are also expected to be accompanied by a characteristic number of spectral changes. This number depends on whether the substitutions are adjoining or separated, as well as on the intervening sequence in case the mutations are separated. Single nucleotide substitutions, isolated by a sequence that contains at least one A, G, C, and T, are each associated with 10 spectral differences as outlined above. In general, the analysis of complex sequence variants will require (elaborate) computational approaches. One possible algorithm involves the comparison of the experimentally observed spectra with those generated on the basis of all possible sequences in the short region to which the sequence variation is confined. Such an algorithm will identify the sequence variant or, in case of ambiguities, the different matching sequences. This procedure illustrates that the present methods may be applied to the *de novo* sequencing of short regions of a target sequence. It will be recognized that, in practice, the experimental observations will not only set the boundaries but will also define the length of the variant region such that the algorithm need not consider insertions or deletions. Additional experimentally derived information, such as the absence of a particular nucleotide, can further limit the sequence space the algorithm has to explore. In particular applications, the complex sequence variants may be previously known and may thus be part of the set of reference sequences. In such cases, the experimentally observed spectra may be directly correlated to those predicted for the reference sequences. There would however still be a need to compute whether such correlation is unique. The advantage of previous knowledge is that the experimental approach can be adapted such that the output information indeed relates uniquely to the potentially occurring complex sequence variations.

### Computer algorithm

The present invention, in part, rests on the insight that computational analysis of the spectra obtained in a set of complementary cleavage reactions, and comparison of these data with the computationally predicted spectral changes from the known reference sequence, as illustrated herein, is an important step in the unambiguous determination of the presence, the nature and the location of sequence variations. More specifically, the computational approaches to simulate the experiment illustrated herein are necessary to determine whether a unique relation exists between the spectra obtained and a particular sequence variation. Accordingly, one aspect of the present invention contemplates a method which utilizes a computer algorithm or method capable of computing the spectral differences resulting from one or more nucleotide differences between the target nucleic acid and the reference nucleic acid, the method and algorithm comprising subjecting the reference nucleic acid and sequence variants thereof (*i*.*e*., target nucleic acid having nucleotide differences) to the different base specific cleavages to generate oligonucleotide fragments, computing the mass of each oligonucleotide fragment, generating the mass spectra of the oligonucleotide fragments from the reference nucleic acid and the sequence variants thereof for each of the base specific cleavage reactions, and matching these computationally derived mass spectra with the spectra obtained experimentally in the different base specific cleavage reactions.

In one preferred embodiment the computer algorithm is designed to systematically compute the spectra of all possible simple nucleotide variations of the reference nucleic acid, including but not limited to all possible single nucleotide substitutions, deletions and insertions. Since most of the genetic diversity found in living organisms involves single nucleotide variations, most of the experimentally observed sequence variations can be identified with the methods and algorithms of the present invention, meaning that one or more matches may be found between the observed spectra and the computationally derived mass spectra. In case a unique match is found, the sequence variation in the target nucleic acid is unique. When more than one match is found between spectra, the sequence variation cannot be established unambiguously.

It will be obvious to the person skilled in the art that different approaches may be used for performing the computational analysis, such as, but not limited to, performing the computational analysis on the complete reference sequence, or performing a serial computational analysis on segments of the reference sequence using, for example, a sliding window. The latter approach will enable the identification of different sequence variants occurring in different parts of the reference sequence.

In another embodiment, the methods and computer algorithms of the present invention are designed to explore all possible nucleotide sequences in a limited segment of the reference sequence. Such methods and algorithms may be used when the preceding approach fails to give a match, demonstrating that the sequence variation does not correspond to a simple nucleotide variation in the reference nucleic acid. This may be the case when more than one nucleotide change occurs within a short region, such that one or more cleavage products contain multiple nucleotide alterations. The region corresponding to these cleavage products can then be explored further by computing the spectra for all possible sequence permutations and determining the matching sequence. It is anticipated that given sufficient computing power, such methods and algorithms may be used for *de novo* sequencing using mass spectral data generated according to the present invention.

### Applications of the present methods

The methods of the present invention are particularly well suited for rapidly and accurately re-sequencing nucleic acids from a variety of biological sources including, but not limited to, plants, animals, fungi, bacteria and viruses. Re-sequencing implies the detection and mapping of both previously known as well as unknown sequence variations (*e.g.* mutations and polymorphisms) relative to a related reference sequence. One of the most notable distinctions with respect to conventional gel-electrophoretic analysis of fragment ladders, is that generally each particular sequence (variation) results in a distinct and characteristic set of (mass) peaks. This feature makes the present methods effective for the reliable scoring of heterozygous samples, the simultaneous sequencing of multiple target regions from a single biological sample (*i.e*., multiplexing), as well as the simultaneous analysis of the analogous regions from different samples (*i.e.,* pooling). The use of pools of individual samples should permit the cast-effective identification of previously unknown sequence variations in a population. This aspect of the invention properties makes the present methods valuable for clinical and public health studies. Very often such studies rely on samples (*e*.*g*., saliva, blood, swabs, paraffin-embedded tissue, biopsy material) that are cellularly and genetically heterogeneous and, consequently, require assays that can detect mutations at a low ratio of mutant over wild-type allele.

An additional advantage of the present methodology is that it can be tuned (by reducing the number of complementary cleavage reactions) such that the diagnostic sequencing is limited to particular positions in a target nucleic acid, a feature useful for the unambiguous scoring of previously identified mutations or polymorphisms. The processes described herein can be used, for example, to diagnose any of the more than 3000 genetic diseases currently known (*e.g.*, hemophilias, thalassemias, Duchenne Muscular Dystrophy, Huntington's Disease, Alzheimer's Disease and Cystic Fibrosis) or genetic defects yet to be identified. In addition, certain DNA sequences may predispose an individual to any of a number of diseases or conditions such as diabetes, artherosclerosis, obesity, various autoimmune diseases and cancer (*e.g.*, colorectal, breast, ovarian, lung). Depending on the biological sample, the diagnosis for a genetic disease or genetic predisposition can be performed either pre- or post-natally using the methods of the present invention. Re-sequencing of nucleic acids derived from infectious organisms using the methods of the present invention may reveal the basis of pathogenicity and may also be useful to identify the variation(s) that cause drug-resistance. For example, mutations in the protease/reverse transcriptase region of the human immunodeficiency virus (HIV) have been implicated in the decreased sensitivity towards the antiviral activity of protease and reverse transcriptase (RT) inhibitors. The re-sequencing of the nucleic acid encoding these viral domains is therefore of special interest to monitor disease progression (*see* Example 1). Similarly, sequencing, according to the present invention, may be useful to determine the antibiotic-resistance phenotype of certain bacteria [*e.g. Mycobacterium tuberculosis;* Head S. *et al., Mol. Cell. Probes* **13:** 81-87 (1999); Troesch A. *et al., J. Clin. Microbiol*. **37:** 49-55 (1999)].

In other embodiments, the present methods are directed to the identification and classification of target nucleic acids. Analyses according to the present invention characterize nucleic acids at a level essentially equal to sequence determination. Therefore, interrogated unknown sequences may be unambiguously identified by comparison of the obtained mass spectra with those known or predicted for a plurality of reference sequences. In this exercise, novel sequences that have no matching reference database sequence may also be found. The use of the methods for expression profiling (*i.e.,* the analysis of cDNA libraries) as well as whole-genome sequencing is exemplified in Example 6 and 7, respectively. Other applications include the determination of identity or heredity (*e.g.,* paternity or maternity).

### Kirs for practicing the invention

Kits for diagnostic sequencing of one or more target nucleic acids in a sample are also provided. In preferred embodiments, such kits comprise one or more reference nucleic acids, various reagents for sequence specific cleavage protocols, and computer algorithm(s). Such kits may optionally also contain nucleic acid amplification reagents. Additionally, the kits may contain reagents for the preparation of modified nucleic acids, including but not limited to modified nucleotide substrates. The kits may also contain buffers providing conditions suitable for certain enzymatic or chemical reactions. In addition, the kits may contain reagents, such as solid supports, for purposes of isolating certain nucleic acids and preparing nucleic fragments for mass spectrometric analysis.

The foregoing aspects of the invention are illustrative and should not be construed to limit the invention as set out in the appended claims. Variations in some aspects as well as alternative procedures will be readily recognized by one of ordinary skill in the art.

Example 1 describes modeling the diagnostic sequence analysis of a 1200 base-pair region of HIV-1 using methods of the present invention.

Example 2 describes methods for base-specific cleavage by modifying the nucleic acid template to be cleaved.

Example 3 illustrates the diagnostic sequencing of the RNase-T1 coding region according to the methods of the present invention.

Example 4 illustrates the analysis of a ∼1000 base-pair nucleic acid.

Example 5 illustrates the use of the present invention for genotyping, including multiplex genotyping.

Example 6 illustrates the use of the present invention for transcription profiling.

Example 7 illustrates the use of the present invention for whole genome resequencing.

### EXAMPLE 1

### Modeling the Diagnostic Sequence Analysis of a 1200 Base-pair

### Region of HIV-1

The methods of the present invention have been utilized on a 1200 base-pair sequence derived from human immunodeficiency virus type 1 (HIV-1; HXB2 isolate; Genbank accession number K03455; position 2161 to 3360). This sequence was used as a model in computer simulations to examine the overall performance of the method, as well as the occurrence of ambiguities. The selected region encompasses the entire protease gene and the first ∼270 codons of reverse transcriptase [compare with Hertogs K. *et al., Antimicrob. Agents Chemother.* **42:** 269-276 (1998)]. The genotyping/re-sequencing of this domain of clinical isolates of HIV is of special interest in order to monitor the emergence of drug resistance-associated mutations. Single as well as multiple changes have been implicated in the decreased sensitivity towards the antiviral activity of protease and RT inhibitors [Hertogs K. *et al., Antimicrob. Agents Chemother.* **42:** 269-276 (1998); Schinazi R. *et al., Int. Antivir. News* **4:** 95-107 (1996) and references cited therein].

The principal objective of the computer simulation was to examine the performance of the re-sequencing method for detecting and mapping SNPs. To this end we have performed computational simulation analyses in which we have systematically mutated each nucleotide one by one in the 1200 base-pair sequence. For each mutation we have calculated the molecular masses of the cleavage products that would be generated from a given segment of the sequence in the different four RNase digestion reactions, namely upon RNase-T1 and RNase-U2 cleavage of the (+) and (-) strands. The comparison of these masses with those of the reference cleavage products from the original sequence identifies the masses of the diagnostic fragments associated with each mutational change, *i*.*e*., fragments that either appear or disappear as a result of the mutation. The underlying assumption in this analysis was that in order to be measurable, the fragment must have a molecular mass different from those of the other cleavage products generated in the same reaction. Furthermore, we have assumed that the resolution of the mass spec analysis is limited to mass differences larger than either 5 Da or 0.1%. In other words fragments whose mass difference with other fragments in the same digest is smaller than 5 Da or 0.1% were not scored in the analysis. The quantitative aspects of a mass spectrum (*i*.*e*. peak heights) were not considered in the present simulation study. For each mutational change we have computed the number of fragments that are diagnostic for the presence of the mutation. Mutational changes were scored as detectable when there was at least one diagnostic fragment (showing a spectral change). In addition, we have examined whether the mutational changes can also be mapped unambiguously. To this end we have compared the sets of diagnostic fragments associated with each mutation. Mutations that yield unique sets of fragments can be mapped unambiguously, while mutations that give the same sets cannot be distinguished from one another.

In a first simulation analysis we have computed the fraction of SNPs that may be detected and mapped using respectively 1, 2 and 4 RNase digestion reactions. To this end we have performed a systematic single nucleotide substitution simulation on a 200-base-pair segment of the HIV sequence. For each of the four different RNase digestion reactions [RNase-T1 and RNase-U2 cleavage of the (+) and (-) strands] we have calculated the number of detectable diagnostic fragments and have analyzed whether these fragments are unique for each mutation. The results summarized in Figure 2 show that in each of the single RNase digest reactions a large fraction (55% to 85%) of the mutations are detected. In contrast, only a small fraction (20% to 30%) of these mutational variations can be mapped unambiguously. The principal reason is that many different mutational changes result in the same mass differences. The fraction of mutations that can be mapped increases to around 60% to 70% when the data of two RNase digest reactions are combined. The further combination of the data from the four different cleavage reactions allows 96% of the mutational changes to be positioned unambiguously and illustrates the advantages of the methods of the present invention. Close inspection of the sequence ambiguities reveals that about half of these involve C to U (or conversely A to G) transitions. Because the difference in molecular mass between C and U residues is only 1 Da, the mass difference in the cleavage products of the strand carrying the pyrimidine base is too small to be detectable. Consequently one might expect that these mutational changes may become detectable when using m⁵U instead of U. Computational simulations using m⁵U on the same 200 base-pair sequence shows that the fraction of mutations that can be mapped unambiguously increases to 98%. Consequently all further simulations are based on the use of the analog m⁵U. These results demonstrate that the four mononucleotide-specific RNase digests are both necessary and sufficient for re-sequencing of most sequences with a high degree of accuracy.

It will be obvious that the quality of the sequences obtained with the methods of the invention will be strongly influenced by the size of the sequence segments that are examined. Indeed, the larger the size of the segment, the larger the statistical chance that certain relevant diagnostic fragments may coincide with other cleavage products generated in the same reaction. We have therefore performed a systematic single nucleotide substitution simulation analysis on the 1,200 base-pair HIV sequence using different size segments, namely 100, 200, 300 and 600 base-pairs. In each simulation a total of 3,600 single mutational substitutions was analyzed. For each of the four different RNase digest reactions both the number and the patterns of the measurable diagnostic fragments were computed using the detection limits described above. Figure 3 shows the distribution of the number of diagnostic fragments obtained with the 3,600 mutational changes in the four different analyses. The results clearly indicate that a larger percentage of the single nucleotide substitutions is associated with fewer diagnostic spectral changes when using larger segments of DNA.

In each simulation we determined both the number of detectable SNPs as well as the fraction of SNPs that can be mapped unambiguously. The results of the computational simulations summarized in Figure 4 show that almost all the mutational changes are detected in the four different analyses. Of the 3,600 SNPs, the number that escaped detection were respectively 0, 1, 3 and 9 using 100 base-pair, 200 base-pair, 300 base-pair and 600 base-pair segments, respectively. In contrast, the fraction of mutational variations that can be mapped unambiguously decreases much more when using longer segments. While only 1% of the SNPs are ambiguous when analyzing 100 base-pair segments, that fraction increases to almost 10% with 600 base-pair segments. Close inspection of the ambiguities shows that the majority of these involve nearby (often adjacent) pairs of identical bases where the analysis can determine the nature of the mutation but fails to identify which of the bases is changed.

In conclusion, the results of the simulations show that the methods of the invention are effective for re-sequencing and that even large segments may be used when only a limited number of positions need to be analyzed. Also, it appears that in most cases a computer-aided simulation study will be essential in the experimental design as well as the data interpretation when using the methods of the present invention. Most importantly, the simulations will indicate whether spectral changes are unambiguously linked to particular sequence variations.

### EXAMPLE 2

### Base-Specific Cleavage by Modification of the Template

The present example illustrates that the specificity of cleavage by a nucleolytic reagent may be further confined through the modification of the target template such that particular phosphodiester bonds resist cleavage. More particularly, it is demonstrated that RNase-A, which normally cleaves at the 3'-side of both C- and U-residues, becomes mononucleotide-specific when the target incorporates the 2'-deoxy analog of one of these nucleotides. A region of the plasmid vector pGEM3-Zf(+) (Promega, Madison, WI), encompassing the multi-cloning site as well as the phage T7 promoter sequences, was used as a model *(see* Figure 5).

The first step towards the sequence analysis according to the present invention involved the amplification of the 158 base-pair test sequence. The reaction was carried out in a total volume of 50 l using 12.5 pmol each of the forward and reverse primer, 200 M of each dNTP, 0.25 l Taq DNA polymerase (5 U/l; Promega, Madison, WI), 1.5 mM MgCl₂ and a buffer supplied with the enzyme. After an initial incubation at 94°C for 2 min, 40 cycles of the following temperature program were performed: 94°C for 30 sec, 50°C for 30 sec, and 72°C for 15 sec. The sample was kept an additional 15 min at 72°C and then chilled. The PCR reaction product was purified (High Pure PCR Product Purification Kit; Roche Diagnostics Belgium, Brussels, Belgium) and subsequently used for transcription of one specific strand. A mutant T7 RNA polymerase (T7 R&DNA™ polymerase; Epicentre, Madison, WI) with the ability to incorporate both dNTPs and rNTPs was used in the transcription reactions. In addition to a transcription with the regular ribonucleotide substrates, one reaction was performed where CTP was replaced by dCTP, while in two more separate transcriptions either dUTP or dTTP replaced UTP. The transcription reactions were run in a 50 l volume containing: 40 mM Tris-Ac (pH 8.0), 40 mM KAc, 8 mM spermidine, 5 mM dithiothreitol, 15 mM MgCl₂, 1 mM of each rNTP, 5 mM of dNTP (in these cases the appropriate NTP was excluded), ∼40 nM DNA template (∼2 pmol), and 250 units T7 R&DNA™ polymerase. Incubation was performed at 37°C for 2 hours. After transcription, the full-length T7 *in vitro* transcripts (118 nucleotides) were purified by allowing them to anneal to the 5'-biotinylated form of the complementary reverse PCR primer (Figure 5) followed by capture of the biotinylated annealing products onto streptavidin-coated magnetic beads. To this end, 50 pmol biotinylated reverse primer was added to the transcription reactions. The mixtures were first incubated 5 min at 70°C and, subsequently, ∼30 min at room temperature. Then, a slight excess of Sera-Mag™ streptavidin magnetic microparticles [Seradyn Inc, Indianapolis, IN; resuspended in 50µl of 2 M NaCl, 20 mM Tris-HCl (pH 8.0), 2 mM EDTA] was added and the resultant mixture incubated at room temperature for 30 min with agitation. A magnetic particle collector (MPC; Dynal, Oslo, Norway) was used to collect the beads, remove the supernatant and, subsequently, to wash the beads three times with 100 l 100 mM (NH₄)₃-citrate. The beads were finally resuspended in 3 l 25 mM (NH₄)₃-citrate containing 0.5 g bovine pancreas RNase-A (50 U/mg; Roche Diagnostics Belgium, Brussels, Belgium) and incubated at room temperature for about 30 min to digest the transcripts to completion. 1 l of this RNase reaction was removed and added to 5 l matrix solution. This 1:1 acetonitrile:H₂O matrix solution is saturated with 3-hydroxypicolinic acid (∼100 mg/ml), and further contains 25 mM (NH₄)₃-citrate, (occasionally) 2 pmol/ l of an oligonucleotide serving as an internal standard, and cation-exchange beads in (NH₄)⁺-form (Dowex 50W-X2; Sigma, Saint-Louis, MO) to minimize the presence of sodium and potassium adducts. After incubating the mixture at room temperature for 15 min, 1 l was put on the sample plate and allowed to dry. Mass spectra were collected using a Reflex III mass spectrometer (Bruker Daltonik GmbH, Bremen, Germany).

The RNase-A cleavage products predicted for each of the four transcripts are shown in Table II. Note that the mass calculation of the predicted fragments assumes a 3'-phosphate group and not the 2',3'-cyclic phosphate intermediate of the cleavage reaction. Overall, the experimentally obtained spectra (Figure 6) are in excellent agreement with the predictions. The absence of some of the smallest 3-mers (Figure 6A and 6C) may be related to the mass-gate that was applied to eliminate the non-informative mono- and di-nucleotide digestion products. The predicted 3'-proximal fragment TGTTTC (1830, 1 Da) is only poorly ascertained in Figure 6C, *i.e.,* the spectrum deriving from the dU-transcript. This result, along with other observations, suggests that fragments with a relatively high dU-content are detected with a significantly lower sensitivity using the present MS methodology. The 2817 Da peak in Figure 6D corresponds to the double protonated form of the added oligonucleotide. Some of the expected fragments cannot be resolved because they have an identical composition. Also, the digestion products of the regular transcript that differ by one Da only *(e.g.* the difference between CMP and UMP; Table II) cannot be seen as distinct peaks in Figure 6A. In total, the data convincingly demonstrate that RNase-A behaves as a C-specific RNase when dTTP or dUTP is substituted for UTP, and as a U-specific reagent when dC rather than C is incorporated into the substrate transcripts. This high level of nucleobase specificity is achieved even under the over-digestion conditions used in the present Example.

The protocol described in the present Example is illustrative and certain modifications and variations will occur to the skilled artisan. The immobilization of the transcripts represents an easy means to prepare the material for MS analysis, *e.g.*, removal of all other reaction components and exchange of Na⁺ and K⁺ counter-ions for (NH4)⁺ (note that the subsequent RNase digestion does not require any reagents that are 'incompatible' with MS). While other methods, such as chromatography, may be used to prepare the transcripts or the derived digestion products for MS analysis, the present method is favorable in that it is readily amenable to automation and high-throughput analysis. In repeat experiments, yielding essentially the same results as described herein, the transcripts were digested in water and ∼15 nanoliter of these digests was directly applied onto a Spectrochip™ (Sequenom Inc., San Diego, CA) for analysis by MALDI-TOF-MS.

### EXAMPLE 3

### Diagnostic Sequencing of the RNase-T1 Coding Region

The present example illustrates the application of the methods of the invention to the re-sequencing of a portion of the RNase-T1 coding region. We selected the RNase-T1 coding region because of the availability of a collection of site-directed mutants [Steyaert J., *Eur. J. Biochem.* **247:** 1-11 (1997)] which had previously been sequenced using the classical dideoxy chain termination method. The wild-type and mutant sequences, used in the present example, are shown in Figure 7.

### a. Analysis of the wild-type RNase-T1 sequence

The experiments were performed essentially as described in Example 2. First, the selected wild-type RNase-T1 target sequences were amplified by PCR with the following primers: The resultant amplicon was subsequently re-amplified using either a forward or a reverse primer that incorporates the T7 promoter site as a 5' non-annealing extension (*see* Figure 7A): Subsequently, each of the resultant promoter-appended amplicons was used as template in two separate transcription reactions. The T7 R&DNA polymerase (Epicentre, Madison, WI) was used to prepare transcripts that incorporate dCMP or dUMP instead of respectively CMP and UMP (referred to as the dC- and dU-transcripts). The transcription reactions were carried out as described in Example 2, except that each rNTP was present at 2 mM and incubation was performed overnight at 37°C. The four full-length T7-transcripts were purified by annealing with a biotinylated oligonucleotide that matches with the transcript 3'-end *(i.e.* the biotinylated form of either the forward or the reverse PCR primer used in the first amplification step) and subsequent capture onto streptavidin microparticles. After extensive washing with (NH₄)₃-citrate, the transcripts were eluted. The beads were resuspended in 3 l of water and kept at 90°C for 2 min, immediately followed by collection of the beads with the magnet and transfer of the supernatant to a fresh tube. Then, the obtained amplified target nucleic acids were digested to completion by the addition of 1 l of 100 mM (NH₄)₃-citrate containing RNase-A. Finally, the reaction products were analyzed by MALDI-TOF-MS.

A graphical representation of the spectra is shown in Figure 8A-D. The predicted degradation products are listed in Table III. As with the pGEM3-Zf(+) transcripts the obtained spectra are in good agreement with the predictions. A few peaks that are most likely the result of double protonation were also observed (see Figure 8B). The T-reaction on the (-) strand suggests the occurrence of transcripts with an extra non-template encoded nucleotide at the 3'-end [Milligan J. *et al., Nucleic Acids Res*. **15:** 8783-8798 (1987)]. Indeed, in addition to the expected 3'-terminal fragment, a prominent peak is observed that coincides with the same fragment containing an extra G-residue (Figure 8D and Table III). The absence of the expected 3'-terminal fragment from the C-reaction on the (+) strand (1153 Da; Figure 8A) may be explained by this same phenomenon. In this case, cleavage of the 3'-extended transcript would occur and result in the 3'-phosphorylated (rather than the 3'-OH) form of the predicted fragment, a product which would coincide with another fragment of the same digestion (1233.7 Da; Table III).

### b. Analysis of selected RNase-T1 single point mutations

Four single nucleotide substitutions were chosen (mutant #1, #2, #3, and #4 in Figure 7B). Each of the mutant sequences was analyzed as described for the wild-type RNase-T1 coding region (Example 3*a*). The results are summarized in Table IV. Table IV shows, for each mutation, which 5 fragments of the wild-type RNase-T1 reference sequence are affected by the mutation as well as the 5 fragments that are mutation-specific. It also shows which changes are missing, and consequently on how many, out of the ten theoretical data points, the mutation identification is actually based. Spectral changes are missing because they involve fragments that are too small (<3-mer) or not unique. Also, a few fragments were not experimentally observed, *e.g.,* one 3-mer as well as the largest fragments with a mass of ≥9,8 Kda. Of particular interest are the results concerning mutation #2. These results indeed best illustrate the present invention. In this particular case, all four mono-nucleotide specific cleavage reactions result in the detection of a mutation, *i.e*. one will notice that the sequence differs from the wild-type RNase-T1 coding region. However none of these reactions, when taken alone, leads to the unambiguous mapping of the mutation. The C-reaction on the (+) strand results in a new fragment of 1947 Da. Not only the single nucleotide mutation #2 can explain the creation of such a 6-mer [composition = A₃G(dU)C]. For example, this is also the case for a double mutation that converts the sequence CTACTAC into CAAGTAC *(see* Figure 7); the TAC peak will not be lost because of the presence of a third such 3-mer. The T-reaction on the (+) strand results in a spectrum where the mass of one fragment has increased by 56 Da when compared to the reference spectrum. This suggests the replacement of a dC by a G. Because the cleavage product contains three dC residues, it is not possible to position the substitution. The C-reaction on the (-) strand is at first sight the most informative; a large reference fragment is affected by the cleavage. The sequence of the fragment (GTAG₁TT---TG₂GATC)(SEQ ID NO: 20) is however such that both the G₁->C and the G₂->C mutation can explain the observed products of 9814 Da and 1289 Da [composition = GA(dU)C]. Finally, the T-reaction on the (-) strand is the least informative and the appearance of a peak of 944 Da [A(dC)U] can be explained in many different ways. An A(dC)U-fragment is, for example, generated by substitution of the T₁-residue for a C in the sequence stretch TAT₁TT (*see* Figure 7). In conclusion, mutation #2 exemplifies that in some cases the nature and position of a sequence variation may only be determined by a combination of at least two different complementary cleavage reactions.

### c. Analysis of a mixture of wild-type and mutant RNase-T1 sequences

The analyses shown in Table IV can be used to simulate experiments where equimolar mixtures of the wild-type RNase-T1 sequence and one of the single nucleotide substitutions are examined. In such cases, which mimic heterozygotic genotypes, the spectra contain a number of novel fragments in addition to all those derived from the (wild-type) reference sequence. The characterization and location of the mutation/polymorphism is therefore necessarily based on the novel fragments only. Unambiguity requires that the novel fragments are sufficient to uniquely define the mutation. Those of skill in the art will realize that zygosity determination is straightforward using the present methods because each allele is associated with a distinct set of peaks.

We performed a number of experiments where on particular single nucleotide mutant (*e*.*g*., mutant #3; Figure 7B) was mixed with wild-type RNase-T1 such that the mutant allele was present at the following fractions: 1:2. 1:5, 1:10, 1:20, 1:50,1:100,1:200,1:500 and 1:1000. the experiment mimics the analyses of pools of samples characterized by different allele frequencies. First, equivalent quantities of the wild-type and mutant target sequences were synthesized by PCR amplification using conditions where the primers are limiting and completely consumed. After mixing the two amplicons in the desired ratios, the material was re-amplified. Then, transcripts of the (-) strand were prepared and digested as described above, except that transcriptions were performed using all four nucleotide triphosphate substrates in the ribo-form (rNTPs) and that cleavage was carried out with RNase-T1 instead of RNase-A. Each of the digestion reactions was measured 5 times. Cleavage with the RNase-T1 enzyme generates a polymorphic 15-mer fragment which reads: AAAUCAAAACCUUCG(SEQ ID NO: 21), where the underlined residue is changed to A by mutation #3 (*refer to* Figure 7A and 7B). The mass of the wild-type and the mutant fragment is 4807,91 Da and 4830,95 Da, respectively; the mutation causes a shift of 23 Da. We found that there was an excellent linear correlation between the allele frequencies and the relative peak heights (R²=0,97) and that the peak associated with the mutant allele could still be identified with confidence when it represented 5-10% of the material. It should be noted that in other experiments the minimum ratio of mutant over wild-type allele that can be detected might be significantly lower. Indeed, in the present example, the reliable detection of the 'mutant peak' was somewhat encumbered by the occurrence of an extra peak as evidenced by the control spectrum recorded for the wild-type target nucleic acid. This extra peak may possibly be attributed to a low level of Na⁺-adduct of the wild-type fragment (22 Da mass shift). In all, the latter data indicate that homologous target nucleic acids can be pooled and analyzed simultaneously; in addition to revealing certain sequence variations, the methods of the present invention may permit the allele frequencies to be estimated among the pool of biological samples. While diagnostic sequence determination as disclosed herein relies primarily on the appearances and disappearances of peaks as well as peak shifts, the present example indicates that certain quantitative aspects of a spectrum *(e.g.,* peak height and peak area) can be included in the sequence analysis and yield complementary valuable information.

### d. Analysis of RNase-T1 multiple mutants

The methods of the present invention are not limited to the analysis of single nucleotide substitutions. Complex variations can also be sequenced. Table IV lists the spectral changes that are predicted to be associated with a number of RNase-T1 multiple mutants, more particularly double and triple mutants (mutant #5, #6, #7, and #8 in Figure 7B). As described above, multiple mutants are associated with a characteristic number of spectral changes. In the case of multiple substitutions, with no deletions or insertions involved, the number of affected reference fragments is always identical to the number of novel fragments. For double mutants the number of spectral changes ranges from 12, in case the mutations are adjoining (mutant #5), to a maximum of 20, in case the mutations are separated by a sequence that contains at least one A, G, C, and T. In the latter case, the double mutant is to be treated as two concurrent but independent single nucleotide substitutions. Triple mutants are associated with a minimum of 14 spectral changes (mutant #7). As with single nucleotide substitutions, not all the theoretical spectral changes can or may be observed and part of the information will be lost. In the vast majority of the cases however a systematic computational analysis, based on the obtained spectra and the reference nucleic acid sequence(s), can unambiguously identify and locate the sequence variations.

### EXAMPLE 4

### Mass Spectrometric Analysis of a ∼1000 Base-Pair Region

The methods of the invention are designed to overcome the limitation of the short read lengths encountered with current MS-based sequencing methodologies that involve the analysis of fragment-ladders. One can envision that, depending on the application, target regions of several hundred or even a few 1000 base-pairs can be analyzed. The present example demonstrates that a large number of oligonucleotide fragments can be analyzed simultaneously by the methods of the present invention and that, consequently, the detection platform does not impose a limit on the methodology.

Following the scheme presented in Example 2, a 1012 base-pair region of the plasmid vector pGEM3-Zf(+) (Promega, Madison, WI) was amplified and the resultant amplicon, subsequently, used for preparation of a 972 nucleotides long *in vitro* T7 transcript (see Figure 5). The transcript incorporated dCMP instead of CMP such that a U-specific cleavage could be performed by RNase-A. The cleavage products predicted for this transcript, are listed in Table V. Figure 9 shows the most relevant parts of the experimentally obtained spectrum. The primary conclusion from the experimental data is that complex mono-nucleotide specific digestion reactions, consisting of>200 cleavage products, can be analyzed by mass spectrometry. The vast majority of the about 67 predicted distinct peaks are readily identified. Only a few of the 4-mer fragments are not or barely detectable. It also appears that in the present experiment the assignment of some peaks requires the assumption that (at least a portion of) certain digestion products contains a 2',3 '-cyclic phosphate instead of a 3'-phosphate group. Such peaks differ from the parent peaks by -18 Da. It is well known that cyclic phosphates result from the transesterification cleavage reaction and that these intermediates get hydrolyzed in a slower second reaction step.

### EXAMPLE 5

### Genotyping

The methods of the present invention are also useful for the diagnostic sequencing of multiple non-contiguous regions of a sample nucleic acid. This renders the present methods useful for the genome-wide discovery as well as the routine scoring of polymorphisms (*e*.*g*. SNPs) and mutations at multiple loci in genomic DNA. Such multiplex genotyping is conceptually no different than re-sequencing; both require that alterations are characterized and positioned unequivocally. Similar to experiments involving a single target sequence described above, a computer simulation can be performed to find out which ones of the observed spectral changes is uniquely linked to particular genomic alterations. Since multiplex genotyping only requires the identification/diagnosing of a number of variant positions, it will be recognized by those of skill in the art that (i) the complexity (*i.e.* the combined length) of the multiple target sequences may be significantly greater than in the case of full re-sequencing, and (ii) a single specific cleavage reaction may often suffice for both allele and zygosity identification. Applications which involve the use of two sequence-specific cleavages that each positively identify one of the two alternative forms of a series of bi-allelic SNPs are also possible using the methods of the present invention. For example, many C to T transitions, the most common type of point mutations and polymorphisms in human, may be easily scored by a combination of C- and T(U)-specific reactions. It is worth mentioning that heterozygous samples analyzed using gel-electrophoretic sequencing are often difficult to identify with confidence. With the methods described herein, the detection of heterozygosity is unambiguous because of the presence of both the wild-type and the mutation specific set of mass spectral peaks.

Multiplex genotyping will generally involve the co-amplification of genomic regions. In the case of previously known SNP genetic markers, co-amplification of selected loci can be achieved by using dedicated primer pairs [Wang *et al., Science* **250:** 1077-1081 (1998)]. Alternatively, a more generic approach can be adopted for both the discovery and the subsequent routine scoring of a set of SNPs where the preparation of target sequences comprises the concomitant amplification of multiple short restriction fragments derived from the sample nucleic acid. This 'random sampling' method may be particularly useful with organisms that have a high polymorphism content *(e.g.,* more than 1 SNP in 100 base-pairs). This co-amplification can be achieved by ligating to the ends of the restriction fragments adaptor sequences that incorporate the target sites for a single PCR primer pair. In this approach, the average size of the amplicons must be small such that the majority incorporates ≥1 SNP while, additionally, the total number of the amplicons must be sufficiently small so that their combined length is amenable to analysis by the present methods. These requisites can be met by the appropriate choice of restriction enzymes and the use of methods that permit the selective amplification of discrete subsets of restriction fragments [Vos P. *et al., Nucleic Acids Res.* **23:** 4407-4414 (1995); Zabeau M. and Vos P., EP 0534858 (1993); Kikuya Kato, *Nucleic Acids Res.* **23:** 3685-3690 (1995)] and as described herein. For example, a first restriction enzyme that cleaves rarely in the genome under study can be combined with a second reagent that generates fragments with an average size of about 100 base-pairs *(e.g.,* a combination of two enzymes with tetra-nucleotide recognition sites). The number of fragments edged by the two different restriction sites should preferably be less than 100,000; a suitable subset of these can readily be amplified by the use of selective primers [Vos P. *et al., Nucleic Acids Res.* **23**:4407-4414 (1995)]. In addition, a PCR protocol, characterized by a highly shortened elongation time, can be used such that the amplification of short fragments is strongly favored thereby further reducing the number and the average size of the amplicons. During the selective co-amplification of genomic fragments or in a subsequent PCR step, a first primer can be used that attaches a full promoter sequence *(e.g.,* one deriving from bacteriophage T7, T3 or SP6; *supra*) to the amplicons. The second strand may be synthesized by extension of a primer that contains a ribonucleotide residue at, for example, the penultimate position. Following PCR amplification, the primer sequences can be removed from this second strand by RNase digestion, and the resultant truncated strand transcribed with the aid of the first primer. This procedure minimizes the common sequences that are connected to the target restriction fragments.

### EXAMPLE 6

### cDNA Library Analyses - Transcription Profiling

Diagnostic sequencing will, generally, be performed on a defined nucleic acid, *i*.*e*. one will know to what reference sequence the target nucleic acid corresponds. However, the re-sequencing methods according to the present invention can also be used to identify or classify certain sequences. In such experiments, the interrogated nucleic acid *(e.g.* a random clone of DNA) will typically correspond to an unknown portion of a (much) larger sample sequence or represent one out of a plurality of nucleic acids present in a biological sample, or a combination of both. The mass spectra derived from the unknown nucleic acid are compared to those known or predicted for the related reference sequence(s), or portions thereof. Note that, in this type of experiments, some of the interrogated target sequences need not necessarily have their counterparts in the reference sequences, and vice versa. It will be realized that sequence identification according to the present methods may, at the same time, reveal possible sequence variations. Interrogated sequences may thus be classified as identical to one of the database sequences, as a variant of such as a reference sequence or as novel in case no matching sequence is found.

It should be recognized that analyses that involve at least the four complementary mono-nucleotide specific cleavage reactions identify unknown sequences with a resolution essentially equal to sequence determination. At the same time, the MS-based methods described herein allow fast data acquisition and are amenable to high-throughput. Therefore, the present methods are useful to identify and catalogue nucleic acids at an unprecedented scale and speed. One application consists of the analysis of cDNA libraries for the purpose of: (i) the assembly of unigene libraries *(i.e.* the identification/removal of replicate clones), (ii) the identification of novel genes or novel variants of previously identified genes, and (iii) transcription profiling. The speed and throughput of the present method should permit the processing of more clones and, hence, a more in depth analysis of a cDNA library.

A variety of methods are known in the art for transcription profiling, *i*.*e*. the analysis of the transcription in both qualitative and quantitative terms. In one method, the expressed-sequence-tag (EST) approach, the mRNA population is assessed by partial sequencing of randomly selected cDNA clones. Global changes in gene-expression patterns are deduced from the EST ratios among two compared cDNA libraries [Lee N. *et al., Proc. Natl. Acad. Sci. USA* **92:** 8303-8307 (1995)]. The methods described herein may be used to catalogue expressed genes with a similar level of resolution but considerably higher speed and throughput. First, a library of unidirectionally cloned cDNAs is constructed in a vector that permits transcription of the inserted sequences. Preferably, the 3'-end of the cDNAs is located adjacent to the promoter. Template for transcription can be prepared by amplification of the promoter-cDNA cassette using a pair of vector-specific primers. Alternatively, vector DNA is prepared and cleaved at a restriction site within the vector close to the 5'-end of the inserted cDNA *(e.g.* ∼25 base-pairs). Preferably, the restriction site at which the templates are cleaved should have a low occurrence frequency within the cDNAs under study. Run-off transcripts, synthesized from PCR product or digested vector, are characterized by a common 3'-end, consisting of vector sequences, which allows the isolation of full-length transcripts as described in Example 2. An alternative strategy involves treatment of the vector DNAs with a restriction reagent such that not only all templates are digested at the cDNA 5'-end but that a vast majority is also cleaved within the cDNA at some distance from the 3'-end (*e*.*g*. a few hundred base-pairs). The restriction reagent may be a single enzyme or a combination of two or more restriction enzymes. Ligation of an adaptor to the digestion product(s) [see Vos P. *et al., Nucleic Acids Res.* **23:** 4407-4414 (1995)] can be considered so as to obtain full-length transcripts with a common 3'-end enabling their isolation as described in Example 2. However, transcripts that incorporate a biotin group at the 5'-end may also be prepared [Hahner S. *et al., Nucleic Acids Res.,* **25:** 1957-1964 (1997)], providing an alternative means for their immobilization. Digestion within the cDNAs is an attractive option in that different partial cDNAs deriving from the same transcript are made congruent by this procedure and thereby facile to identify. The full-length run-off transcripts are finally subjected to complementary sequence-specific cleavage reactions, and the resultant digestion products analyzed by MS as disclosed herein.

Those of skill in the art will recognize the advantages of the transcript profiling method outlined above. Comparable to the EST approach, cDNAs are identified at the sequence-level, i.e. the ultimate level of resolution. Thus, while the method involves fragmentation of the interrogated nucleic acid, its level of resolution far exceeds that attained by fingerprinting techniques [Prashar Y. and Weissman S., *Proc. Natl. Acad. Sci. USA* **93:** 659-663 (1996); Bachem C. *et al., The Plant Journal* **9:** 745-753 (1996); Ivanova N. and Belyavsky A., *Nucleic Acids Res.* **23:** 2954-2958 (1995); Liang P. and Pardee A., *Science* **257:** 967-971 (1992)]. In contrast to hybridization-based approaches [Schena M. *et al., Science* **270:** 467-470 (1995); Wodicka L. *et al., Nature Biotechnology* **15:** 1359-1367 (1997)] the method can identify both known and previously unknown sequences. Also, it should prove faster then methods requiring gel-electrophoretic fractionation.

### EXAMPLE 7

### Whole-Genome Re-Sequencing

In the past couple of years the technology for sequencing entire genomes, especially those of microorganisms, has come to maturity. More than 50 microbial genomes are scheduled to be completed by the year 2000, and the benefits emerging from this vast body of knowledge are rapidly becoming clear [Clayton R. *et al., Curr. Opinion Microbiol*. **1:** 562-566 (1998)]. It seems clear that sequencing entire microbial genomes is becoming routine and that microbial genetics is entering the era of 'comparative genomics'. Knowledge of the complete genome sequence is the ultimate tool in phylogenetic analyses, allows gene/functional diversity studies, and fundamentally changes the manner in which research is conducted in an organism. At the present time, a substantial portion of each new genome sequence has no database match. One may expect to see a greater proportion of orthologous genes in the future, when the microbial species diversity is better represented. At that point, when most of the sequences generated will be similar to already known sequences, global genome analyses could be performed rapidly, accurately, and cost-effectively using a re-sequencing strategy as described herein rather than by *de novo* sequencing methods. Similar evolutions may be anticipated outside the bacterial genetics field where genome projects for many (model) organisms are ongoing or have already been finished *(e.g., Drosophila melanogaster, Caenorhabditis elegans,* human, mouse, *Arabidopsis thaliana*, and rice).

The methods of the present invention may be readily adapted to the re-sequencing of entire (bacterial) genomes or megabase nucleic acid regions. This may be accomplished with the use of a shotgun approach that involves the sequence analysis of unselected subclones that harbor random fragments according to the methods of the present invention. The assembly of all the independent, random sequences is fundamentally different from that in a *de novo* sequencing project [Fleischmann R. *et al., Science* **269:** 496-512 (1995)] because of the availability of a reference sequence that serves as a scaffold. The assembly into a single complete sequence comes down to matching each set of experimentally obtained spectra with a portion of the reference sequence. The computational approaches required to accomplish this are similar to those that are needed for the analysis of cDNA libraries, outlined in Example 6. In both cases one does not know in advance the reference sequence, if at all existing, for a given interrogated target region. It should be noted, however, that the present shotgun approach might be even more demanding in terms of computational power because of the undefined ends of the segments. At the same time, the algorithms must be capable of mapping the variations that occur between the target and the reference sequence. It is expected that a shotgun approach with its built-in redundancy *(i.e.,* most sequences will be covered several-fold) should prove useful for the comprehensive comparison of a pair of related genomes. An alternative for the shotgun approach strategy consists of the analysis of clones from one or more libraries of restriction enzyme fragments or the analysis of defined amplicons generated with locus specific primer pairs.

**Table I:**

| Detection of the twelve possible point mutations that can occur in DNA by the methods of the present invention. Each substitution is associated with the loss (- sign) and gain (+ sign) of a cleavage site. In addition, each mutation affects the mass of two digestion products as indicated. Mass differences shown in bold face result from the incorporation of m⁵U in both transcripts (see text for details). | | | | | |
|---|---|---|---|---|---|
| Mutation | | RNase T1 | | RNase U2 | |
| (+) strand | (-) strand | (+) transcript | (-) transcript | (+) transcript | (-) transcript |
| **transitions** | | | | | |
| A->G | T->C | + | -1 Da **-15 Da** | - | -1 Da **-15 Da** |
| G->A | C->T | - | +1 Da **+15 Da** | + | +1 Da **+15 Da** |
| T->C | A->G | -1 Da **-15 Da** | + | -1 Da **-15 Da** | - |
| C->T | G->A | +1 Da **+15 Da** | - | +1 Da **+15 Da** | + |

| **transversions** | | | | | |
|---|---|---|---|---|---|
| A->C | T->G | -24 Da | + | - | +39 Da +**25 Da** |
| C->A | G->T | +24 Da | - | + | -39 Da -**25 Da** |
| T->G | A->C | + | -24 Da | +39 Da **+25 Da** | - |
| G->T | C->A | - | +24 Da | -39 Da **-25 Da** | + |
| T->A | A->T | +23 Da **+9 Da** | -23 Da **-9 Da** | + | - |
| A->T | T->A | -23 Da -**9 Da** | +23 Da +**9 Da** | - | + |
| C->G | G->C | + | - | +40 Da | -40 Da |
| G->C | C->G | - | + | -40 Da | +40 Da |

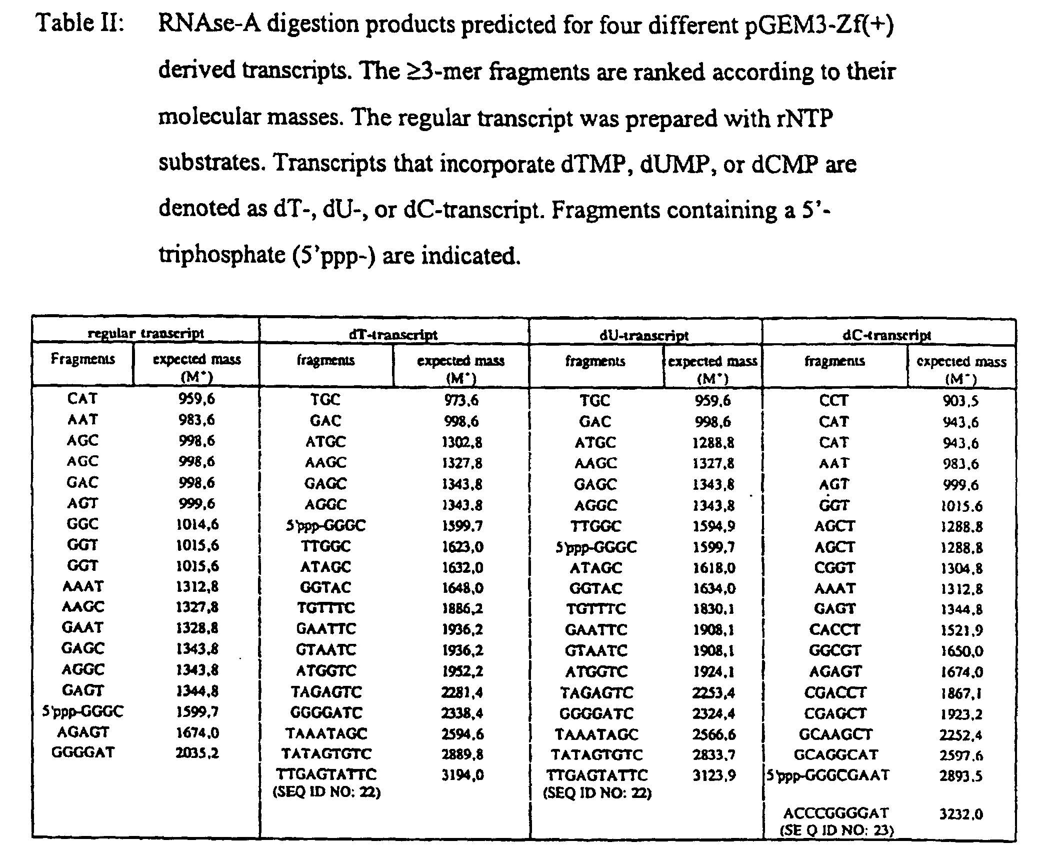

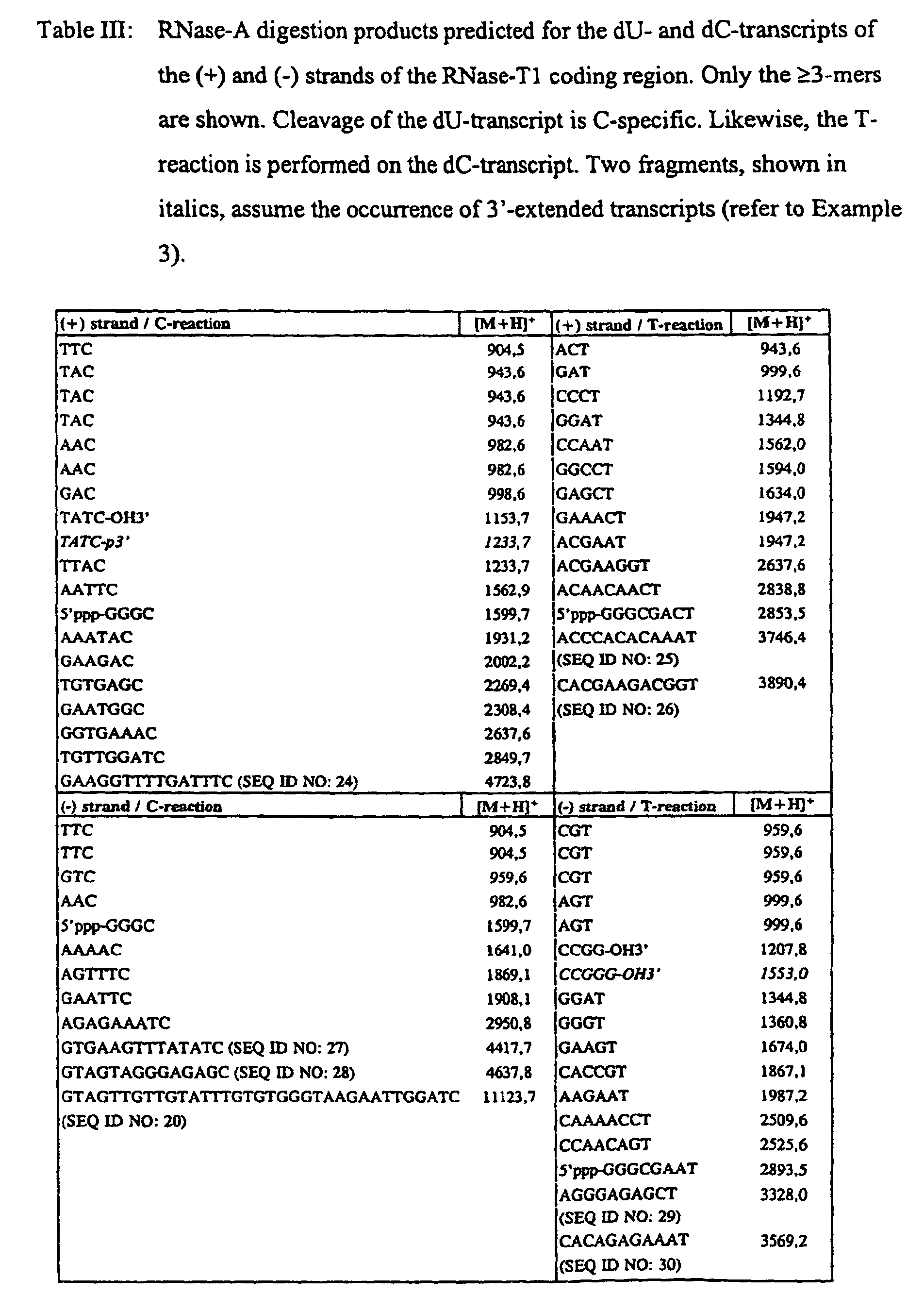

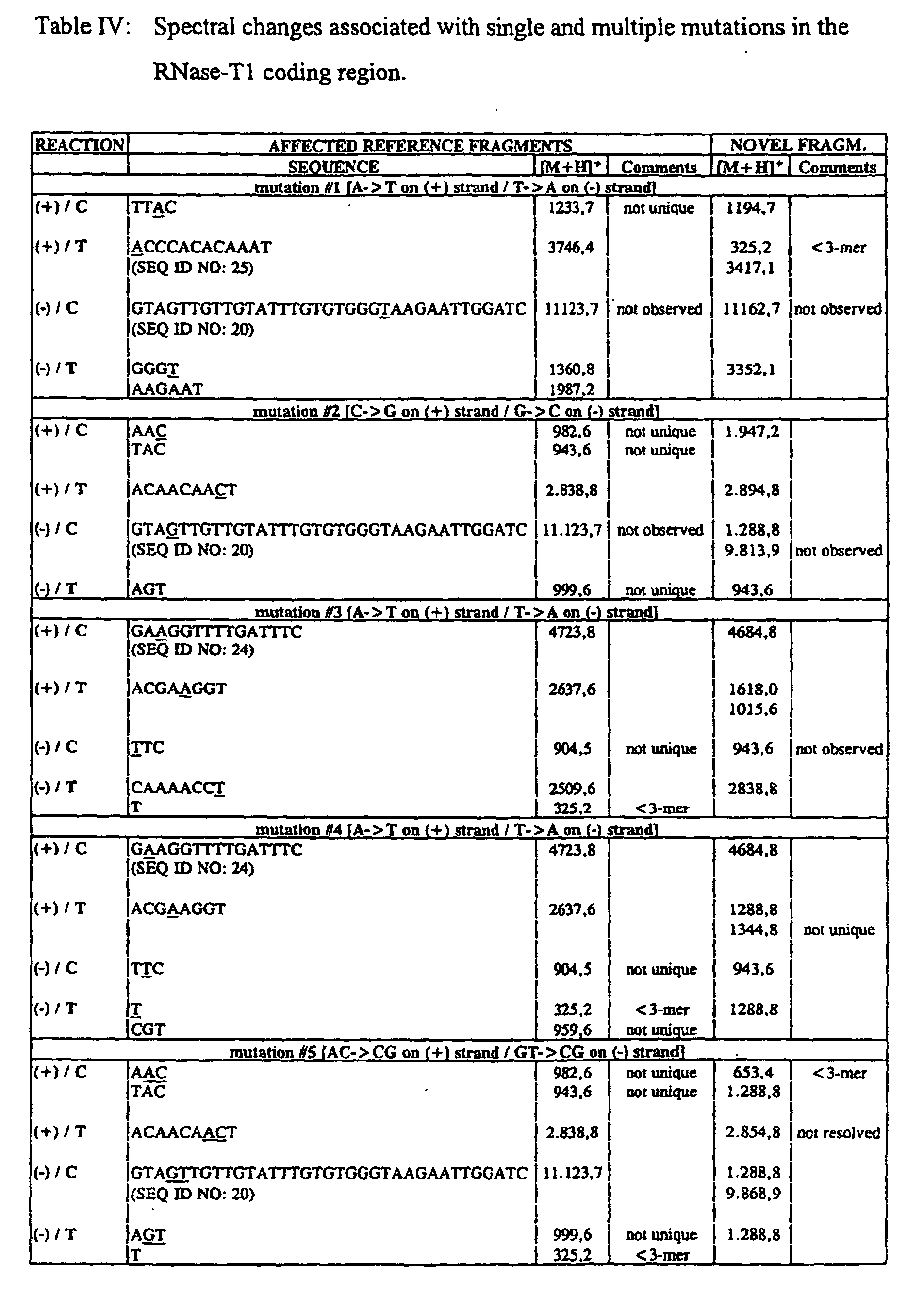

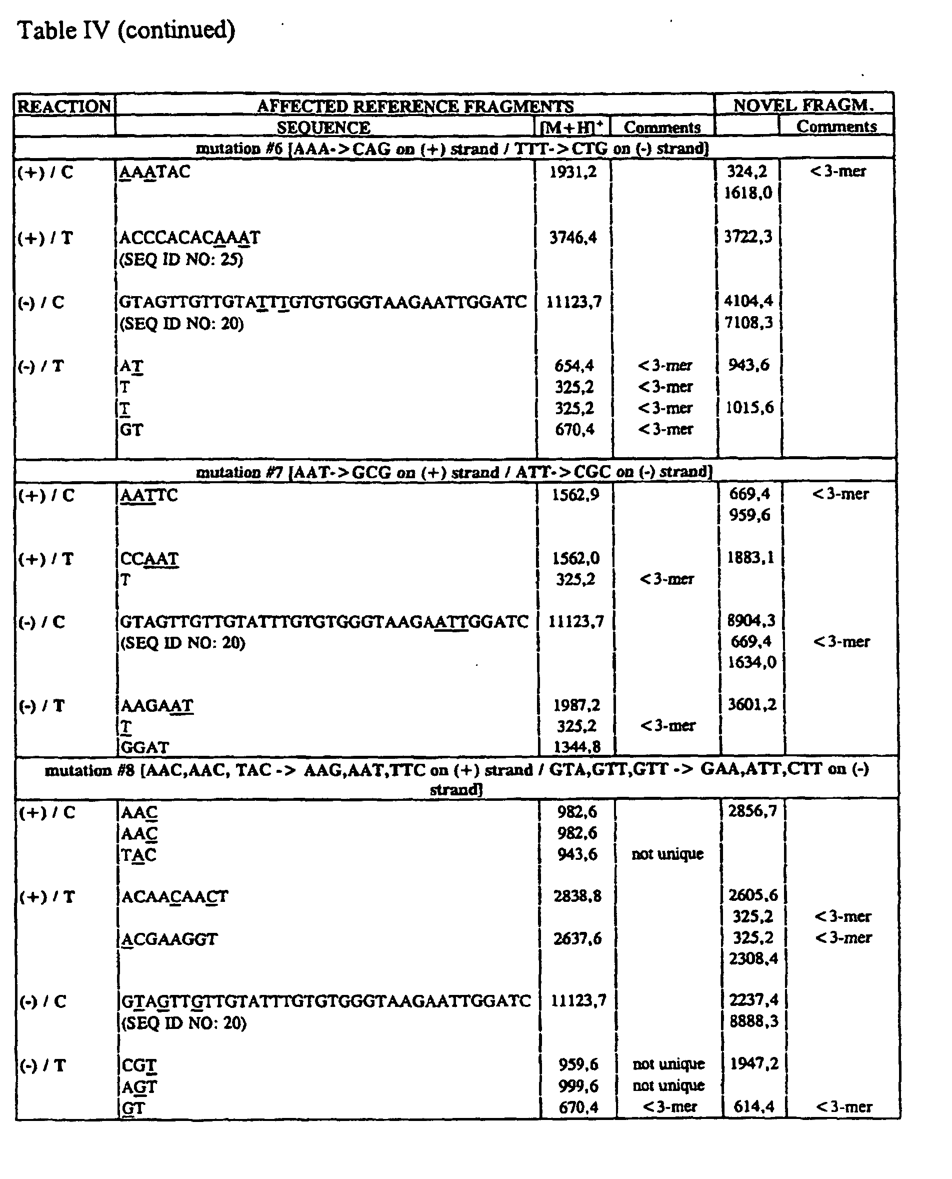

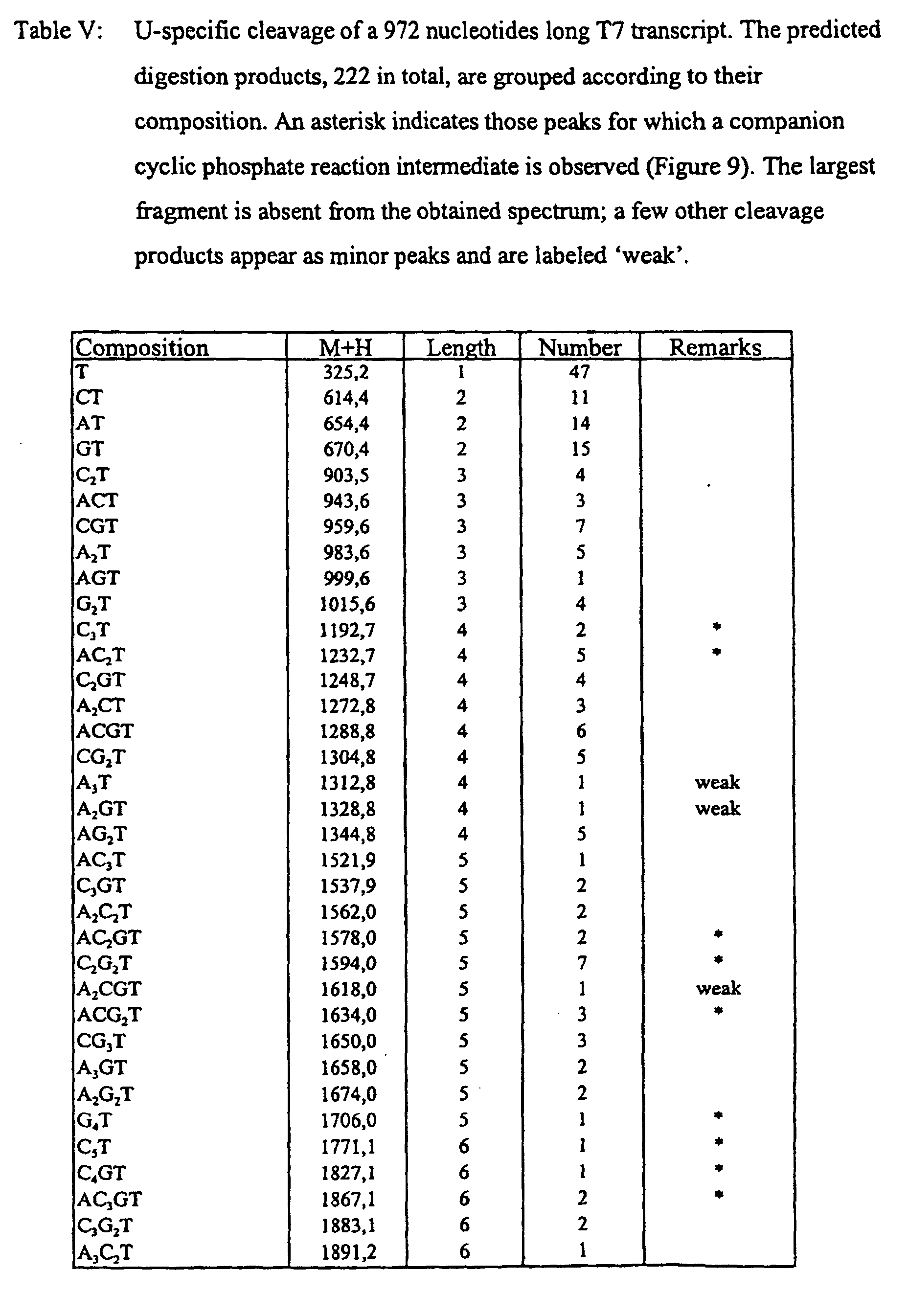

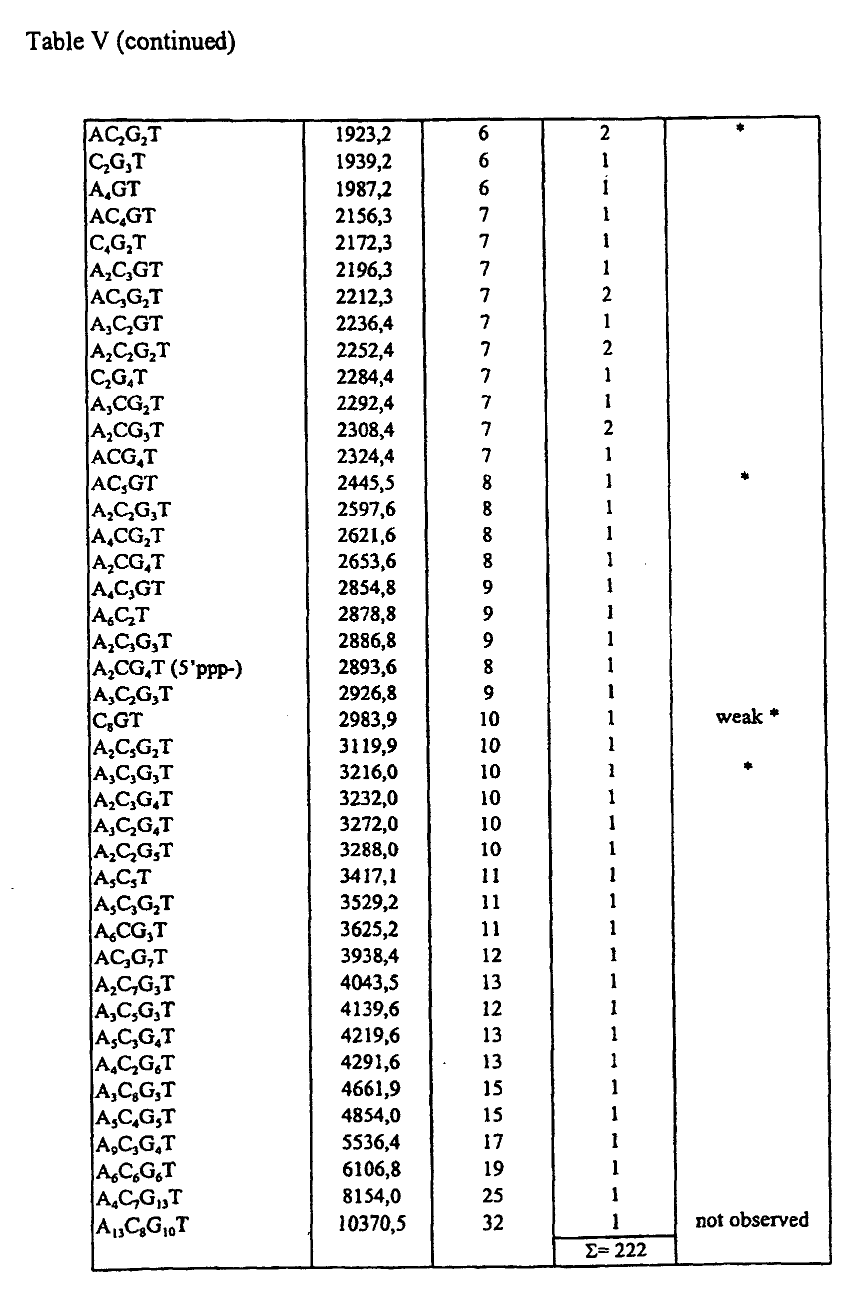

## Claims

1. A method for mass spectrometry based determination and/or analysis of the sequence of a target nucleic acid present in a biological sample, said method comprising the steps of:
(a) deriving from said biological sample said target nucleic acid;
(b) subjecting said target nucleic acid obtained from step (a) to a set of four separate base-specific complementary cleavage reactions, wherein each cleavage reaction generates a non-ordered set of fragments; which is a set of fragments without a common endpoint.
(c) analyzing the sets of non-ordered fragments obtained from step (b) by mass spectrometry; and,
(d) performing a systematic computational analysis on the mass spectra obtained from step (c) to determine and/or analyse the sequence of said target nucleic acid,
wherein said complementary cleavage reactions refer to target nucleic acid digestions **characterized by** varying specificity and/or to digestion of alternative forms of the target sequence.

2. The method according to claim 1 wherein said biological sample is derived from an organism selected from the group consisting of eukaryotes, prokaryotes, and viruses.

3. The method according to claim 1 or 2 wherein said target nucleic acid is selected from the group consisting of single stranded DNA, double stranded DNA, cDNA, single stranded RNA, double stranded RNA, DNA/RNA hybrid, and DNA/RNA mosaic nucleic acid.

4. The method according to any of the claims 1 to 3 wherein said target nucleic acid is derived by (a) consecutive amplification procedure(s) selected from the group consisting of *in vivo* cloning, polymerase chain reaction (PCR), reverse transcription followed by the polymerase chain reaction (RT-PCR), strand displacement amplification (SDA), and transcription based processes.

5. The method according to claim 4 wherein said amplified target nucleic acid is a transcript generated from a single stranded or a double stranded target nucleic acid by a process comprising the steps of:
(a) linking operatively a transcription control sequence to said target nucleic acid; and
(b) transcribing one or both strands of the target nucleic acid of step a) using (an) RNA polymerase(s) that recognize(s) the transcription control sequence.

6. The method according to claim 5 wherein said transcription control sequences are operatively linked to the target nucleic acid by PCR amplification using primers that incorporate the transcriptional control sequences as 5'-extensions.

7. The method according to claims 5 or 6 wherein the transcription control sequence is selected from the group consisting of eukaryotic transcription control sequences, prokaryotic transcription control sequences, and viral transcription control sequences.

8. The method according to claim 7 wherein the prokaryotic transcription control sequence is selected from the group consisting of T3, T7, and SP6 promoters.

9. The method according to claim 8 wherein the RNA polymerases which utilize the T3, T7, or SP6 promoters are either wild type or mutant RNA polymerases, the mutant polymerases being capable of incorporating into the transcript non-canonical substrates with a 2'-deoxy, 2'-O-methyl, 2'-fluoro or 2'-amino substituent.

10. The method according to claim 9 wherein the mutant RNA polymerase is either T7 or SP6 mutant polymerase.

11. The method according to any of the claims I to 10 wherein the derived target nucleic acid incorporates (a) nucleoside(s) that are (is) modified on the base, the sugar, and/or the phosphate moiety, wherein the modifications alter the specificity of the cleavage by the cleavage reagent(s) and/or the mass and/or the length of the cleavage products.

12. The method according to claim 11 wherein the modification is introduced through the enzymatic incorporation of modified deoxynucleoside triphosphates, modified ribonucleoside triphosphates, and/or modified dideoxynucleoside triphosphates; or wherein the modification is introduced chemically, or wherein the modification is introduced through a combination of both methods.

13. The method according to claim 11 or 12 wherein the modification consists of a 2'-deoxy, 2'-*O*-methyl, 2'-fluoro or 2'-amino substituent on the nucleotide triphosphates.

14. The method according to claim 11 or 12 wherein the modification consists of phosphorothioate internucleoside linkages or phosphorothioate internucleoside linkages further reacted with an alkylating reagent.

15. The method according to claim 11 or 12 wherein the modification consists of a methyl group on C5 of the uridine-5'-monophosphate subunits.

16. The method according to claim 11 or 12 wherein the modification consists of nucleotides that incorporate alternative isotopes.

17. The method according to any of the claims 1 to 16, wherein said target nucleic acid of step (a) is purified prior to cleavage.

18. The method according to claim 17 wherein said purification is achieved through immobilization or by chromatography.

19. The method according to any of the claims 1 to 18 wherein the complementary cleavage reactions are selected from the group consisting of enzymatic cleavage, chemical cleavage and physical cleavage.

20. The method according to claim 19 wherein the complementary cleavage reactions are **characterized by** a relaxed mono-nucleotide, mono-nucleotide, relaxed di-nucleotide, or di-nucleotide specificity.

21. The method according to claim 19 or 20 wherein said target nucleic acid is subjected to chemical digestion reaction consisting of treatment with alkali or with reagents used in the Maxam & Gilbert sequencing method.

22. The method according to claim 19 or 20 wherein said target nucleic acid is subjected to enzymatic cleavage reaction using (an) enzyme(s) selected from the group consisting of endonucleases and exonucleases.

23. The method according to claim 22 wherein said target nucleic acid is subjected to enzymatic cleavage reaction using (an) endonuclease(s), selected from the group consisting of restriction enzymes, RNA endonucleases, DNA endonucleases and non-specific phosphodiesterases.

24. The method according to claim 23 wherein the endonuclease(s) is (are) (a) selective or non-selective RNA endonuclease(s), selected from the group consisting of the G-specific T₁ ribonuclease, the A-specific U₂ ribonuclease, the A/U specific phyM ribonuclease, the U/C specific ribonuclease A, the C-specific chicken liver ribonuclease (RNaseCL3) and cusativin, non-specific RNase-I, and pyrimidine-adenosine preferring RNases isolated from *E. coli, Enterobacter sp.,* or *Saccharomyces cerevisiae*.

25. The method according to any of the claims 1 to 8, 11, 12, 14, 17 to 20, 22 and 23 wherein said target nucleic acid is phosphorothioate-modified single stranded DNA or RNA, and wherein the nucleic acid digestions are performed with the nuclease P1.

26. The method according to any of the claims 1 to 24 wherein said target nucleic acid is a mosaic RNA/DNA nucleic acid or a modified mosaic RNA/DNA nucleic acid, prepared with mutant polymerases, and wherein the cleavage reagents are RNA endonucleases, DNA endonucleases or alkali.

27. The method according to any of the claims 1 to 24 and 26 wherein said target nucleic acid is a transcript, a modified transcript, a mosaic RNA/DNA transcript or a modified mosaic RNA/DNA transcript, prepared with wild type or mutant RNA polymerases, and wherein the cleavage reagents are (is a) selective or non-selective RNA endonuclease(s) or alkali.

28. The method according to any of the claims 1 to 24 and 26 to 27
wherein said target nucleic acid is a mosaic RNA/DNA transcript that incorporates either dCMP, dUMP or dTMP, prepared with mutant T7 or SP6 polymerase, and wherein the cleavage reagent is a pyrimidine-specific Rnase.

29. The method according to claim 28, wherein said pyrimidine-specific Rnase is RNase-A.

30. The method according to any of the claims 1 to 29, wherein the set of non-ordered fragments of step (b) is additionally purified using cation exchange beads.

31. The method according to any of the claims 1 to 30, wherein the set of non-ordered fragments of step (b) is applied onto a solid support.

32. The method according to claim 31 wherein said solid support is chosen from a group consisting of solid surfaces, plates and chips.

33. The method according to any of the claims 1 to 32 wherein the mass spectrometric analysis of the nucleic acid fragments is performed using a mass spectrometric method selected from the group consisting of Matrix-Assisted Laser Desorption/Ionization-Time-of-flight (MALDI-TOF), Electrospray-Ionization (ESI), and Fourier Transform-Ion Cyclotron Resonance (FT-ICR).

34. The method according to any of the claims 1 to 33 for mass spectometry based determination and/or analysis of a target nucleic acid present in a biological sample, wherein for said target nucleic acid a reference nucleic acid sequence is known; with said method comprising an additional step wherein the mass spectra of the non-ordered fragments obtained in step c) of claim 1 are compared with the known or predicted mass spectra for a reference nucleic acid sequence, and deducing there from, by systematic computational analysis, all or part of the nucleotide sequence of the target nucleic acid, and comparing the deduced nucleic acid sequence with the reference nucleic acid to determine whether the target nucleic acid has the same sequence or a different sequence from the reference nucleic acid.

35. The method according to claim 34 wherein the nucleic acid sequence difference that is determined is a deletion, substitution, insertion or combinations thereof.

36. The method according to claim 35 wherein the nucleic acid sequence difference is a Single Nucleic Polymorphism (SNP).

37. The method according to any of the claims 34 to 36 for scoring known as well as unknown nucleotide sequence variations of a target nucleic acid present in a biological sample.

38. The method according to claim 37 wherein the mass spectometry based determination and/or analysis of said known or unknown nucleotide sequence variations allows the identification of the various allelic sequences of a certain region/gene, the scoring of disease-associated mutations, the detection of somatic variations, or studies in the field of molecular evolution.

39. The method according to any of the claims 34 to 38 for genome wide genotyping of a biological sample.

40. The method according to claim 34 wherein the mass spectra obtained for a target nucleic acid are compared with the mass spectra predicted for a plurality of reference nucleic acids thereby identifying/detecting a target nucleic acid in a biological sample.

41. The method according to any of the claims 1 to 33 for mass spectometry based determination and/or analysis of the sequence of a target nucleic acid of unknown sequence present in a biological sample.

42. The method according to claim, 41 for mass spectometry based determination and/or analysis of the sequence of a target nucleic acid of unknown sequence present in a biological sample, wherein, if the sequence is not uniquely defined after step (d) of claim 1, steps (a) through (d) are repeated, thereby generating modified forms of said target nucleic acid and/or different portions of said target nucleic acid, and performing supplementary mono- and/or di-nucleotide specific cleavage reactions rendering supplementary sets of non-ordered fragments until the combined data converge into a unique sequence solution.

43. The method according to claim 41 or 42 for genome wide genotyping of a biological sample.

44. The method according to any of the claims 1 to 43, wherein the target nucleic acid is prepared by the concomitant amplification of multiple fragments.

45. The method according to any of the claims 1 to 38, 41, 42 or 44,
wherein said biological sample comprises a pool of samples.

46. A kit for mass spectrometry based determination and/or analysis of the sequence of a target nucleic acid present in a biological sample according to a method of any of the claims 1 to 45, the kit comprising:
(a) nucleotide triphosphates;
(b) (a) polymerase(s);
(c) (a) nucleic acid cleaving agent(s) to perform different base specific cleavage reactions and
(d) a computer program comprising codes for performing a systematic computational analysis on the mass spectra obtained from the mass spectrometrical analysis of non-ordered fragments, which is a set of fragments without a common endpoint, when executed on a data processing system,
wherein said program comprises the steps of 1/ subjecting a reference nucleic acid and sequence variants thereof to the different base specific cleavages to generate fragments, computing the mass of each fragment, generating the mass spectra of the fragments from a reference nucleic acid and the sequence variants thereof for each of the base specific cleavage reactions, and, 2/ matching these computationally derived mass spectra with the spectra obtained experimentally in the different base specific cleavage reactions.

47. A kit according to claim 46 which further comprises cation exchange beads in order to purify the non-ordered set of fragments generated by means of a method according to any of the claims 1 to 45.

48. A kit according to claim 47 which further comprises a solid support whereon the non-ordered set of fragments may be applied.

49. Use of a kit according to any of the claims 46 to 49 for mass spectometry based determination and/or analysis of the sequence of a target nucleic acid, for determining and/or analyzing sequence differences, for scoring known as well as unknown nucleotide sequence variations, for detecting/identifying, or, for performing genome wide genotyping using a target nucleic acid present in a biological sample, for which target nucleic acid a reference nucleic acid sequence is known.

50. Use of a kit according to any of the claims 46 to 49 for mass spectometry based determination and/or analysis of the sequence of a target nucleic acid, for detecting/identifying, or, for performing genome wide genotyping using a target nucleic acid of unknown sequence present in a biological sample.

## Patentansprüche

1. Verfahren für die Bestimmung und/oder Analyse der Sequenz einer Ziel-Nukleinsäure in einer biologischen Probe auf Basis von Massenspektrometrie, wobei das Verfahren die Schritte umfasst:
(a) aus der biologischen Probe die Ziel-Nukleinsäure zu erhalten;
(b) die in Schritt (a) erhaltene Ziel-Nukleinsäure einem Set von vier separaten Basen-spezifischen komplementären Spaltungsreaktionen zu unterwerfen, wobei jede Spaltungsreaktion einen nicht-geordneten Satz von Fragmenten erzeugt, wobei dieser ein Satz von Fragmenten ohne gemeinsamen Endpunkt ist;
(c) die Sätze nicht-geordneter Fragmente, die mittels Massenspektrometrie aus Schritt (b) erhalten wurden, zu analysieren; und
(d) eine systematische Computer-Analyse anhand der in Schritt (c) erhaltenen Massenspektren durchzuführen und die Sequenz der Ziel-Nukleinsäure zu bestimmen und/oder zu analysieren,
wobei die komplementären Spaltungsreaktionen sich auf Verdaus der Ziel-Nukleinsäuren beziehen, **dadurch gekennzeichnet, dass** die Spezifität und/oder der Verdau der alternativen Form der Ziel-Sequenz variiert werden.

2. Das Verfahren nach Anspruch 1, wobei die biologische Probe von einem Organismus abstammt, der ausgewählt wurde aus der aus Eukaryoten, Prokaryoten und Viren bestehenden Gruppe.

3. Das Verfahren nach Anspruch 1 oder 2, wobei die Ziel-Nukleinsäure ausgewählt ist aus der aus Einzelstrang-DNA, Doppelstrang-DNA, cDNA, Einzelstrang-RNA, Doppelstrang-RNA, DNA/RNA-Hybriden und DNA/RNA-Mosaiknukleinsäuren bestehenden Gruppe.

4. Das Verfahren nach einem der Ansprüche 1 bis 3, wobei die Ziel-Nukleinsäure erhalten wird durch (ein) konsekutive(s) Amplifikationsverfahren, das (die) ausgewählt ist (sind) aus der aus *in vivo*-Klonieren, Polymerase-Kettenreaktion (PCR), reverser Transkription und nachfolgender Polymerase-Kettenreaktion (RT-PCR), Strang-Austausch-Amplifikation (SDA) und Prozessen auf Basis der Transkription bestehenden Gruppe.

5. Das Verfahren nach Anspruch 4, wobei die amplifizierte Ziel-Nukleinsäure ein Transkript ist, das von einer Einzelstrang- oder Doppelstrang-Ziel-Nukleinsäure mittels eines Verfahrens erzeugt wurde, das die folgenden Schritte umfasst:
(a) eine Sequenz für die Kontrolle der Transkription operativ mit der Ziel-Nukleinsäure zu verbinden und
(b) einen oder beide Stränge der Ziel-Nukleinsäure aus Schritt (a) unter Verwendung von mindestens einer RNA-Polymerase, die die Sequenz zur Kontrolle der Transkription erkennt, zu transkribieren.

6. Das Verfahren nach Anspruch 5, wobei die Sequenzen zur Kontrolle der Transkription mit der Ziel-Nukleinsäure durch PCR-Amplifikation unter Verwendung von Primem, die die Sequenzen zur Kontrolle der Transkription als 5'-Verlängerungen einbauen, operativ verknüpft sind.

7. Das Verfahren nach Anspruch 5 oder 6, wobei die Sequenz zur Kontrolle der Transkription ausgewählt ist aus der aus eukaryotischen Sequenzen zur Kontrolle der Transkription, prokaryotischen Sequenzen zur Kontrolle der Transkription und viralen Sequenzen zur Kontrolle der Transkription bestehenden Gruppe.

8. Das Verfahren nach Anspruch 7, wobei die prokaryotische Sequenz zur Kontrolle der Transkription ausgewählt ist aus der aus dem T3-, T7- und SP6-Promotor bestehenden Gruppe.

9. Das Verfahren nach Anspruch 8, wobei die RNA-Polymerasen, die sich des T3-, T7- oder SP6-Promotors bedienen, entweder Wildtyp- oder mutierte RNA-Polymerasen sind,
wobei die mutierten Polymerasen nicht-kanonische Substrate mit einem 2'-Desoxy-, 2'-O-Methyl-, 2'-Fluor- oder 2'-Amino-Substituenten in das Transkript einbauen können.

10. Das Verfahren nach Anspruch 9, wobei die mutierte RNA-Polymerase die mutierte T7-oder SP6-Polymerase ist.

11. Das Verfahren nach einem der Ansprüche 1 bis 10, wobei die Ziel-Nukleinsäure mindestens ein Nukleosid enthält, das in der Base, im Zucker und/oder im Phosphat-Rest modifiziert ist, wobei diese Modifikation(en) die Spezifität der Spaltung mit dem Spaltungsreagens bzw. den Spaltungsreagentien und/oder die Masse und/oder die Länge der Spaltungsprodukte verändert.

12. Das Verfahren nach Anspruch 11, wobei die Modifikation durch den enzymatischen Einbau von modifizierten Desoxynukleosid-Triphosphaten, modifizierten Ribonukleosid-Triphosphaten und/oder modifizierten Didesoxynukleosid-Triphosphaten eingeführt wird; oder wobei die Modifikation chemisch eingeführt wird, oder wobei die Modifikation durch eine Kombination von beiden Verfahren eingeführt wird.

13. Das Verfahren nach Anspruch 11 oder 12, wobei die Modifikation aus einem 2'-Desoxy-, 2'-O-Methyl-, 2'-Fluor- oder aus einem 2'-Amino-Substituenten an den Nukleotid-Triphosphaten besteht.

14. Das Verfahren nach Anspruch 11 oder 12, wobei die Modifikation aus Phosphorthioat-Intemukleosid-Bindungen oder Phosphorthioat-Internukleosid-Bindungen, die außerdem mit einem Alkylierungsmittel umgesetzt wurden, besteht.

15. Das Verfahren nach Anspruch 11 oder 12, wobei die Modifikation aus einer MethylGruppe am C5 der Uridin-5'-monophosphat-Untereinheiten besteht.

16. Das Verfahren nach Anspruch 11 oder 12, wobei die Modifikation aus Nukleotiden besteht, die alternative Isotopen enthalten.

17. Das Verfahren nach einem der Ansprüche 1 bis 16, wobei die Ziel-Nukleinsäure aus Schritt (a) vor der Spaltung gereinigt wird.

18. Das Verfahren nach Anspruch 17, wobei die Reinigung mittels Immobilisierung oder Chromatographie erzielt wird.

19. Das Verfahren nach einem der Ansprüche 1 bis 18, wobei die komplementären Spaltungsreaktionen ausgewählt sind aus der aus enzymatischer Spaltung, chemischer Spaltung und physikalischer Spaltung bestehenden Gruppe.

20. Das Verfahren nach Anspruch 19, wobei die komplementären Spaltungsreaktionen **gekennzeichnet sind durch** eine Spezifität für relaxiertes Mononukleotid, für Mononukleotid, für relaxiertes Dinukleotid oder für Dinukleotid.

21. Das Verfahren nach Anspruch 19 oder 20, wobei die Ziel-Nukleinsäure einer chemischen Abbaureaktion unterworfen wird, die aus der Behandlung mit Alkali oder mit Reagenzien, wie sie in dem Maxam & Gilbert-Sequenzierverfahren verwendet werden, besteht.

22. Das Verfahren nach Anspruch 19 oder 20, wobei die Ziel-Nukleinsäure unter Verwendung von mindestens einem Enzym, ausgewählt aus der aus Endonukleasen und Exonukleasen bestehenden Gruppe, einer enzymatischen Spaltungsreaktion unterworfen wird.

23. Das Verfahren nach Anspruch 22, wobei die Ziel-Nukleinsäure unter Verwendung mindestens einer Endonuklease, ausgewählt aus der aus Restriktionsenzymen, RNA-Endonukleasen, DNA-Endonukleasen und nicht-spezifischen Phosphodiesterasen bestehenden Gruppe, einer enzymatischen Spaltungsreaktion unterworfen wird.

24. Das Verfahren nach Anspruch 23, wobei die mindestens eine Endonuklease eine selektive oder nicht-selektive RNA-Endonuklease ist, die ausgewählt ist aus der aus G-spezifischer T₁-Ribonuklease, A-spezifischer U₂-Ribonuklease, A/U-spezifischer phyM-Ribonuklease, U/C-spezifischer Ribonuklease A, C-spezifischer Ribonuklease aus Hühnerleber (RNaseCL3) und Cusativin, nicht-spezifischer RNase-I und Pyrimidin-Adenosin bevorzugenden RNasen, die aus *E. coli, Enterobakter sp.* oder *Saccharomyces cerevisiae* isoliert werden, bestehenden Gruppe.

25. Das Verfahren nach einem der Ansprüche 1 bis 8, 11, 12, 14, 17 bis 20, 22 und 23, wobei die Ziel-Nukleinsäure Phosphorthioat-modifizierte Einzelstrang-DNA oder -RNA ist, und wobei die Nukleinsäure-Verdaus mit Nuklease P1 durchgeführt werden.

26. Das Verfahren nach einem der Ansprüche 1 bis 24, wobei die Ziel-Nukleinsäure eine Mosaik-RNA/DNA-Nukleinsäure oder eine modifizierte Mosaik-RNA/DNA-Nukleinsäure ist, die mit mutierten Polymerasen hergestellt wurde, und wobei die Spaltungsreagentien RNA-Endonukleasen, DNA-Endonukleasen oder Alkali sind.

27. Das Verfahren nach einem der Ansprüche 1 bis 24 und 26, wobei die Ziel-Nukleinsäure ein Transkript, ein modifiziertes Transkript, ein Mosaik RNA/DNA-Transkript oder ein modifiziertes Mosaik RNA/DNA-Transkript ist, das mit Wildtyp- oder mutierten RNA-Polymerasen hergestellt wurde, und wobei die Spaltungsreagentien mindestens eine selektive oder nicht-selektive RNA-Endonuklease oder Alkali ist.

28. Das Verfahren nach einem der Ansprüche 1 bis 24 und 26 bis 27, wobei die Ziel-Nukleinsäure ein Mosaik-RNA/DNA-Transkript ist, das entweder dCMP, dUMP oder dTMP enthält und mit mutierter T7- oder SP6-Polymerase hergestellt wurde, und wobei das Spaltungsreagens eine Pyrimidin-spezifische RNase ist.

29. Das Verfahren nach Anspruch 28, wobei die Pyrimidin-spezifische RNase RNase-A ist.

30. Das Verfahren nach einem der Ansprüche 1 bis 29, wobei der Satz von nicht-geordneten Fragmenten aus Schritt (b) zusätzlich unter Verwendung von Kationen-Austauscherkügelchen gereinigt wird.

31. Das Verfahren nach einem der Ansprüche 1 bis 30, wobei der Satz an nicht-geordneten Fragmenten aus Schritt (b) auf einen festen Träger aufgebracht wird.

32. Das Verfahren nach Anspruch 31, wobei der feste Träger ausgewählt ist aus der aus festen Oberflächen, Platten und Chips bestehenden Gruppe.

33. Das Verfahren nach einem der Ansprüche 1 bis 32, wobei die massenspektrometrische Analyse der Nukleinsäure-Fragmente unter Verwendung eines massenspektrometrischen Verfahrens, ausgewählt aus der aus MALDI-TOF (Matrix-assistierte Laser-Desorption/Ionisation-Flugzeit), Elektrospray-Ionisation (ESI) und FT-ICR (Fourier-Transformation-Ionen-Cyclotron-Resonanz) bestehenden Gruppe, durchgeführt wird.

34. Das Verfahren nach einem der Ansprüche 1 bis 33 für die Bestimmung und/oder Analyse der Sequenz einer Ziel-Nukleinsäure in einer biologischen Probe auf Basis von Massenspektrometrie, wobei für die Ziel-Nukleinsäure eine Referenz-Nukleinsäure-Sequenz bekannt ist; wobei das Verfahren einen zusätzlichen Schritt umfasst, in dem die Massenspektren der nicht-geordneten Fragmente, die in Schritt (c) von Anspruch 1 erhalten wurden, mit den bekannten oder vorhergesagten Massenspektren für eine Referenz-Nukleinsäure-Sequenz verglichen werden, und in dem davon mittels systematischer Computeranalyse die gesamte oder ein Teil der Nukleotid-Sequenz der Ziel-Nukleinsäure abgeleitet werden und die abgeleitete Nukleinsäure-Sequenz mit der Referenz-Nukleinsäure verglichen wird, um so zu bestimmen, ob die Ziel-Nukleinsäure dieselbe Sequenz oder eine von der Referenz-Nukleinsäure verschiedene Sequenz aufweist.

35. Das Verfahren nach Anspruch 34, wobei der Unterschied in der Nukleinsäure-Sequenz, der ermittelt worden ist, eine Deletion, eine Substitution, eine Insertion oder eine Kombination von diesen ist.

36. Das Verfahren nach Anspruch 35, wobei der Unterschied in der Nukleinsäure-Sequenz ein SNP (Einzelnukleotid-Polymorphismus) ist.

37. Das Verfahren nach einem der Ansprüche 34 bis 36 für die Ermittlung von bekannten wie auch unbekannten Variationen in der Nukleotidsequenz von Ziel-Nukleinsäuren in einer biologischen Probe.

38. Das Verfahren nach Anspruch 37, wobei die Bestimmung und/oder Analyse der bekannten oder unbekannten Variationen in der Nukleotidsequenz auf Basis von Massenspektrometrie die Identifizierung von verschiedenen allelischen Sequenzen einer bestimmten Region bzw. eines bestimmten Gens, die Ermittlung von mit Erkrankungen assoziierten Mutationen, den Nachweis von somatischen Variationen und Studien auf dem Gebiet der molekularen Evolution erlauben.

39. Das Verfahren nach einem der Ansprüche 34 bis 38 für das Genom-weite Bestimmen des Genotyps einer biologischen Probe.

40. Das Verfahren nach Anspruch 34, wobei die für eine Ziel-Nukleinsäure erhaltenen Massenspektren verglichen werden mit den Massenspektren, die für eine Vielzahl von Referenz-Nukleinsäuren vorhergesagt werden, wodurch eine Ziel-Nukleinsäure in einer biologischen Probe identifiziert/nachgewiesen wird.

41. Das Verfahren nach einem der Ansprüche 1 bis 33 für die Bestimmung und/oder Analyse der Sequenz einer Ziel-Nukleinsäure unbekannter Sequenz in einer biologischen Probe auf Basis von Massenspektrometrie.

42. Das Verfahren nach Anspruch 41 für die Bestimmung und/oder Analyse der Sequenz einer Ziel-Nukleinsäure unbekannter Sequenz in einer biologischen Probe auf Basis von Massenspektrometrie, wobei, falls die Sequenz nicht einheitlich definiert ist nach Schritt (d) von Anspruch 1, die Schritte (a) bis (d) wiederholt werden, wodurch modifizierte Formen der Ziel-Nukleinsäure und/oder verschiedene Teile der Ziel-Nukleinsäure erzeugt werden, und wobei ergänzende für Mono- und/oder Dinukleotide spezifische Spaltungsreaktionen durchgeführt werden, die zu ergänzenden Sätzen von nicht-geordneten Fragmenten führen, bis die kombinierten Daten zu einer einzigen Sequenz-Lösung konvergieren.

43. Das Verfahren nach Anspruch 41 oder 42 für das Genom-weite Bestimmen des Genotyps einer biologischen Probe.

44. Das Verfahren nach einem der Ansprüche 1 bis 43, wobei die Ziel-Nukleinsäure mittels gleichzeitiger Amplifikation von vielen Fragmenten hergestellt wird.

45. Das Verfahren nach einem der Ansprüche 1 bis 38, 41, 42 oder 44, wobei die biologische Probe einen Pool von Proben umfasst.

46. Kit für die Bestimmung und/oder Analyse der Sequenz einer Ziel-Nukleinsäure in einer biologischen Probe auf Basis von Massenspektrometrie nach dem Verfahren nach einem der Ansprüche 1 bis 45, wobei das Kit umfasst:
(a) Nukleotid-Triphosphate;
(b) mindestens eine Polymerase;
(c) mindestens ein Nukleinsäure-Spaltungsreagens für die Durchführung verschiedener basenspezifischer Spaltungsreaktionen; und
(d) ein Computerprogramm, das Codes zur Durchführung einer systematischen Computeranalyse der Massenspektren umfasst, die mittels der massenspektrometrischen Analyse von nicht-geordneten Fragmenten, die ein Satz von Fragmenten ohne einen gemeinsamen Endpunkt sind, wenn sie auf einem Datenverarbeitungssystem ausgeführt werden, erhalten werden,
wobei das Programm die Schritte umfasst, 1/ eine Referenz-Nukleinsäure und Sequenz-Varianten von dieser verschiedenen Basen-spezifischen Spaltungen zu unterwerfen, um Fragmente zu erzeugen, die Masse jedes Fragments zu erfassen, die Massenspektren der Fragmente von einer Referenz-Nukleinsäure und die Sequenz-Varianten davon für jede der Basen-spezifischen Spaltungsreaktionen zu erzeugen, und 2/ diese mittels Rechner abgeleiteten Massenspektren mit den Spektren abzugleichen, die experimentell in den verschiedenen Basen-spezifischen Spaltungsreaktionen erhalten wurden.

47. Das Kit nach Anspruch 46, das weiterhin Kationen-Austauscher-Kügelchen umfasst, um den nicht-geordneten Satz von Fragmenten, erzeugt mittels des Verfahrens nach einem der Ansprüche 1 bis 45, zu reinigen.

48. Das Kit nach Anspruch 47, das weiterhin einen festen Träger umfasst, auf den der nichtgeordnete Satz von Fragmenten aufgetragen werden kann.

49. Verwendung des Kits nach einem der Ansprüche 46 bis 49 für die Bestimmung und/oder Analyse der Sequenz einer Ziel-Nukleinsäure auf Basis von Massenspektrometrie, für die Bestimmung und/oder Analyse von Sequenzunterschieden, für das Ermitteln von bekannten wie auch unbekannten Nukleotid-Sequenzabweichungen, für den Nachweis/die Identifizierung oder für das Durchführen einer Genom-weiten Bestimmung des Genotyps unter Verwendung einer Ziel-Nukleinsäure in einer biologischen Probe, für die eine Referenz-Nukleinsäure-Sequenz bekannt ist.

50. Verwendung eines Kits nach einem der Ansprüche 46 bis 49 für die Bestimmung und/oder Analyse der Sequenz einer Ziel-Nukleinsäure auf Basis von Massenspektrometrie, für den Nachweis/die Identifizierung oder für die Durchführung einer Genom-weiten Bestimmung des Genotyps unter Verwendung einer Ziel-Nukleinsäure unbekannter Sequenz in einer biologischen Probe.

## Revendications

1. Procédé de spectrométrie de masse, basé sur la détermination et/ou l'analyse de la séquence d'un acide nucléique cible présent dans un échantillon biologique, ledit procédé comprenant les étapes consistant à :
(a) dériver dudit échantillon biologique ledit acide nucléique cible ;
(b) soumettre ledit acide nucléique cible obtenu à partir de l'étape (a) à un groupe de quatre réactions de clivage complémentaires séparées spécifiques à une base, la réaction de clivage générant un groupe de fragments non ordonnés, qui est un groupe de fragments sans point final commun ;
(c) analyser des groupes de fragments non ordonnés obtenus à partir de l'étape (b) par spectrométrie de masse ; et
(d) réaliser une analyse systématique par ordinateur des spectres de masse obtenu à l'étape (c) pour déterminer et/ou analyser la séquence dudit acide nucléique cible,
dans lequel lesdites réactions de clivage complémentaires font référence aux digestions d'acides nucléiques cibles, **caractérisées par** la variation de la spécificité et/ou à la digestion de formes alternatives de ladite séquence cible.

2. Procédé selon la revendication 1, dans lequel ledit échantillon biologique est dérivé d'un organisme choisi dans le groupe comprenant les eucaryotes, les procaryotes et les virus.

3. Procédé selon la revendication 1 ou 2, dans lequel ledit acide nucléique cible est choisi dans le groupe comprenant un ADN à brin simple, un ADN à double brin, un ADNc, un ARN à brin simple, un ARN à double brin, un hybride ADN/ARN et un acide nucléique mosaïque ADN/ARN.

4. Procédé selon l'une quelconque des revendications 1 à 3, dans lequel ledit acide nucléique cible est dérivé par un/des procédé(s) d'amplification consécutif(s) choisi(s) dans le groupe comprenant le clonage in vivo, la réaction en chaîne par polymérase (PCR), la transcription inverse suivie par la réaction en chaîne par polymérase (PCR-RT), l'amplification par déplacement de brin (SDA) et les processus à base de transcription.

5. Procédé selon la revendication 4, dans lequel ledit acide nucléique cible amplifié est un transcrit généré à partir d'un acide nucléique cible à brin simple ou à double brin par un procédé comprenant les étapes consistant à :
(a) lier de manière fonctionnelle une séquence de contrôle de la transcription audit acide nucléique cible ; et
(b) transcrire l'un ou les deux brins de l'acide nucléique cible de l'étape a) en utilisant une/des ARN polymérase(s) qui reconnaît/reconnaissent la séquence de contrôle de la transcription.

6. Procédé selon la revendication 5, dans lequel lesdites séquences de contrôle de la transcription sont liées de manière fonctionnelle à l'acide nucléique cible par amplification par PCR en utilisant des amorces qui incorporent les séquences de contrôle transcriptionnelles en tant que prolongements à l'extrémité 5'.

7. Procédé selon les revendications 5 ou 6, dans lequel la séquence de contrôle de la transcription est choisie dans le groupe comprenant des séquences eucaryotes de contrôle de la transcription, des séquences procaryotes de contrôle de la transcription et des séquences virales de contrôle de la transcription.

8. Procédé selon la revendication 7, dans lequel la séquence de contrôle procaryote de la transcription est choisie dans le groupe comprenant les promoteurs T3, T7 et SP6.

9. Procédé selon la revendication 8, dans lequel lesdites ARN polymérases utilisant les promoteurs T3, T7 ou SP6 sont des ARN polymérases soit de type sauvage, soit de type mutant, les polymérases mutantes pouvant être incorporées dans les substrats non canoniques des transcrits avec un substituant 2'-désoxy, 2'-O-méthyle, 2'-fluoro ou 2'-amino.

10. Procédé selon la revendication 9, dans lequel l'ARN polymérase mutante est soit la polymérase T7 mutante, soit la polymérase SP6 mutante.

11. Procédé selon l'une quelconque des revendications 1 à 10, dans lequel l'acide nucléique cible dérivé comprend un/des nucléoside(s) qui est/sont modifié(s) sur la base, la fraction de sucre et/ou la fraction phosphate, les modifications changeant la spécificité du clivage par le/les réactif(s) de clivage et/ou la masse et/ou la longueur des produits de clivage.

12. Procédé selon la revendication 11, dans lequel la modification est introduite par incorporation enzymatique de désoxynucléosides triphosphates modifiés, de ribonucléosides triphosphates modifiés, et/ou de didésoxynucléosides triphosphates modifiés ; ou dans lequel la modification est introduite chimiquement, ou dans lequel la modification est introduite par une combinaison des deux procédés.

13. Procédé selon la revendication 11 ou 12, dans lequel la modification comprend un substituant 2'-désoxy, 2'-O-méthyle, 2'-fluoro ou 2'-amino sur les nucléotides triphosphates.

14. Procédé selon la revendication 11 ou 12, dans lequel la modification comprend des liaisons de phosphorothioate internucléoside ou des liaisons de phosphorothioate internucléoside réagissant en outre avec un réactif alkylant.

15. Procédé selon la revendication 11 ou 12, dans lequel la modification comprend un groupe méthyle en C5 des sous-unités uridine-5'-monophosphate.

16. Procédé selon la revendication 11 ou 12, dans lequel la modification comprend des nucléotides qui incorporent des variantes d'isotopes.

17. Procédé selon l'une quelconque des revendications 1 à 16, dans lequel l'acide nucléique cible de l'étape (a) est purifié avant clivage.

18. Procédé selon la revendication 17, dans lequel ladite purification est réalisée par immobilisation ou par chromatographie.

19. Procédé selon l'une quelconque des revendications 1 à 18, dans lequel les réactions de clivage complémentaires sont choisies dans le groupe comprenant le clivage enzymatique, le clivage chimique et le clivage physique.

20. Procédé selon la revendication 19, dans lequel les réactions de clivage complémentaires sont **caractérisées par** une spécificité de mono-nucléotide relâché, de mono-nucléotide, de di-nucléotide relâché ou de di-nucléotide.

21. Procédé selon la revendication 19 ou 20, dans lequel ledit acide nucléique cible est soumis à une réaction de digestion chimique comprenant le traitement avec des réactifs alcalins ou avec des réactifs utilisés dans la méthode de séquençage de Maxam & Gilbert.

22. Procédé selon la revendication 19 ou 20, dans lequel ledit acide nucléique cible est soumis à une réaction de clivage enzymatique utilisant une/des enzyme(s) choisie(s) dans le groupe comprenant des endonucléases et des exonucléases.

23. Procédé selon la revendication 22, dans lequel l'acide nucléique cible est soumis à une réaction de clivage enzymatique utilisant une/des endonucléase(s) choisie(s) dans le groupe comprenant des enzymes de restriction, des endonucléases d'ARN, des endonucléases d'ADN et des phosphodiestérases non spécifiques.

24. Procédé selon la revendication 23, dans lequel la/les endonucléase(s) est/sont une/des endonucléase(s) d'ARN sélective(s) ou non sélective(s), choisie(s) dans le groupe comprenant la ribonucléases T₁ G-spécifique, la ribonucléase U₂ A-spécifique, la ribonucléase phyM A/U-spécifique, la ribonucléase A, U/C-spécifique, la ribonucléase de foie de poulet C-spécifique (RNaseCL3) et la cusativine, la RNase-I non spécifique et les RNases préférant la pyrimidine-adénosine isolées de *E. coli, de Enterbacter sp.,* ou de *Saccharomyces cerevisiae*.

25. Procédé selon l'une quelconque des revendications 1 à 8, 11, 12, 14, 17 à 20, 22 et 23, dans lequel ledit acide nucléique cible est un ADN ou un ARN à brin simple à modification de phosphorothioate et dans lequel les digestions d'acide nucléique sont réalisées avec la nucléase P1.

26. Procédé selon l'une quelconque des revendications 1 à 24, dans lequel ledit acide nucléique cible est un acide nucléique d'ARN/ADN mosaïque ou un acide nucléique d'ARN/ADN mosaïque modifié, préparé avec des polymérases mutantes, et dans lequel les réactifs de clivage sont des endonucléases d'ARN, des endonucléases d'ADN ou des réactifs alcalins.

27. Procédé selon l'une quelconque des revendications 1 à 24 et 26, dans lequel ledit acide nucléique cible est un transcrit, un transcrit modifié, un transcrit d'ARN/ADN mosaïque ou un transcrit d'ARN/ADN mosaïque modifié, préparé avec des ARN polymérases de type sauvage ou mutant, et dans lequel les réactifs de clivage est/sont une/des endonucléase(s) d'ARN sélective(s) ou non sélective(s) ou des réactifs alcalins.

28. Procédé selon l'une quelconque des revendications 1 à 24 et 26 ou 27, dans lequel ledit acide nucléique cible est un transcrit d'ARN/ADN mosaïque qui comprend du dCMP, dUMP ou du dTMP, préparés avec la polymérase T7 ou SP6 mutante, et dans lequel le réactif de clivage est une RNase spécifique de la pyrimidine.

29. Procédé selon la revendication 28, dans lequel ladite RNase spécifique de la pyrimidine est la RNase-A.

30. Procédé selon l'une quelconque des revendications 1 à 29, dans lequel le groupe de fragments non ordonnés de l'étape (b) est purifié en outre à l'aide de billes d'échange de cations.

31. Procédé selon l'une quelconque des revendications 1 à 30, dans lequel le groupe de fragments non ordonnés de l'étape (b) est appliqué sur un support solide.

32. Procédé selon la revendication 31, dans lequel ledit support solide est choisi dans le groupe comprenant les surfaces solides, les plaques et les puces.

33. Procédé selon l'une quelconque des revendications 1 à 32, dans lequel l'analyse par spectrométrie de masse des fragments d'acide nucléique est réalisée en appliquant un procédé de spectrométrie de masse choisi dans le groupe comprenant la spectrométrie de masse MALDI-TOF, la spectrométrie de masse ESI et la spectrométrie de masse ICR à transformée de Fourier (FT-ICR).

34. Procédé selon l'une quelconque des revendications 1 à 33 pour la détermination par spectrométrie de masse et/ou analyse d'un acide nucléique cible présent dans un échantillon biologique, dans lequel pour ledit acide nucléique cible, une séquence d'acide nucléique de référence est connue ; ledit procédé comprenant une étape supplémentaire dans laquelle les spectres de masse des fragments non ordonnés obtenus à l'étape c) de la revendication 1 sont comparés avec les spectres de masse connus ou prévus pour une séquence d'acide nucléique de référence, et la déduction à partir de ceux-ci, par analyse systématique par ordinateur, de toutes les séquences de nucléotides ou des parties de celles-ci de l'acide nucléique cible et la comparaison de la séquence d'acide nucléique avec celle de l'acide nucléique de référence pour déterminer si l'acide nucléique cible présente la même séquence ou une séquence différente de celle de l'acide nucléique de référence.

35. Procédé selon la revendication 34, dans lequel la différence de la séquence d'acide nucléique qui est déterminée est une délétion, une substitution, une insertion ou des combinaisons de celles-ci.

36. Procédé selon la revendication 35, dans lequel la différence de séquence de l'acide nucléique est un polymorphisme de nucléotide unique (SNP).

37. Procédé selon l'une quelconque des revendications 34 à 36 pour la cotation des variations de séquences de nucléotides connues ou inconnues de l'acide nucléique cible présent dans un échantillon biologique.

38. Procédé selon la revendication 37, dans lequel la détermination basée sur la spectrométrie de masse et/ou l'analyse desdites variations connues ou inconnues de la séquence de nucléotide permet l'identification de diverses séquences alléliques d'une certaine région/d'un certain gène, la cotation des mutations associées à une maladie, la détection des variations somatiques ou des études dans le domaine de l'évolution moléculaire.

39. Procédé selon l'une quelconque des revendications 34 à 38, pour le génotypage sur l'ensemble du génome d'un échantillon biologique.

40. Procédé selon la revendication 34, dans lequel les spectres de masse obtenus pour un acide nucléique cible sont comparés aux spectres de masse prévus pour une pluralité d'acides nucléiques de référence, en identifiant/détectant ainsi un acide nucléique cible dans un échantillon biologique.

41. Procédé selon l'une quelconque des revendications 1 à 33, pour la détermination basée sur la spectrométrie de masse et/ou l'analyse de la séquence d'un acide nucléique cible d'une séquence inconnue présente dans un échantillon biologique.

42. Procédé selon la revendication 41 pour la détermination basée sur la spectrométrie de masse et/ou l'analyse de la séquence d'un acide nucléique cible d'une séquence inconnue présente dans un échantillon biologique, dans lequel, si la séquence n'est pas définie de manière unique après l'étape (d) de la revendication 1, les étapes (a) à (d) sont répétées, générant ainsi des formes modifiées dudit acide nucléique cible et/ou différentes parties dudit acide nucléique cible, et réalisant des réactions de clivage spécifiques supplémentaires de mono- et/ou di- nucléotides donnant des groupes supplémentaires de fragments non ordonnés jusqu'à ce que les données combinées convergent en une solution de séquence unique.

43. Procédé selon la revendication 41 ou 42 pour la génotypage sur l'ensemble du génome d'un échantillon biologique.

44. Procédé selon l'une quelconque des revendications 1 à 43, dans lequel l'acide nucléique cible est préparé par l'amplification concomitante de fragments multiples.

45. Procédé selon l'une quelconque des revendications 1 à 38, 41, 42 ou 44, dans lequel ledit échantillon biologique comprend un pool d'échantillons.

46. Kit pour la détermination basée sur la spectrométrie de masse et/ou pour l'analyse de la séquence d'un acide nucléique cible présent dans un échantillon biologique selon un procédé de l'une quelconque des revendications 1 à 45, le kit comprenant :
(a) des nucléotides triphosphates ;
(b) une/des polymérase(s) ;
(c) un/des agent(s) de clivage de l'acide nucléique pour réaliser différentes réactions de clivages spécifiques d'une base, et
(d) un programme informatique comprenant des codes pour effectuer une analyse systématique par ordinateur sur les spectres de masse obtenus à partir de l'analyse spectrométrique de la masse des fragments non ordonnés, qui est un groupe de fragments sans point final commun, lorsqu'elle est exécutée sur un système de traitement de données,
ledit programme comprenant les étapes consistant 1) à soumettre un acide nucléique de référence et des variantes de séquence de celui-ci aux différents clivages spécifiques d'une base pour générer des fragments, à calculer par ordinateur la masse de chaque fragment, à générer les spectres de masse des fragments à partir d'un acide nucléique de référence et les variantes de séquence de ceux-ci pour chacune des réactions de clivage spécifiques d'une base et 2) à faire correspondre ces spectres de masse dérivés par informatique avec les spectres obtenus de manière expérimentale dans différentes réactions de clivage spécifiques à une base.

47. Kit selon la revendication 46 qui comprend en outre des billes d'échange de cations afin de purifier le groupe non ordonné de fragments générés au moyen d'un procédé selon les revendications 1 à 45.

48. Kit selon la revendication 47, qui comprend en outre un support solide sur lequel le groupe non ordonné de fragments peut être appliqué.

49. Utilisation d'un kit selon l'une quelconque des revendications 46 à 48 pour la détermination basée sur la spectrométrie de masse et/ou l'analyse de la séquence d'un acide nucléique cible, pour déterminer et/ou analyser des différences de séquence, pour coter des variations connues ou inconnues de séquences de nucléotides, pour détecter/identifier ou pour réaliser un génotypage sur l'ensemble du génome en utilisant un acide nucléique cible présent dans un échantillon biologique, une séquence d'acide nucléique de référence étant connue pour l'acide nucléique cible.

50. Utilisation d'un kit selon l'une quelconque des revendications 46 à 48, pour la détermination par spectrométrie de masse et/ou analyse de la séquence d'un acide nucléique cible pour détecter/identifier ou pour réaliser un génotypage sur l'ensemble du génome en utilisant un acide nucléique cible présent dans un échantillon biologique.


### SEQUENCE LISTING

<110> METHEXIS NV
<120> SEQUENCING BY A COMBINATION OF MONONUCLEOTIDE-SPECIFIC DIGESTION AND MASS SPECTROMETRY
<130> 29314/35410A
<140>
   <141>
<150> 60/131,984
   <151> 1999-04-30
<160> 30
<170> PatentIn Ver. 2.1
<210> 1
   <211> 120
   <212> DNA
   <213> Homo sapiens
<220>
   <223> exon 5 of human p53
<400> 1
   tactcccctg ccctcaacaa gatgttttgc caactggcca agacctgccc tgtgcagctg 60
   tgggttgatt ccacaccccc gcccggcacc cgcgtccgcg ccatggccat ctacaagcag 120
<210> 2
   <211> 40
   <212> DNA
   <213> Artificial Sequence
<220>
   <223> Description of Artificial Sequence: synthetic
<220>
   <223> pGEM3-Zf(+) derived nucleotide
<400> 2
   gtaaaacgac ggccagtgaa ttgtaatacg actcactata                        40
<210> 3
   <211> 972
   <212> DNA
   <213> Artificial Sequence
<220>
   <223> Description of Artificial Sequence: synthetic
<220>
   <223> pGEM3-Zf(+) derived nucleotide
<400> 3
   gggcgaattc gagctcggta cccggggatc ctctagagtc gacctgcagg catgcaagct 60
   tgagtattct atagtgtcac ctaaatagct tggcgtaatc atggtcatag ctgtttcctg 120
   tgtgaaattg ttatccgctc acaattccac acaacatacg agccggaagc ataaagtgta 180
   aagcctgggg tgcctaatga gtgagctaac tcacattaat tgcgttgcgc tcactgcccg 240
   ctttccagtc gggaaacctg tcgtgccagc tgcattaatg aatcggccaa cgcgcgggga 300
   gaggcggttt gcgtattggg cgctcttccg cttcctcgct cactgactcg ctgcgctcgg 360
   tcgttcggct gcggcgagcg gtatcagctc actcaaaggc ggtaatacgg ttatccacag 420
   aatcagggga taacgcagga aagaacatgt gagcaaaagg ccagcaaaag gccaggaacc 480
   gtaaaaaggc cgcgttgctg gcgtttttcc ataggctccg cccccctgac gagcatcaca 540
   aaaatcgacg ctcaagtcag aggtggcgaa acccgacagg actataaaga taccaggcgt 600
   ttccccctgg aagctccctc gtgcgctctc ctgttccgac cctgccgctt accggatacc 660
   tgtccgcctt tctcccttcg ggaagcgtgg cgctttctca tagctcacgc tgtaggtatc 720
   tcagttcggt gtaggtcgtt cgctccaagc tgggctgtgt gcacgaaccc cccgttcagc 780
   ccgaccgctg cgccttatcc ggtaactatc gtcttgagtc caacccggta agacacgact 840
   tatcgccact ggcagcagcc actggtaaca ggattagcag agcgaggtat gtaggcggtg 900
   ctacagagtt cttgaagtgg tggcctaact acggctacac tagaagaaca gtatttggta 960
   tctgcgctct gc                                                     972
<210> 4
   <211> 131
   <212> DNA
   <213> Artificial Sequence
<220>
   <223> Description of Artificial Sequence: synthetic
<220>
   <223> PCR products and transcripts
<400> 4
   taatacgact cactataggg cgacttcacg aagacggtga aactgttgga tccaattctt 60
   acccacacaa atacaacaac tacgaaggtt ttgatttctc tgtgagctct ccctactacg 120
   aatggcctat c                                                      131
<210> 5
   <211> 134
   <212> DNA
   <213> Artificial Sequence
<220>
   <223> PCR products and transcripts
<220>
   <223> Description of Artificial Sequence: synthetic
<400> 5
   taatacgact cactataggg cgaattcgta gtagggagag ctcacagaga aatcaaaacc 60
   ttcgtagttg ttgtatttgt gtgggtaaga attggatcca acagtttcac cgtcttcgtg 120
   aagtttatat ccgg 134
<210> 6
   <211> 45
   <212> DNA
   <213> Artificial Sequence
<220>
   <223> Description of Artificial Sequence: synthetic
<220>
   <223> reference nucleotide
<400> 6
   ggatccaatt cttacccaca caaatacaac aactacgaag gtttt 45
<210> 7
   <211> 45
   <212> DNA
   <213> Artificial Sequence
<220>
   <223> Description of Artificial Sequence: synthetic
<220>
   <223> mutant 1
<400> 7
   ggatccaatt ctttcccaca caaatacaac aactacgaag gtttt 45
<210> 8
   <211> 45
   <212> DNA
   <213> Artificial Sequence
<220>
   <223> Description of Artificial Sequence: synthetic
<220>
   <223> mutant 2
<400> 8
   ggatccaatt cttacccaca caaatacaac aagtacgaag gtttt 45
<210> 9
   <211> 45
   <212> DNA
   <213> Artificial Sequence
<220>
   <223> Description of Artificial Sequence: synthetic
<220>
   <223> mutant 3
<400> 9
   ggatccaatt cttacccaca caaatacaac aactacgatg gtttt 45
<210> 10
   <211> 45
   <212> DNA
   <213> Artificial Sequence
<220>
   <223> Description of Artificial Sequence: synthetic
<220>
   <223> mutant 4
<400> 10
   ggatccaatt cttacccaca caaatacaac aactacgtag gtttt 45
<210> 11
   <211> 45
   <212> DNA
   <213> Artificial Sequence
<220>
   <223> Description of Artificial Sequence: synthetic
<220>
   <223> mutant 5
<400> 11
   ggatccaatt cttacccaca caaatacaac acgtacgaag gtttt 45
<210> 12
   <211> 45
   <212> DNA
   <213> Artificial Sequence
<220>
   <223> Description of Artificial Sequence: synthetic
<220>
   <223> mutant 6
<400> 12
   ggatccaatt cttacccaca ccagtacaac aactacgaag gtttt 45
<210> 13
   <211> 45
   <212> DNA
   <213> Artificial Sequence
<220>
   <223> Description of Artificial Sequence: synthetic
<220>
   <223> mutant 7
<400> 13
   ggatccgcgt cttacccaca caaatacaac aactacgaag gtttt 45
<210> 14
   <211> 45
   <212> DNA
   <213> Artificial Sequence
<220>
   <223> Description of Artificial Sequence: synthetic
<220>
   <223> mutant 8
<400> 14
   ggatccaatt cttacccaca caaatacaag aatttcgaag gtttt 45
<210> 15
   <211> 13
   <212> DNA
   <213> Artificial Sequence
<220>
   <223> Description of Artificial Sequence: synthetic
<400> 15
   ctagcccccg atc 13
<210> 16
   <211> 25
   <212> DNA
   <213> Artificial Sequence
<220>
   <223> Description of Artificial Sequence: primer
<400> 16
   ccggatataa acttcacgaa gacgg 25
<210> 17
   <211> 26
   <212> DNA
   <213> Artificial Sequence
<220>
   <223> Description of Artificial Sequence: primer
<400> 17
   gataggccat tcgtagtagg gagagc 26
<210> 18
   <211> 37
   <212> DNA
   <213> Artificial Sequence
<220>
   <223> Description of Artificial Sequence: primer
<400> 18
   taatacgact cactataggg cgacttcacg aagacgg 37
<210> 19
   <211> 41
   <212> DNA
   <213> Artificial Sequence
<220>
   <223> Description of Artificial Sequence: primer
<400> 19
   taatacgact cactataggg cgaattcgta gtagggagag c 41
<210> 20
   <211> 35
   <212> DNA
   <213> Artificial Sequence
<220>
   <223> Description of Artificial Sequence: synthetic
   <220>
   <223> reference fragment
<400> 20
   gtagttgttg tatttgtgtg ggtaagaatt ggatc 35
<210> 21
   <211> 15
   <212> RNA
   <213> Artificial Sequence
<220>
   <223> Description of Artificial Sequence: synthetic
<220>
   <223> polymorphic 15-mer fragment
<400> 21
   aaaucaaaac cuucg 15
<210> 22
   <211> 10
   <212> DNA
   <213> Artificial Sequence
<220>
   <223> RNAse-A digestion products
<220>
   <223> Description of Artificial Sequence: synthetic
<400> 22
   ttgagtattc 10
<210> 23
   <211> 10
   <212> DNA
   <213> Artificial Sequence
<220>
   <223> RNAse-A digestion products
<220>
   <223> Description of Artificial Sequence: synthetic
<400> 23
   acccggggat 10
<210> 24
   <211> 15
   <212> DNA
   <213> Artificial Sequence
<220>
   <223> RNAse-A digestion products
<220>
   <223> Description of Artificial Sequence: synthetic
<400> 24
   gaaggttttg atttc 15
<210> 25
   <211> 12
   <212> DNA
   <213> Artificial Sequence
<220>
   <223> RNAse-A digestion products
<220>
   <223> Description of Artificial Sequence: synthetic
<400> 25
   acccacacaa at 12
<210> 26
   <211> 12
   <212> DNA
   <213> Artificial Sequence
<220>
   <223> RNAse-A digestion products
<220>
   <223> Description of Artificial Sequence: synthetic
<400> 26
   cacgaagacg gt 12
<210> 27
   <211> 14
   <212> DNA
   <213> Artificial Sequence
<220>
   <223> RNAse-A digestion products
<220>
   <223> Description of Artificial Sequence: synthetic
<400> 27
   gtgaagttta tatc 14
<210> 28
   <211> 14
   <212> DNA
   <213> Artificial Sequence
<220>
   <223> RNAse-A digestion products
   <220>
   <223> Description of Artificial Sequence: synthetic
<400> 28
   gtagtaggga gagc 14
<210> 29
   <211> 10
   <212> DNA
   <213> Artificial Sequence
<220>
   <223> RNAse-A digestion products
<220>
   <223> Description of Artificial Sequence: synthetic
<400> 29
   agggagagct 10
<210> 30
   <211> 11
   <212> DNA
   <213> Artificial Sequence
<220>
   <223> RNAse-A digestion products
<220>
   <223> Description of Artificial Sequence: synthetic
<400> 30
   cacagagaaa t 11